(19) Europäisches Patentamt / European Patent Office / Office européen des brevets

(11) **EP 4 431 519 A1**

(12) **EUROPEAN PATENT APPLICATION**
published in accordance with Art. 153(4) EPC

(43) Date of publication:
**18.09.2024 Bulletin 2024/38**

(21) Application number: **22883600.3**

(22) Date of filing: **19.10.2022**

(51) International Patent Classification (IPC):
***C07K 1/113*** (2006.01) ***C40B 40/08*** (2006.01)
***C40B 40/10*** (2006.01) ***C12N 15/11*** (2006.01)
***C12P 21/02*** (2006.01) ***C12N 9/10*** (2006.01)

(52) Cooperative Patent Classification (CPC):
**C07K 1/113; C12N 9/10; C12N 15/11; C12P 21/02; C40B 40/08; C40B 40/10**

(86) International application number:
**PCT/JP2022/038924**

(87) International publication number:
**WO 2023/068296 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.10.2021 JP 2021170768**

(71) Applicant: **The University of Tokyo**
**Bunkyo-ku, Tokyo 113-8654 (JP)**

(72) Inventors:
- **SUGA, Hiroaki**
  **Tokyo 113-8654 (JP)**
- **GOTO, Yuki**
  **Tokyo 113-8654 (JP)**
- **ZHANG Yuchen**
  **Tokyo 113-8654 (JP)**
- **OKADA, Masahiro**
  **Tokyo 113-8654 (JP)**

(74) Representative: **D Young & Co LLP**
**3 Noble Street**
**London EC2V 7BQ (GB)**

Remarks:
The complete document including Reference Table(s) and the Sequence Listing(s) can be downloaded from the EPO website

(54) **METHOD FOR PRODUCING COMPOUNDS, METHOD FOR PRODUCING COMPOUND LIBRARY, COMPOUND LIBRARY, AND SCREENING METHOD**

(57) The present invention relates to a method for producing compounds that have at least one of the structures represented by the following formula (I) or (II), the method comprising a step of contacting a compound having at least one structure represented by the following formula (III) or (IV) with a prenyltransferase to introduce a prenyl group into the structure, wherein the prenyltransferase is LimF or an enzyme homologous thereto.

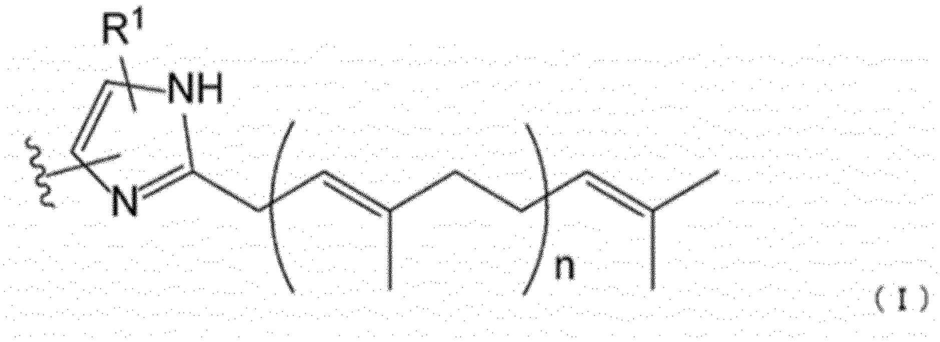

(I)



Processed by Luminess, 75001 PARIS (FR)

(Cont. next page)

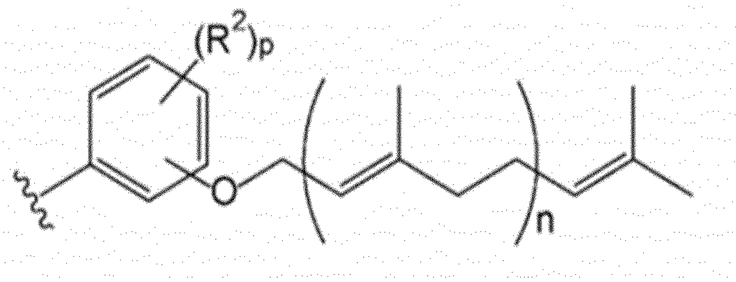

(II)

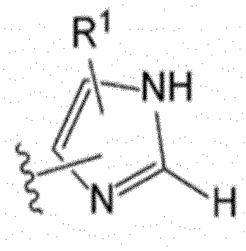

(III)

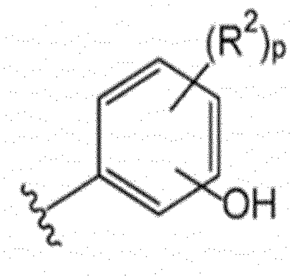

(IV)

[FIG. 7]

Fig. 7a

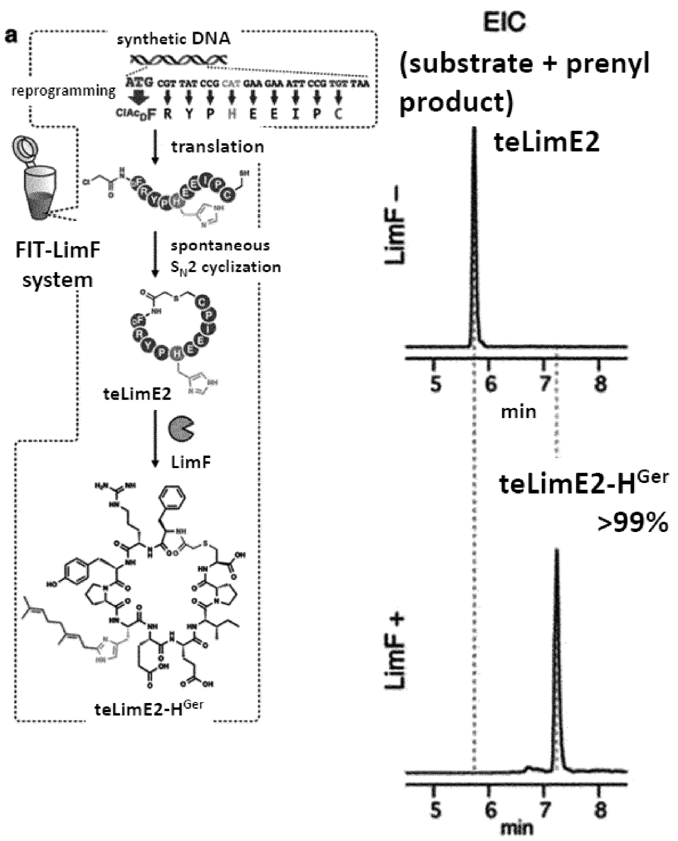




(Cont. next page)

Fig. 7b-c

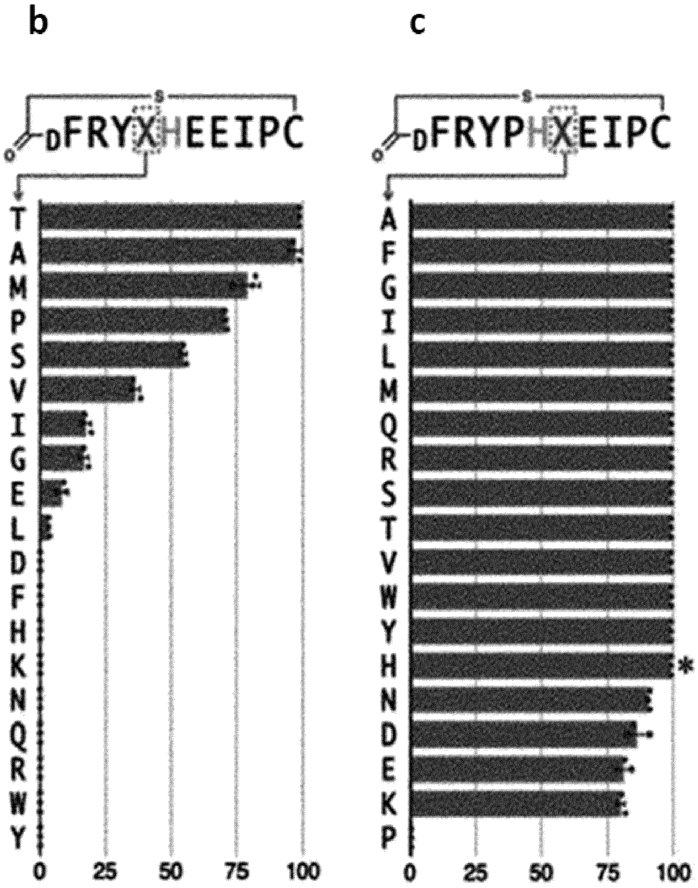


Fig. 7d-e

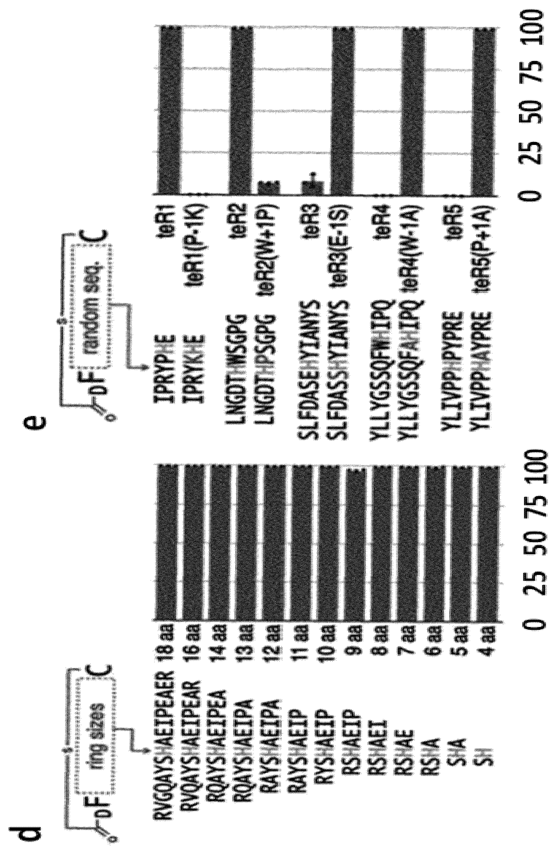




(Cont. next page)

Fig. 7f

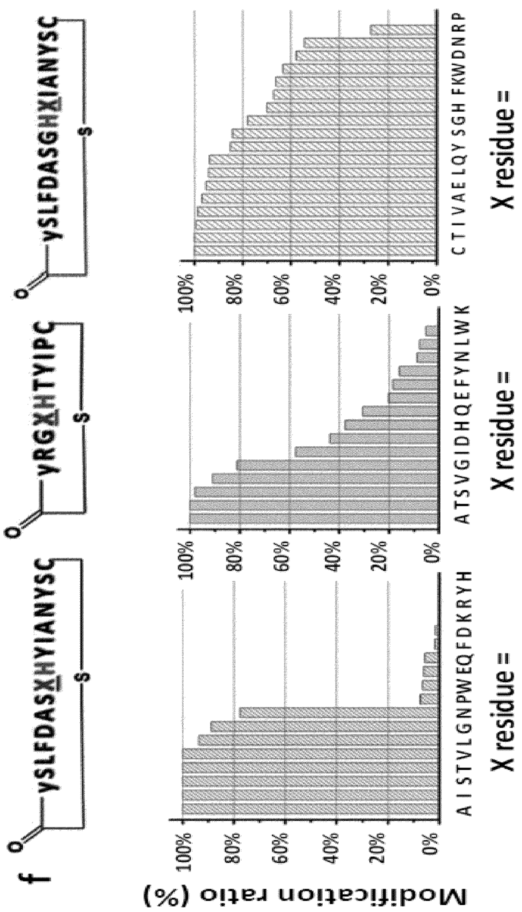
f
ySLFDASXHYIANYSC
yRGXHTYIPC
ySLFDASGHXIANYSC
Modification ratio (%)
100%
80%
60%
40%
20%
0%
A I S T V L G N P W E Q F D K R Y H
A T S V G I D H Q E F Y N L W K
C T I V A E L Q Y S G H F K W D N R P
X residue =
X residue =
X residue =

## Description

### Field of Art

The present invention relates to a method for producing a compound, a method for producing a compound library, a compound library, and a screening method. The present invention further relates to a method for producing a peptide or protein, a method for producing a peptide or protein library, a peptide or protein library, and a screening method.

### Background Art

Prenylation is a universal modification throughout the primary metabolism, as well as in various natural product biosynthetic pathways including for terpenoids, polyketides, non-ribosome peptides, and ribosomally synthesized and post-translationally modified peptides (RiPPs). Prenylation often plays an important role in the diverse biological activities of these natural products. This is because prenyl groups generally enhance the lipophilicity of molecules and their interactions with lipid membranes thereby providing desirable bioavailability and membrane permeability. These characteristics of prenyl groups are of particular interest in the development of peptide formulations known to have low membrane permeability. Alkylation is known as one of the most successful and important strategies to overcome low membrane permeability and is used medicinally for the development of peptide formulations such as liraglutide and insulin detemir.

Therefore, developing new biocatalysts for the prenylation of peptides is not only expanding the toolbox for synthetic biology but also advancing the design of physiologically active peptides for drug discovery.

Prenyltransferases (PTase), which are involved in biosynthesis of cyanobactins, a class of RiPPs, are interesting enzymes that can be used as prenylation biocatalysts for various peptides. Such prenyltransferases, so-called F-family enzymes, have a shortened $\alpha/\beta$ PTase barrel fold structure having a specific cavity exposed to a solvent and allow for the binding of large peptide substrates. Although this unique structure shows strict selectivity for prenyl group donor and acceptor residues, the overall substrate peptide sequence has a low selectivity, thus such enzymes are a promising biocatalyst for easy and versatile peptide alkylation. F-family prenyltransferases catalyze various prenylation despite having similar tertiary structures. To this point, specific prenyltransferases having Tyr O-prenylation activity, Trp C- or N-prenylation activity, Arg N-prenylation activity, Ser/Thr O-prenylation activity, or terminal N- and C-prenylation activity for a peptide substrate have been reported in this family (patent document 1, non-patent documents 1 to 6).

### Prior Art Documents

#### Patent Documents

Patent Document 1: WO 2020/080490

#### Non-Patent Documents

Non-Patent Document 1: Hao, Y et al. Molecular basis for the broad substrate selectivity of a peptide prenyltransferase. Proc. Natl. Acad. Sci. USA, 113, 14037-14042 (2016).

Non-Patent Document 2: Dalponte, L. et al. N-Prenylation of tryptophan by an aromatic prenyltransferase from the cyanobactin biosynthetic pathway. Biochemistry, 57, 6860-6867 (2018).

Non-Patent Document 3: Tianero, M.D. et al. Metabolic model for diversity-generating biosynthesis. Proc. Natl. Acad. Sci. USA, 113, 1772-1777 (2016).

Non-Patent Document 4: Phan, C.-S. et al. Argicyclamides A-C Unveil Enzymatic Basis for Guanidine Bis-prenylation. J. Am. Chem. Soc., 143, 10083-10087 (2021).

Non-Patent Document 5: Parajuli, A. et al. A Unique Tryptophan C-Prenyltransferase from the Kawaguchipeptin Biosynthetic Pathway. Angew. Chem., 55, 3596-3599 (2016).

Non-Patent Document 6: Okada, M. et al. Stereospecific prenylation of tryptophan by a cyanobacterial post-translational modification enzyme. Org. Biomol. Chem., 14, 9639-9644 (2016).

## Summary of Invention

### Problem to Be Solved by Invention

Among previously reported prenylations, C-prenylation is one of the most attractive modifications. This is because the formation of carbon-carbon bonds is of central importance to both biochemistry and organic chemistry for constructing the carbon backbone of molecules.

However, it is known that most F-family prenyltransferases catalyze the prenylation of electron-rich heteroatoms. The only known prenyltransferase having a C-prenylation activity in the cyanobactin biosynthetic gene cluster (BGC) is KgpF derived from kawaguchipeptin BGC that catalyzes Trp C-3 prenylation (non-patent documents 5 and 6). Therefore, in order to provide a potential biocatalyst for expanding the chemical space of prenylated natural products and alkylation of various physiologically active peptides, it is desirable to search for a novel prenyltransferase that catalyzes C-prenylation of an unprecedented acceptor.

Furthermore, it is also desirable to search for prenyltransferases and prenylation methods that are not limited to C-prenylation but can prenylate a variety of substrates.

The purpose of the present invention is to provide: a novel method for producing a prenylated compound, which can solve any of the problems described above; a novel method for producing a prenylated compound library; and the like.

### Means for Solving Problem

As a result of intensive studies to solve the above problems, the present inventors have discovered that a prescribed prenyltransferase such as LimF can not only catalyze a new C-prenylation, but also catalyzes prenylation of various substrates, leading to complete the present invention.

That is, the present invention is as described below.

[1] A method for producing a compound having at least one of the structures represented by the following formulas (I) or (II):

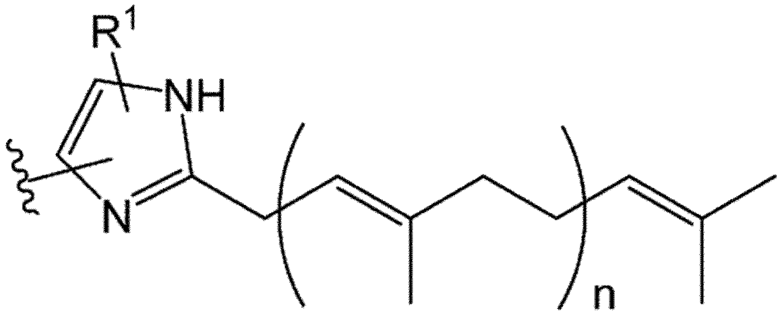

( I )

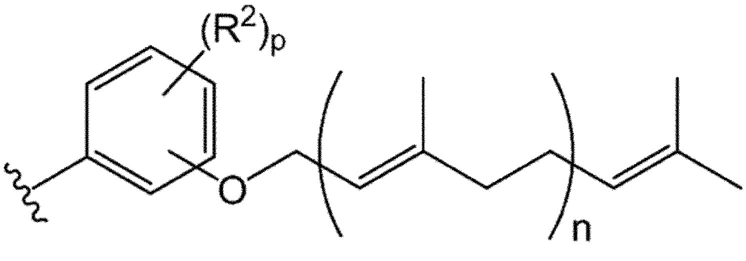

( I I )

(in formula (I) above, $R^1$ is a hydrogen atom or optional substituent, and n represents an integer of from 0 to 11; in formula (II) above, each $R^2$ is independently optional substituent, where p represents an integer of from 0 to 4 and n represents an integer of from 0 to 11),

comprising the step of:

contacting a compound having at least one of the structures represented by the following formulas (III) or (IV) with prenyltransferase to introduce a prenyl group into the structure,

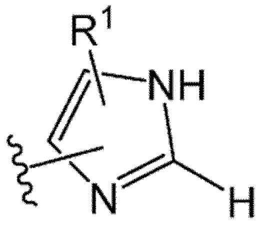

(III)

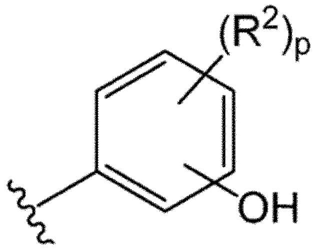

(IV)

(in formula (III) and formula (IV) above, $R^1$, $R^2$, and p are defined as in the foregoing),
wherein the prenyltransferase is LimF or an enzyme homologous thereto.

[2] The method according to [1], wherein a peptide or protein having an amino acid sequence containing at least one His, Tyr, or derivative thereof is contacted with the prenyltransferase, and a prenyl group is introduced into at least one His residue, Tyr residue, or derivative residue thereof.
[3] A method for producing a compound library containing prenylated peptides or proteins, comprising a step of:

contacting a compound library containing peptides or proteins having an amino acid sequence containing at least one His, Tyr, or derivative thereof with prenyltransferase to introduce a prenyl group to the at least one His residue, Tyr residue, or derivative residue thereof,
wherein the prenyltransferase is LimF or an enzyme homologous thereto.

[4] The method according to [3], further comprising translating an mRNA library through a cell-free translation system to prepare the compound library containing peptides or proteins having an amino acid sequence containing at least one His, Tyr, or derivative thereof.
[5] The method according to [3] or [4], wherein the peptides or the proteins in the compound library are bound to a genotype.
[6] The method according to any one of [5], further comprising a process for preparing the library of compounds containing the peptides or proteins that are bound to genotype by an mRNA display method,
wherein the process comprising steps of:

preparing an mRNA library of respective mRNA that encode the peptides or proteins having an amino acid sequence containing at least one His, Tyr, or derivative thereof;
binding puromycin to the 3' terminal of the respective mRNA in the mRNA library to produce a puromycin-bound mRNA library; and
translating the puromycin-bound mRNA library through the cell-free translation system.

[7] The method according to any one of [1] to [6], wherein the prenyltransferase comprises prenyltransferase derived from *Limnothrix* sp. or *Symploca* sp. or an enzyme homologous thereto.
[8] The method according to any one of [1] to [6], wherein the prenyltransferase comprises an enzyme containing an amino acid sequence that corresponds to the following (1), (2), or (3). In this aspect, the prenyltransferase may contain an enzyme containing an amino acid sequence corresponding to (4). The prenyltransferase may include an enzyme having an amino acid sequence corresponding to the following (1), (2), (3) or (4).

(1) an amino acid sequence represented by any of SEQ ID NOS: 1 to 12
(2) an amino acid sequence that has one or more amino acids deleted, replaced, or added in an amino acid sequence represented by any of SEQ ID NOS: 1 to 12
(3) an amino acid sequence that is 80% or more homology with the amino acid sequence represented by SEQ

ID NO: 1
(4) an amino acid sequence that is 80% or more homology with the amino acid sequence represented by SEQ ID NO: 2

[9] The method according to any one of [1] to [6], wherein the prenyltransferase is at least one selected from the group consisting of enzymes having an amino acid selected from the group consisting of the amino acid sequence represented by SEQ ID NO: 1, the amino acid sequence represented by SEQ ID NO: 2, and amino acid sequences that have one or a plurality of amino acids deleted, replaced, or added in these amino acid sequences.
[10] The method according to any one of [1] to [9], wherein the prenylated compound is a peptide or protein having a cyclic structure formed by four or more amino acids.
The method according to any one of [1] to [10], wherein the compound brought into contact with the prenyltransferase is a peptide or protein, wherein the peptide or protein has a substructure represented by Xaa1-Xaa2, or has an amino acid sequence that has His, Tyr, or a derivative thereof at the N-terminal, wherein Xaa1 is a neutral amino acid and Xaa2 is His, Tyr, or a derivative thereof.
The method according to any one of [1] to [11], wherein the compound brought into contact with the prenyltransferase is a peptide or protein, wherein the peptide or protein has a substructure represented by Xaa2-Xaa3, or has an amino acid sequence that has His, Tyr, or a derivative thereof at the C-terminal, wherein Xaa2 is His, Tyr, or a derivative thereof and Xaa3 is any amino acid other than Pro and a derivative thereof.
A compound library, produced by the method according to any one of [1] to [12].
A method for screening compounds to identify a compound that binds to a target substance, comprising steps of:

contacting the compound library produced by the production method according to any one of [1] to [12] with a target substance; and
selecting a compound that binds to the target substance.

## Effect of Invention

According to the present invention, it is possible to provide: a novel method for producing a prenylated compound, which can any of the problems described above; a novel method for producing a prenylated compound library; and the like.

## Brief Description of Drawings

[FIG. 1] A diagram showing an SSN showing a cluster of PTase obtained from Blastp analysis. (a) A diagram showing an SSN showing a cluster of KgpF obtained from a Blastp analysis using Ptase as a query. (b) A diagram showing an SSN showing a cluster of LimF obtained from a BLASTp analysis using Ptase as a query.
[FIG. 2] A diagram showing that an estimated cyanobactin gene cluster (BGC) derived from *Limnothrix* sp. CACIAM 69d includes LimF, which is a unique Ptase. (a) A phylogenetic analysis including LimF. For AgeMTPT, the Ptase domain region (275 to 559) was used for alignment. LimF, Ser/Thr O-Ptase, Tyr O-Ptase, Trp N/C-Ptase, and terminal N/O-Ptase are highlighted in orange (LimF), blue (LynF, TolF, TruF1), light blue (PirF, PagF), green (KgpF, AcyF), and gray (MusF1, MusF2, AgeMTPT), respectively. (b) Shows cyanobactin-related BGCs found in cyanobacteria *Limnothrix* sp. CACIAM 69d. The limE3 gene is encoded by a distant locus. (c) Shows a sequence alignment in which several known cyanobactin precursors are compared with an estimated precursor peptide (LimE1-3) found in limBGC. The recognition sequence of A-family protease (RSII) and the recognition sequence of G-family macrocyclic enzyme (RSII) are emphasized in blue. The core peptide (CP) region is emphasized in red.
[FIG. 3] A diagram showing that limnothamide, which is a geranylation product, was obtained by LimF treatment in a test tube of bcLimE2. (a) An LC-MS analysis of bcLimE2 before and after 12 hours of LimF treatment. The extracted ion chromatogram (EIC) shown includes all possible charge states of the substrate bcLimE2 and the geranylation product. Also shows a mass spectrum obtained by integrating peak regions observed by EIC. (b) The structure of limnothamide identified by NMR analysis. Illustrates COSY and HMBC correlations for identifying C-forward geranylation of His position at C2.
[FIG. 4] A diagram showing the MS/MS spectrum of (a) bcLimE2 and (b) bcLimE2 modified with LimF, and where these are located. Fragmentation types (b type and a type) have been noted.
[FIG. 5] Fig.5 shows the effects of (a) temperature, (b) pH, and (c) metal on a LimF-catalyzed bcLimE2 prenylation reaction. (d) Time-dependent analysis of LimF under standard conditions. (e) Shows the MgCh concentration dependence of LimF activity. (f) Shows the GPP concentration dependence of LimF activity. (g) Shows reaction rate of prenylation reactions facilitated by LimF performed under various bcLimE2 substrate concentrations.

[FIG. 6] Fig.6 shows results of His prenylation by LimF of various His-containing dipeptides (Ac-XH-$NH_2$ AND Ac-HX-$NH_2$). Note that the prenylation reaction was performed for 16 hours at 20 μM of LimF.

[FIG. 7] A diagram showing one-pot *in vitro* biosynthesis of prenylated teMP using a FIT-LimF system. (a) Illustrates expression and modification of teLimE2 in the FIT-LimF system. EICs containing all possible charge states of substrate teLimE2 and its corresponding prenylation products are shown. The modification efficiency calculated based on the area of the peak observed by EIC is shown on a chromatogram. (b) Illustrates -1-position mutation introduction of teLimE2. The bar graph shows the modification efficiency of variants in the LimF catalyst prenylation. The results of three independent experiments are shown as black dots, the mean is shown in a bar graph, and the standard deviation is shown in an error bar. (c) Illustrates + l-position mutation introduction of teLimE2. The asterisk indicates that His at -1 was modified. (d) Illustrates LimF modification of teMP of different ring sizel(e) Illustrates modification by LimF for teMP (teR1-5) having an arbitrarily selected whole sequence and at the -1 or +1 position variants thereof. (f) Illustrates -1- and +1-position mutations in various peptides. "y" in the sequence of the cyclic peptide means D-Tyr.

[FIG. 8] A diagram showing the results of one-pot *in vitro* biosynthesis of prenylated teMP using a FIT-LimF system. (a) Shows the prenylation efficiency of cyclic peptides having various random whole sequences by LimF. (b) Shows prenylation of cyclic peptide having two or more His residues by LimF. (c) Shows modification of His derivatives incorporated by genetic code reprogramming. Structural changes of each derivative residue compared to the His residue are emphasized in red.

[FIG. 9] A diagram showing Tyr O-prenylation by LimF. (a) Shows that the LimF modification of teR6 forms two different prenylation products. EICs containing all possible charge states of teR6, which is the substrate, and single prenylation products thereof, are shown. The peaks corresponding to His-prenylation and Tyr-prenylation products (teR6-$H^{Ger}$ and teR6-$Y^{Ger}$) are highlighted by dotted orange and light blue lines. (b) Shows the mass spectra of teR6-$H^{Ger}$ and teR6-$Y^{Ger}$. For teR6-$Y^{Ger}$, a peak resulting from the disappearance of the prenyl moiety is shown. (c) The structure of the prenylated tripeptide identified by NMR analysis. Illustrates 2D-NMR correlation for identifying Tyr O-prenylation is shown. (d) Shows change of prenylation by -1-position mutation introduction of teR6-H5A. The bar graph shows conversion efficiency by LimF. The asterisk indicates that His at -1 was prenylated.

[FIG. 10] Fig. 10 shows the effects of (a) temperature, (b) pH, and (c) metal on a prenylation reaction of teR6-H5A facilitated by LimF. Also shows the concentration dependence of (d) GPInd (e) MgCh on the prenylation reaction of teR6-HSA facilitated by LimF. (f) Shows the reaction rate of the prenylation reaction when the concentration of teR6-H5A is changed.

[FIG. 11] A diagram showing the prenylation efficiency of LimF and variants thereof for different reaction modes. The mean value of the modification efficiency in the three independent reactions is shown. Results showing marked His-prenylation and Tyr-prenylation are emphasized in orange (second row, columns 2 and 3; fifth row, column 2) and light blue (second row, columns 4 and 5; fifth row, columns 4 and 5; seventh row, column 5; eighth row, column 4 and 5), respectively.

[FIG. 12] A diagram showing prenylation of linear peptides of different lengths by LimF. A product having two prenylated His was also obtained from the linear peptide having the longest sequence.

[FIG. 13] A diagram showing prenylation of high molecular weight proteins (Ac-BSA and GST) by LimF. Shows the mass spectra of (a) Ac-BSA and (b) GST before and after prenylation by LimF, respectively.

[FIG. 14-1] Fig.14-1 shows a portion of the alignment results of SEQ ID NOS: 1 to 13.

[FIG. 14-2] Fig.14-2 shows a portion of the alignment results of SEQ ID NOS: 1 to 13.

[FIG. 15] Fig. 15 shows the results of (a) a serum stability test, (b) a DPP-4 digestion test, and (c) a cAMP production activation test of GLP-1 and derivatives thereof.

[FIG. 16] Fig.16 shows the relationship between the amino acid at the -1 position of His and the geranylation efficiency for LimF and variants thereof. The Q72A variant, the I52A variant, and the wild-type LimF are shown in the upper row, middle row, and lower row, respectively.

[FIG. 17] Fig. 17 shows the sequence alignment of a plurality of prenyltransferases.

[FIG. 18] Fig.18 shows relationship between the type of prenyl group donor and the prenylation efficiency for each LimF variant. The GPP (C10) and the DMAPP (C5) are shown in the upper row and the lower row, respectively.

[FIG. 19] Fig.19 shows a search for LimF variants that can be selectively farnesylated. The GPP (C10) and the FPP (C15) are shown in the upper row and the lower row, respectively.

[FIG. 20] Fig.20 shows the farnesylation efficiency of a non-natural substrate by the LimF_H239G/W273T variant.

## Embodiments of Invention

An embodiment of the present invention (hereinafter referred to "present embodiment") will be described below in detail, but the present invention is not limited thereto, and various modifications are possible without departing from the spirit thereof.

Note that in the present specification, a wavy line in chemical structural formulas mean the binding site of the group represented by that structural formula.

[Method for Producing Prenylated Compound]

The method for producing a compound according to the present embodiment is a method for producing a compound having at least one structure represented by formula (I) or (II), which includes a process wherein a compound having at least one structure represented by formula (III) or (IV) is contacting with a prenyltransferase to introduce a prenyl group into the structure, wherein the prenyltransferase is LimF or an enzyme homologous thereto.

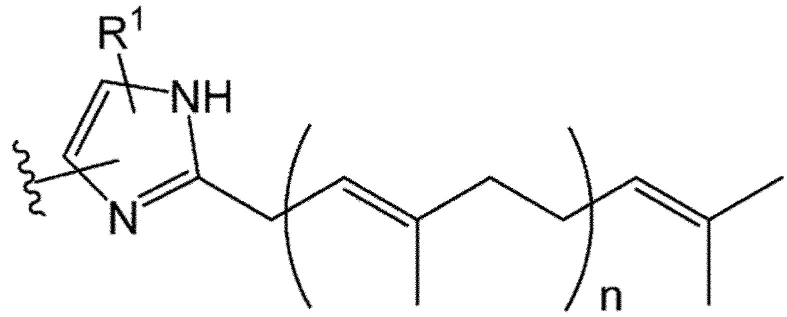

(I)

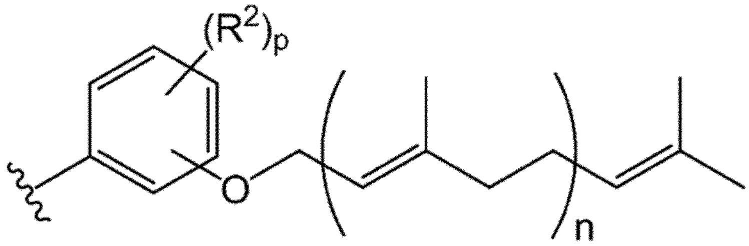

(II)

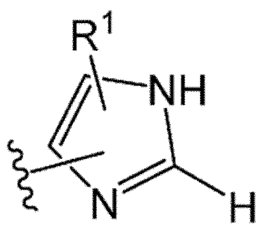

(III)

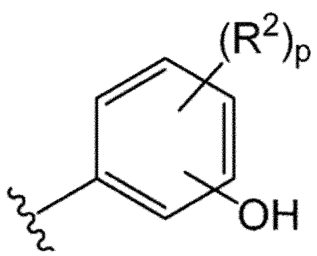

(IV)

Note that in formula (I), $R^1$ is a hydrogen atom or optional substituent, and n represents an integer of 0 to 11; in formula (II), each $R^2$ is independently optional substituent, p represents an integer of 0 to 4, and n represent an integer of 0 to 11; and in formula (III) and formula (IV), $R^1$, $R^2$, and p have the same definitions as in formulas (I) and (II).

According to the method for producing a compound of the present embodiment, a compound having high hydrophobicity can be produced by prenylating a compound having at least one structure represented by formula (III) or (IV) using a prescribed prenyltransferase. Furthermore, because LimF or an enzyme homologous thereto is used as the prenyltransferase in the method for producing the compound of the present embodiment, a variety of compounds

having prenyl groups and having high substrate tolerance for the prenyltransferase can be produced. As described above, through the method for producing the compound of the present embodiment, a prenyl group can be efficiently introduced into a variety of compounds, and a compound having latent high cell membrane affinity and/or high cell membrane permeability can be produced.

A process for obtaining a compound having at least one structure represented by the above formula (I) or (II) by bringing a compound having at least one structure represented by the above formula (III) or (IV) into contact with LimF, which is a prenyltransferase, or an enzyme homologous thereto, is referred to as the "prenylation process."

(Substrate)

The substrate in the prenylation process (hereinafter simply referred to as "substrate") is not particularly limited insofar as it is a compound having at least one structure represented by the above formula (III) or (IV).

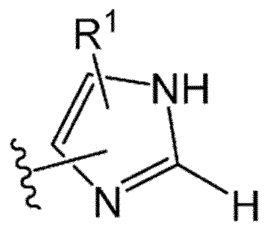

( I I I )

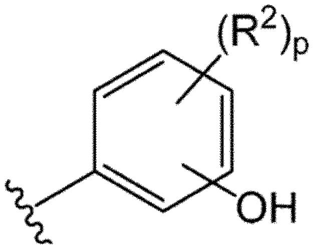

( I V )

In formula (III), $R^1$ is a hydrogen atom or optional substituent, and in formula (IV), each $R^2$ is independently optional substituent, and p represents an integer of 0 to 4.

The structure represented by the above formula (III) means a structure represented by either of formula (III-1) or formula (III-2). Furthermore, the structure represented by the above formula (IV) means a structure represented by any of the following formulas (IV-1), (IV-2), or (IV-3). In formulas (III-1) and (III-2), $R^1$ is synonymous with the definition for formula (III), and in formulas (IV-1), (IV-2), and (IV-3), $R^2$ and p are synonymous with the definitions for formula (IV).

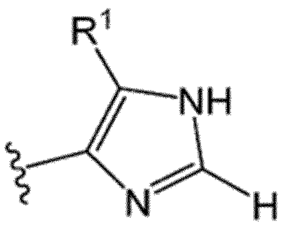

( I I I − 1 )

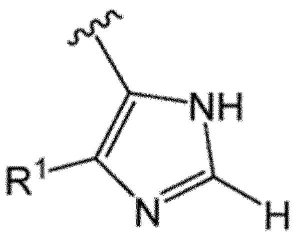

( I I I − 2 )

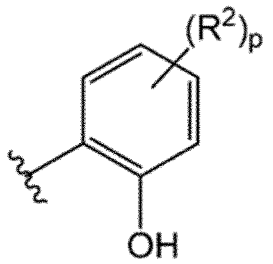

( I V − 1 )

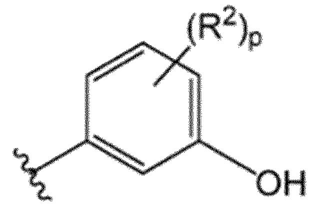

( I V − 2 )

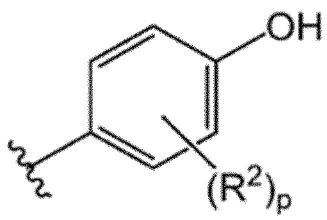

( I V − 3 )

In the substrate, the structure represented by the above formula (III) may be a structure represented by either of the above formula (III-1) or (III-2).

Furthermore, in the substrate, the structure represented by the above formula (IV) may be a structure represented by any of the above formulas (IV-1), (IV-2), and (IV-3), but is preferably a structure represented by formula (IV-3).

In the above formulas (III), (III-1), and (III-2), $R^1$ is a hydrogen atom or optional substituent. Examples of the optional substituent include saturated or unsaturated hydrocarbon groups, hydroxyl groups, alkoxy groups, carboxyl groups, aldehyde groups, amino groups, amide groups, azide groups, mercapto groups (thiol groups), sulfo groups, halogen groups, and heteroaryl groups. The number of carbon atoms in the optional substituent is not particularly limited, and is, for example, 0 or more and 20 or less, preferably 0 or more and 10 or less.

A saturated or unsaturated hydrocarbon group is preferable as a substituent group in $R^1$ of the above formulas (III), (III-1), and (III-2), where the number of carbons of the hydrocarbon group is preferably 1 or more and 10 or less, more preferably 1 or more and 5 or less, and further preferably 1 or more and 3 or less. The saturated or unsaturated hydrocarbon group is preferably an alkyl group.

Examples of preferable aspects of $R^1$ in the above formulas (III), (III-1), and (III-2) include hydrogen atoms and alkyl groups having 1 to 3 carbon atoms, and more preferable aspects include hydrogen atoms and methyl groups.

In the above formulas (IV), (IV-1), (IV-2), and (IV-3), each $R^2$ is independently optional substituent. Examples of the optional substituent include saturated or unsaturated hydrocarbon groups, hydroxyl groups, alkoxy groups, carboxyl groups, aldehyde groups, amino groups, amide groups, azide groups, mercapto groups (thiol groups), sulfo groups, halogen groups, and heteroaryl groups. The number of carbon atoms in the optional substituent is not particularly limited, and is, for example, 0 or more and 20 or less, preferably 0 or more and 10 or less.

A saturated or unsaturated hydrocarbon group is preferable as the substituent in $R^2$ of the above formulas (IV), (IV-1), (IV-2), and (IV-3), where the number of carbon atoms in the hydrocarbon group is preferably 1 or more and 10 or less, more preferably 1 or more and 5 or less, and further preferably 1 or more and 3 or less. The saturated or unsaturated hydrocarbon group is preferably an alkyl group.

In the above formulas (IV), (IV-1), (IV-2) and (IV-3), when there is a plurality of $R^2$s, they may be the same or different.

In the above formulas (IV), (IV-1), (IV-2), and (IV-3), p represents an integer of 0 to 4 (inclusive of both end values; in the present specification, the same applies in the numerical range represented by "to" ). When p is 0, the benzene ring in the above formulas (IV), (IV-1), (IV-2), and (IV-3) do not have a substituent $R^2$, and moieties other than the bonding site represented by the wavy line in the benzene ring and the hydroxyl group in the formulas are hydrogen

atoms.

In the above formulas (IV), (IV-1), (IV-2), and (IV-3), p is preferably 0 to 3, more preferably 0 to 2, further preferably 0 or 1, and even more preferably 0.

The substrate is preferably a compound having at least one structure represented by formula (III') or (IV').

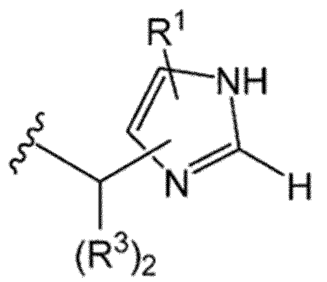

(III')

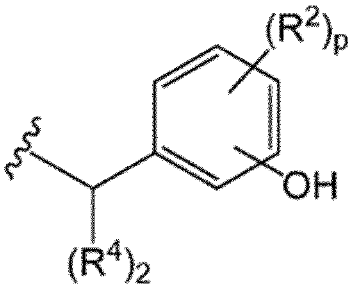

(IV')

In above formula (III'), $R^1$ is synonymous with $R^1$ in the above formula (III), each $R^3$ is independently a hydrogen atom or optional substituent; in the above formula (IV'), $R^2$ and p are synonymous with $R^2$ and p in the above formula (IV); and each $R^4$ is independently a hydrogen atom or optional substituent.

In the structure represented by the above formula (III'), the bonding position in the imidazole ring of $R^1$ is not particularly limited, that is, the structure represented by the above formula (III') may have a structure that corresponds to the above formulas (III-1) and (III-2) in the above formula (III).

In the structure represented by the above formula (IV'), the bonding position of the hydroxyl group in the benzene ring may be any of the ortho position, the meta position, or the para position, that is, the structure represented by the above formula (IV) may have a structure corresponding to the above formulas (IV-1), (IV-2), and (IV-3) in the structure represented by the above formula (IV). In the structure represented by the above formula (IV'), the bonding position of the hydroxyl group in the benzene ring is preferably the para position.

Examples, preferable aspects, and the like (aspects listed as preferable, more preferable, further preferable, even more preferable, and the like; hereinafter, the same is true in the present specification) of $R^1$ in the above formula (III') are the same as for $R^1$ in the above Formula (III). Furthermore, examples, preferable aspects, and the like of $R^2$ and p in the above formula (IV') are the same as for those of $R^2$ and p in the above formula (IV).

$R^3$ in the above formula (III') and $R^4$ in the above formula (IV) are each independently a hydrogen atom or optional substituent. Examples of the optional substituent include saturated or unsaturated hydrocarbon groups, hydroxyl groups, alkoxy groups, carboxyl groups, aldehyde groups, amino groups, amide groups, azide groups, mercapto groups (thiol groups), sulfo groups, halogen groups, and heteroaryl groups. The number of carbon atoms in the optional substituent is not particularly limited, and is, for example, 0 or more and 20 or less, preferably 0 or more and 10 or less. For the two $R^3$s and two $R^4$s, each $R^3$ and $R^4$ may be the same or different.

A saturated or unsaturated hydrocarbon group is preferable as the substituent in $R^3$ of the above formula (III') and $R^4$ in the above formula (IV'), where the number of carbons of the hydrocarbon group is preferably 1 or more and 10 or less, more preferably 1 or more and 5 or less, and further preferably 1 or more and 3 or less. The saturated or unsaturated hydrocarbon group is preferably an alkyl group.

Examples of preferable aspects of $R^3$ in the above formula (III') and $R^4$ in the above formula (IV) include hydrogen atoms and alkyl groups having 1 to 3 carbon atoms, and more preferable aspects include hydrogen atoms and methyl groups.

The two $R^3$s in the above formula (III') may be the same or different. The two $R^4$s in the above formula (IV') may be the same or different.

Preferable aspects of the substrate include a compound having at least one structure where $R^1$ in the above

formula (III') is a hydrogen atom, methyl group, or ethyl group, and the two $R^3$s are hydrogen atoms. Furthermore, preferable aspects of the substrate include a compound having at least one structure where p is 0 in the above formula (IV'), the two $R^4$s are hydrogen atoms, and a hydroxyl group is bound to the para position.

The substrate in the prenylation process is not particularly limited insofar as it is a compound having at least one structure represented by the above formula (III) or (IV) and may be a peptide or a protein, or a compound other than that. The substrate is typically a peptide, a protein, or a low molecular weight compound (including amino acid monomer derivatives). In the present specification, "low molecular weight compound" means a compound having a molecular weight of 1,000 or less.

The substrate is preferably formed such that the surrounding environment of the structure represented by the above formula (III) or (IV) is an environment that can interact with a prenyltransferase.

The substrate in the prenylation process is preferably a peptide or protein having at least one structure represented by the above formula (III) or (IV). In the present specification, "peptide" means a compound in which 2 to 50 amino acids are bound by peptide bonds, and "protein" means a compound in which 51 or more amino acids are bound by peptide bonds. Furthermore, "peptide or protein" in the present specification means a compound in which two or more amino acids are bound by peptide bonds, and the number of amino acids bound is not particularly limited.

In the present specification, "amino acid" is used in its broadest sense and includes, in addition to natural amino acids, artificial amino acid variants and derivatives. Furthermore, the term "amino acid" in the present specification includes proteinogenic amino acids and non-proteinogenic amino acids

When represented in the three letter notation well-known in the industry the proteinogenic amino acids are Arg, His, Lys, Asp, Glu, Ser, Thr, Asn, Gln, Cys, Gly, Pro, Ala, Ile, Leu, Met, Phe, Trp, Tyr, and Val. Furthermore, when represented in the one letter notation well-known in the industry the proteinogenic amino acids are R, H, K, D, E, S, T, N, Q, C, G, P, A, I, L, M, F, W, Y, and V.

Non-proteinogenic amino acids mean natural or non-natural amino acids other than the proteinogenic amino acids.

Examples of amino acids and derivatives thereof include natural proteinogenic L-amino acids, non-natural amino acids, and chemically synthesized compounds having properties well-known in the industry as properties of amino acids.

Examples of non-natural amino acids include, but are not limited to: amino acids having a main chain structure different from that of the natural type such as $\alpha,\alpha$-disubstituted amino acids ($\alpha$-methylalanine and the like), N-alkyl-$\alpha$-amino acids, D-amino acids, $\beta$-amino acids, and $\alpha$-hydroxy acids; amino acids having a side chain structure is different from that of the natural type (norleucine, homohistidine, and the like); amino acids having an excess methylene in a side chain ("homo" amino acids, homophenylalanine, homohistidine, and the like); and amino acids in which a carboxylic acid functional group amino acid in the side chain is replaced with a sulfonic acid group. Specific examples of non-natural amino acids include the amino acids taught in WO 2015/030014.

Furthermore, amino acid derivatives include the aspects protected by protecting groups described later.

When the substrate is a peptide or a protein, the number of amino acids forming the substrate is not particularly limited and may be, for example, 2 or more and 2,000 or less. The number of amino acids forming the substrate may be 3 or more, 4 or more, 5 or more, 6 or more, or 10 or more, and may be 1,500 or less, 1,000 or less, 800 or less, 600 or less, 500 or less, 400 or less, 300 or less, 200 or less, 100 or less, 90 or less, 80 or less, 70 or less, 60 or less, 55 or less, 50 or less, 45 or less, 40 or less, 30 or less, or 20 or less within the above range. The number of amino acids forming the substrate may be, for example, 2 or more and 600 or less, 2 or more and 300 or less, 2 or more and 80 or less, or 2 or more and 60 or less. The number of amino acids that form the substrate may be within the above ranges and may be within a range that is obtained by any combination of the above upper limit values and lower limit values.

When the substrate is a peptide or a protein, the primary structure thereof is not particularly limited and may be, for example, a straight chain or may contain a cyclic moiety. The peptide or protein that is the substrate may be a "lasso type" in which a straight chain peptide is attached to a cyclic structure like a tail. The substrate may have a structure other than that of a peptide or a protein. From the perspective of enhancing target binding and/or cell membrane permeability and/or bioavailability, the substrate is preferably a cyclic peptide.

In the present specification, a peptide or protein having a "cyclic structure" or a "cyclic moiety" means having a closed ring structure formed by two amino acids that are separated by one or more amino acid residues in an amino acid sequence being bound directly or via a linker or the like.

When the substrate is a peptide or protein including a cyclic structure, the closed ring structure in the cyclic structure is not particularly limited, and the two amino acids may be covalently bound via a linker or the like as necessary.

Examples of covalent bonds between the two amino acids include disulfide bonds, peptide bonds, alkyl bonds, alkenyl bonds, ester bonds, thioester bonds, ether bonds, thioether bonds, phosphonate ether bonds, azo bonds, C-S-C bonds, C-N-C bonds, C=N-C bonds, amide bonds, lactam crosslinking, carbamoyl bonds, urea bonds, thiourea bonds, amine bonds, thioamide bonds, and the like. Furthermore, the closed ring structure may be a $N-CO-CH_2-S$ structure or another structure formed by an amino acid having a functional group 1 shown in Table 4 that will be described below

and an amino acid having a corresponding functional group 2.

Moreover, the covalent bond between the two amino acids may be formed by side chains of two amino acids, main chains of two amino acids, a bond between a side chain and a main chain of two amino acids, or the like. When two amino acids are bound to each other in main chains of the amino acids, the closed ring structure is typically formed by peptide bonding.

The closed ring structure that may be included in the peptide or the protein is not limited to a bond between an N-terminal amino acid and a C-terminal amino acid of linear peptides, but may be formed by binding between a terminal amino acid and an amino acid other than terminal amino acids or binding between amino acids other than terminal amino acids. When one of the amino acids bound for cyclic structure formation is a terminal amino acid and the other is a non-terminal amino acid, the cyclic peptide has a lasso-type structure.

When the peptide or protein is a lasso-type, it is preferable that a linear peptide is bound to the C-terminal of the amino acid constituting the cyclic structure. In other words, it is preferable that the N-terminal amino acid and a side chain of the amino acid other than the C-terminal in the linear peptide bond to form a cyclic structure.

The number of amino acid residues that form the cyclic structure of the peptide or protein is not particularly limited insofar as there are 3 or more, but such may be, for example, 4 or more, 5 or more, 6 or more, 7 or more, 8 or more, 9 or 10 or more, and may be 60 or less, 50 or less, 40 or less, 30 or less, 25 or less, 20 or less, 18 or less, or 15 or less.

When the substrate is a peptide that contains a cyclic structure (also referred to as a "cyclic peptide" in the present specification), the number of amino acids that form the cyclic structure is normally 3 or more and 40 or less. The number of amino acids that form the cyclic structure may be 4 or more, 5 or more, 8 or more, or 10 or more, and may be 30 or less, 25 or less, 20 or less, or 18 or less, within the above range.

One preferable aspect of the substrate in the prenylation process is a cyclic peptide having an amino acid sequence including, for example, at least one His, Tyr, or derivative thereof, and the number of amino acids forming the cyclic structure is preferably 3 or more and 30 or less, more preferably 4 or more and 20 or less.

The peptide or protein having at least one structure represented by the above formula (III) or (IV) is a peptide or protein having an amino acid sequence containing at least one His, Tyr, or derivative thereof.

In the present specification, examples of His derivatives include: derivatives having the following structure

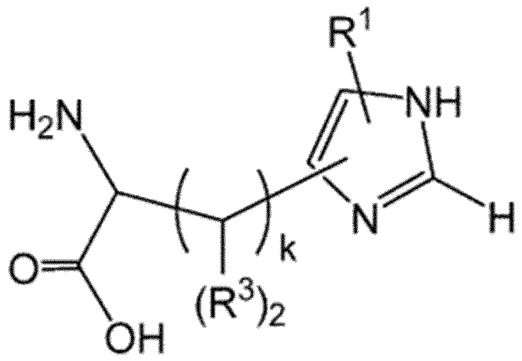

(V)

derivatives thereof and derivatives corresponding to β-amino acids, γ-amino acids, and δ-amino acids of His; and derivatives that correspond to α-alkyl-amino acids (for example, α-alkyl-amino acids having an alkyl group with 1 to 3 carbon atoms, and typically α-methyl-amino acids) of these derivatives.

In the above formula (V), $R^1$ and $R^3$ are synonymous with $R^1$ and $R^3$ in the above formula (III'), respectively, and k represents an integer of 0 to 3.

In the above formula (V): $R^1$ is preferably a hydrogen atom or an alkyl group having 1 to 3 carbon atoms, more preferably a hydrogen atom or a methyl group; each $R^3$ is independently a hydrogen atom, preferably an alkyl group having 1 to 3 carbon atoms, more preferably a hydrogen atom or a methyl group, and further preferably a hydrogen atom; and k preferably represents 1 or 2. Note that when k is 2, the above formula (V) corresponds to a homo amino acid. However, the derivative of His excludes those having the same structure as that of His.

In the present specification, examples of Tyr derivatives include: derivatives having the following structure

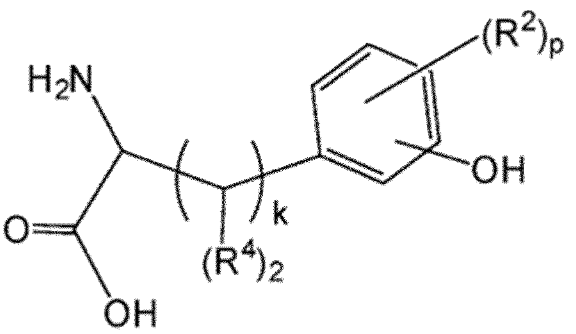

(VI)

derivatives thereof and derivatives corresponding to β-amino acids, γ-amino acids, and δ-amino acids of Tyr; and derivatives that correspond to α-alkyl-amino acids (for example, α-alkyl-amino acids having an alkyl group with 1 to 3 carbon atoms, and typically α-methyl-amino acids) of these derivatives.

In the above formula (VI), $R^2$, $R^4$, and p are synonymous with $R^2$, $R^4$, and p in the above formula (IV'), respectively.

In the above formula (VI): each $R^2$ is, independently, preferably an alkyl group having 1 to 3 carbon atoms, more preferably a methyl group; p is preferably 0 to 2, more preferably 0 or 1, and further preferably 0; each $R^4$ is, independently, preferably a hydrogen atom or an alkyl group having 1 to 3 carbon atoms, more preferably a hydrogen atom or a methyl group, and further preferably a hydrogen atom; the hydroxyl group in the benzene ring is preferably located at the para position; and k preferably indicates 1 or 2. Note that when k is 2, the above formula (VI) corresponds to a homo amino acid. However, the derivative of Tyr excludes those having the same structure as that of Tyr.

Note that in the peptide or protein, the His, Tyr, or the derivative thereof may be the L-form or the D-form. Furthermore, His and Tyr derivatives respectively include a form in which His and derivatives thereof and Tyr and derivatives thereof are protected by protecting groups described later.

The number of His, Tyr, or derivatives thereof in the peptide or protein having an amino acid sequence containing at least one His, Tyr. or derivative thereof as a substrate is not particularly limited. The number of His, Tyr, or derivatives thereof may be 1 or more to 10 or less and, within the above range, may be 9 or less, 8 or less, 7 or less, 6 or less, 5 or less, 4 or less, or 3 or less and may be 1 or more, 2 or more, 3 or more, or 4 or more.

From among His, Tyr, and derivatives thereof, the peptide or protein may contain only His or a derivative thereof, may contain only Tyr or a derivative thereof, or may contain both His or a derivative thereof and Tyr or a derivative thereof.

When the substrate in the prenylation process is a peptide or a protein having an amino acid sequence containing at least one His, Tyr, or derivative thereof, the sequence before and after His, Tyr, or a derivative thereof is not particularly limited.

In the peptide or protein that is the substrate, an amino acid on the N-terminal side adjacent to the His, Tyr, or derivative thereof: preferably, is either not present or is an amino acid other than Lys, Arg, and derivatives thereof (limited to those having a positive charge in a side chain); more preferably, is either not present or is a neutral amino acid or an amino acid showing less steric hindrance; further preferably, is either not present or is a neutral amino acid; and even more preferably, is either nor present or is neutral and has less steric hindrance. That is, it is preferable that the peptide or protein either (1) has a substructure represented by Xaa1-Xaa2 or (2) has an amino acid sequence having His, Tyr, or a derivative thereof at the N-terminal. Here, Xaa1 is an amino acid other than Lys and Arg and is preferably an amino acid other than Lys and Arg and derivatives thereof (limited to those having a positive charge in a side chain), more preferably a neutral amino acid or an amino acid having less steric hindrance, further preferably a neutral amino acid, and even more preferably an amino acid having neutral and lesssteric hindrance. Furthermore, Xaa2 is His, Tyr, or a derivative thereof.

In the peptide or protein that is the substrate, an amino acid on the C-terminal side adjacent to His, Tyr, or a derivative thereof: preferably, is either not present or is an amino acid other than Pro and derivatives thereof (limited to those whose side chains form a ring by binding to a nitrogen atom of an amino group); and more preferably, is either not present or is an amino acid other than those whose side chains form a ring by binding to a nitrogen atom of an amino group. That is, it is preferable that the peptide or protein either (1) has a substructure represented by Xaa2-Xaa3 or (2) has an amino acid sequence having His, Tyr, or a derivative thereof at the C-terminal. Here, Xaa2 is His, Tyr, or a derivative thereof. Furthermore, Xaa3 is an amino acid other than Pro and is preferably an amino acid other than Pro and derivatives thereof (limited to those in which a side chain bonds to a nitrogen atom of an amino group to form a ring), more preferably an amino acid other than those in which a side chain bonds to a nitrogen atom of an amino group to form a ring.

In the present specification, "neutral amino acid" means an amino acid that does not have a basic or acidic side chain in the molecule, and when exemplified by proteinogenic amino acids, examples include Gly, Ala, Phe, Leu, Ile, Cyc, Met, Tyr, Val, Thr, Ser, Pro, Trp, Asn, Gln, and the like.

Furthermore, "amino acids with less steric hindrance" means amino acids whose side chains rise less steric hindrance, and examples include amino acids where the number of atoms other than the hydrogen atoms that constitute the side chain is four or less. Examples of proteinogenic amino acids include Gly, Ala, Ser, Pro, Val, Thr, Cys, Leu, Ile, Asn, Asp, Met, and the like.

Furthermore, "an amino acid that is neutral and has less steric hindrance" means an amino acid that corresponds to a neutral amino acid as in the above definition and an amino acid that has less steric hindrance, and when exemplified by proteinogenic amino acids examples include Gly, Ala, Ser, Pro, Val, Thr, Cys, Leu, Ile, Asn, Met, and the like.

Furthermore, "an amino acid in which a side chain bonds to a nitrogen atom of an amino group to form a ring," for example, is an amino acid having a structure such as the following

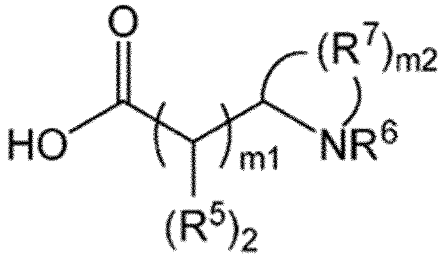

($R^5$ and $R^6$ are each independently a hydrogen atom or optional substituent; each $R^7$ is independently a given divalent group (typically a methylene group), m1 represents an integer of 0 or more, and m2 indicates an integer of 1 or more; m1 is typically 0 or 1), and when exemplified by proteinogenic amino acids, examples include Pro.

When an amino acid on the N-terminal side adjacent to His, Tyr, or a derivative thereof is present in the peptide or protein, preferable proteinogenic amino acids include Thr, Ala, Met, Pro, Ser, Val, Ile, Leu, Gly, Asn, Asp, His, Gln, Glu, Phe, Cys, Trp, and Tyr, more preferably Thr, Ala, Met, Pro, Ser, Val, Ile, Leu, Gly, Glu, Asp, His, Gln, and Asn, further preferably Thr, Ala, Gly, Ser, Val, Leu, Asn, Met, Pro, and Ile.

When an amino acid on the C-terminal side adjacent to His, Tyr, or a derivative thereof is present in the peptide or protein, preferable proteinogenic amino acids include Arg, His, Lys, Asp, Glu, Ser, Thr, Asn, Gln, Cys, Gly, Ala, Ile, Leu, Met, Phe, Trp, Tyr, and Val.

The substrate in the prenylation process may be a peptide or a protein to which a modification such as phosphorylation, methylation, acetylation, adenylylation, ADP ribosylation, glycosylation, polyethylene glycol addition, or the like is made, or two or more types of peptides and/or proteins may be fused directly or via a linker or the like. The peptide or protein may be biotinylated via a linker or the like.

The substrate may be a compound in which a structure other than an amino acid is bound to a peptide or a protein by a covalent bond or a non-covalent bond. Examples of the compound in which a structure other than an amino acid is bound to a peptide or a protein include a compound in which a nucleic acid molecule that is a genotype of the peptide or protein is bound to the peptide or protein directly or through a linker, a compound in which a label (fluorescent substance, radioactive substance, metal nanoparticle, quantum dot, enzyme, or the like) for detecting the peptide or protein is bound to the peptide or protein directly or through a linker, and the like.

The linker bound to the peptide or protein is not particularly limited, and a linker or the like having a structure well-known in the field of peptide synthesis as a linker that links peptides may be used.

The substrate in the prenylation process may be a compound that does not correspond to a peptide or a protein, and a preferable aspect of such a compound is a low molecular weight compound having at least one structure represented by the above formula (III) or (IV). Examples of such a low molecular weight compound include His, Tyr, or an isolated derivative thereof.

Examples of His derivatives and Tyr derivatives include those described above.

The His, Tyr, or derivative thereof may be an L-form or a D-form. Furthermore, the His, Tyr, or derivative thereof may be any of an $\alpha$-amino acid, $\beta$-amino acid, $\gamma$-amino acid, or $\delta$-amino acid, but preferably an $\alpha$-amino acid.

The substrate in the prenylation process may be His, Tyr, or an isolated derivative thereof, in which at least one of an amino group, a carboxyl group, and a side chain functional group is protected by a protective group. The protecting group is not particularly limited insofar as it is generally used in the industry.

Examples of amino acid protecting groups include a benzyloxycarbonyl (Cbz) group, a tert-butoxycarbonyl (Boc) group, a fluorenylmethoxycarbonyl (Fmoc) group, a benzyl group, an allyl group, an allyloxycarbonyl (Alloc) group, and the like.

Examples of carboxyl group protective groups include a methyl group, an ethyl group, a benzyl group, a tert-butyl group, a cyclohexyl group, and the like.

Regarding other functional groups of the amino acid, examples of protecting groups of hydroxyl groups in

serine, threonine, and the like include benzyl groups and tert-butyl groups, and examples of protecting groups of hydroxyl groups in tyrosine include 2-bromobenzyloxycarbonyl groups and tert-butyl groups.

The protection and deprotection of protecting groups may be carried out in accordance with the methods described in Protective Groups In Organic Synthesis Second Edition by T. W. Greene and P. G. M. Wuts John Wiley&Sons, Inc. or equivalent methods.

The His, Tyr, or isolated derivative thereof may be that in which only an amino group, only a carboxyl group, or only a functional group in another side chain is protected by a protecting group, or that in which two or more or all of the amino group, the carboxyl group, and the functional groups in another side chain are protected by a protecting group. The His, Tyr, or isolated derivative thereof may be, for example, that in which the amino group is protected by Fmoc.

The low molecular weight compound other than His, Tyr, or isolated derivatives thereof is not particularly limited insofar as it is a low molecular weight compound having at least one structure represented by the above formula (III) or (IV), but, for example, compounds where pharmacological activity has already been discovered are preferable. By using such a compound as the substrate and producing a prenylated compound by the method of the present embodiment, it is possible to increase the pharmacological activity that has already been discovered and to improve cell membrane affinity and/or cell membrane permeability and/or bioavailability.

Examples of such a low molecular weight compound include cimetidine, which has the structure shown below.

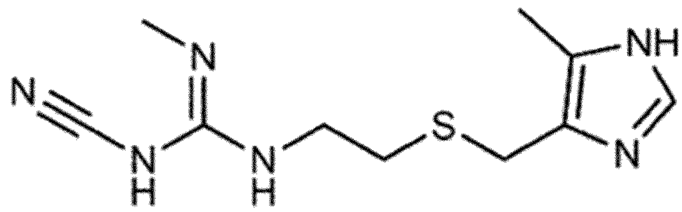

Furthermore, examples of low molecular weight compounds that can be the substrate include compounds having the following structure.

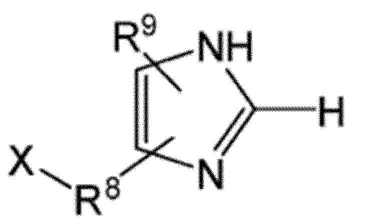

In the above structure, $R^8$ is a divalent hydrocarbon group which may contain a heteroatom, $R^9$ is a hydrogen atom or optional substituent group, and X is any atomic group.

In the above structure, $R^8$ preferably has 1 to 15 carbon atoms and more preferably has 1 to 10 carbon atoms. The heteroatoms that $R^8$ may include are not particularly limited and are, for example, nitrogen atoms, sulfur atoms, and oxygen atoms.

In the above structure, $R^9$ represents a hydrogen atom or optional substituent. Examples of the optional substituent include saturated or unsaturated hydrocarbon groups, hydroxyl groups, alkoxy groups, carboxyl groups, aldehyde groups, amino groups, amide groups, azide groups, mercapto groups (thiol groups), sulfo groups, halogen groups, and heteroaryl groups, and the like. The number of carbon atoms in the optional substituent is not particularly limited, and is, for example, 0 or more and 20 or less, preferably 0 or more and 10 or less. A saturated or unsaturated hydrocarbon group is preferable as the substituent group in $R^9$, where the number of carbons of the hydrocarbon group is preferably 1 or more and 10 or less, more preferably 1 or more and 5 or less, and further preferably 1 or more and 3 or less. The saturated or unsaturated hydrocarbon group is preferably an alkyl group. Examples of preferable aspects of $R^9$ include hydrogen atoms and alkyl groups having 1 to 3 carbon atoms, and more preferable aspects include hydrogen atoms and methyl groups.

In the above structure, X is any atomic group. The number of atoms contained in X is not particularly limited and may be, for example, 1 or more and 20 or less, or 2 or more and 10 or less. The types of atoms included in X are not particularly limited and may include, for example, at least one of carbon atoms, hydrogen atoms, nitrogen atoms, oxygen atoms, sulfur atoms, and phosphorus atoms. The atomic group X may be a moiety mainly contributing to the pharmacological activity of the compound represented by the above formula.

The above substrate may be subjected to a prenylation process by alone or two or more.

Furthermore, the substrate may be in the form of a pharmacologically acceptable salt of any of the compounds

described above.

In the present specification, "pharmacologically acceptable salt" includes, for example, a salt with a base or acid that is acceptable as a pharmaceutical.

Non-limiting specific examples of pharmacologically acceptable salts include addition salts of inorganic acids (hydrochloric acid, hydrobromic acid, hydroiodic acid, sulfuric acid, phosphoric acid, and the like), addition salts of organic acids (p-toluenesulfonic acid, methanesulfonic acid, oxalic acid, p-bromophenyl sulfonic acid, carboxylic acid, succinic acid, citric acid, benzoic acid, acetic acid, and the like), addition salts of inorganic bases (ammonium hydroxide or an alkali or alkaline earth metal hydroxide, carbonate, bicarbonate, and the like), and addition salts of amino acids.

(Prenyltransferase)

The prenylation process is a process for contacting any substrates with a prenyltransferase (PTase), and the prenyltransferase is LimF or an enzyme homologous thereto.

The present inventors discovered that, as in the examples described later, LimF and an enzymes homologous thereto can prenylate any of the above substrates and can catalyze the prenylation of a variety of substrates. It is also found that LimF and enzymes homologous thereto are novel prenyltransferases that catalyze novel C-prenylation.

LimF is a prenyltransferase derived from *Limnothrix* sp. CACIAM 69d and is an enzyme having the amino acid sequence of SEQ ID NO: 1 (listed in Table 1 below described later).

In the present specification, "prenyltransferase" means an enzyme that can bind a prenyl group composed of am isoprene unit having five carbons to a substrate. In the present specification, "prenyl group" is a collective term for monovalent substituents represented by the following structure

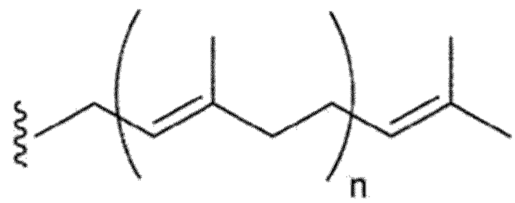

and in the above formula, n represents an integer of 0 to 11. In the above formula, when n is 0, 1, 2, 3, 4, 5, 7, or 9, the groups represented by the above formula are also referred to as dimethyl allyl groups, geranyl groups, farnesyl groups, geranylgeranyl groups, geranylfarnesyl groups, hexaprenyl groups, octaprenyl groups, and decaprenyl groups, respectively. Furthermore, these are sometimes referred to as Cn prenyl groups according to a number of carbons n. For example, the dimethylallyl group, the geranyl group, and the farnesyl group are also referred to as a C5 prenyl group, a C10 prenyl group, and a C15 prenyl group, respectively. Therefore, in the present specification, the term "prenyl group" means including a dimethylallyl group, geranyl group, farnesyl group, geranylgeranyl group, geranylfarnesyl group, hexaprenyl group, octaprenyl group, decaprenyl group, and the like, except for specifically referring to a specific group.

The enzyme homologous to LimF is not particularly limited, but, for example, a protein having an amino acid sequence in which homology with the amino acid sequence of LimF is observed as a result of preparing a sequence alignment using analysis by ClustalW and MEGA7 software and performing phylogenetic analysis can be suitably used. Proteins with amino acid sequences for which homology was observed as a result of the analysis also include proteins annotated as hypothetical proteins.

In addition to enzymes having an amino acid sequence in which one or a plurality of amino acids are deleted, replaced, or added in the amino acid sequence of LimF (SEQ ID NO: 1), examples of enzymes homologous to LimF include enzymes having an amino acid sequence of at least 30%, 40% or more, 50% or more, 60% or more, 70% or more, 75% or more, 80% or more, 85% or more, 90% or more, 92% or more, 95% or 98% or more, or 99% or more homologous to the amino acid sequence of LimF (SEQ ID NO: 1) and having prenyl group transfer activity.

Examples of such enzymes homologous to LimF include enzymes including or having an amino acid sequence represented by any of SEQ ID NOS: 2 to 12 shown in Table 1 (shown as Table 1-1 and 1-2) below. The enzyme having an amino acid sequence of SEQ ID NOS: 2 to 12 are enzymes that, as a result of the phylogenetic analysis and sequence similarity network (sequence similarity network) analysis described in the examples, are predicted to be highly homologous to LimF and have prenyl group transfer activity similar to LimF.

Note that in Table 1, the amino acid sequence represented by SEQ ID NO: 1 is the amino acid sequence of LimF, and SEQ ID NO: 13 is an amino acid sequence in which His at position 172 of LimF is replaced by Leu.

Table 1-1

| SEQ ID NO | Remarks | Amino Acid Sequence | Accession | Organism |
|---|---|---|---|---|
| 1 | LimF | MKKRKSSKVFKSTIAPEEKLRYIGNHKQAFDIEPLYPLALFEEFVATTGDCIIECSGKIKQDQLYPARIDLQFSDKHHFHNIHTSIDFL KRAASRTDVNLNLDILATFLAGNFDYSKVQNILAGIDLRQNLGESKLKLFIRIGDYPAKMAVAKHLCNITPESEAMLRSDTLHIGFDF YLDGRSAIELYPELKKDEFNHPFIYNQLKTILSPEALKPLPLCNLFGIGLSPANEANVLYYHLENIEDFLSYFPINDTARRVHDFYLQ QEGSRRMWVALSESEMKAGRINNVNLYYSKAFTSQNP | RFP52075.1 | Limnothrix sp. CACIAM 69d |
| 2 | | MNQSRDKLLYIGRHKSAFDIDYLYPMDIFEQFVESTGDGLIECSGKIKQDKLYPARFDLQFSDRQYAKNIYASIDFLKRVATRVDVS LDLNLLEQFLGNDFDFSKVQTVLVGVDLRHELHESRLKLFIRIGDYPEKVTLAKKLCGDSPQLQQLLRYDTLHIGFDFNLDGRSAIEL YPEVKKEEFKQPEVYQKLTEILSLAALKPLAISNLMGIGFSQANEAKVLYYHLENIRDFLNYFPINNVARRVHDFYLQQDGSKKM WVALSEAELIAGKINNVNLYYSKAFF | NER99685.1 | Symploca sp. SIO1B1 |
| 3 | | MTTLLESGKKIYYIGIHKQIFEIKNFYPLDIFDSFVNQIETTSENCSLESSCKIELDKLYPARFGIGFTLKNLKQLNVVYEFFQKVESRI DVQINYSLIQQFFGENFDFNKMTEFMVGIDARQELSETKLKIALTIKNYPEKIKTAIALNGGLDKNIYNLLVSNSLHIGFDLSLDGRSEI ELYPYIRNQEFQIFDIQQRLATVLSPQALQFLPICSRICVGLSKANADKVVYFYLKNLNDFLNYFTVNDTARRVHAYYQQQPMREM CVAVQEKQLLGGTIEKMNLYYLI | WP_ 035367793.1 | Dolichospermum circinale |
| 4 | | MTTLLLQRGKEIYYIGIHKQIFEIKNFYPLDIFDSFVTQIETTSENCYLESSCKIERDKLYPARFGIGFTLKNLKQLNVVYEFFQKVESRI DVQINYSLIQQFFGEDFDFNKMTEFMVGVDARPELAESKLKIALTIKDYPEKIKTAIELNGGLDKNIYNLLVSNSLHIGFDLSLDGRSE IELYPYIRNQEFQIFDIQQRLATVLLPQSLQFLPICSRICVGLSKANADKVVYFYLKNLNDFLNYFTVNDTARRVHAYYQQQPMREM CVAVQEKQLLGGTIEKMNLYYLI | QSV52791.1 | Dolichospermum sp. UK1201 |
| 5 | | MKSNKKLYYISAHKHAFEIDNLYPLNLFEGFVERIEKIEKTENCVLESSCKIDHDKLYPVRFNIGFPNNSIKQLHAVMDFFRRVESR VDVKLNLSLFQQFIGNDFKLDKMTDLMLGIDLRRDLSDSRLKIGLTIEDYPEKQKAAVILNNNIDEVTSNLLISNRLHIGFDFYLNGRS EMELYPHIMQQDFQKLDVQQRLSKVLSPPALQVVPACTRICVGISKANRDKIIYYYLENMGDFLNYFTVNDTARKVHAYYLKQPV VEMCVALPESELLAGTTIKNLNLYYLL | WP_ 192846042.1 | Microcystis aeruginosa |
| 6 | | MLKSNKKLYYISAHKHAFEIDNLYPLNLFEGFVERIEKIEKTENCVLESSCKIDHDKLYPVRFNIGFPNNSIKQLHAVMDFFRRVESR VDVKLNLSLFQQFIGNDFKLDKMTDLMLGIDLRRDLSDSRLKIGLTIEDYPEKQKAAVILNNNIDEVTSNLLISNRLHIGFDFYLNGRS EMELYPHIMQQDFQKLDVQQRLSKVLSPPALQVVPACTRICVGISKANRDKIIYYYLENMGDFLNYFTVNDTARKVHAYYLKQPV VEMCVALPESELLAGTTIKNLNLYYLL | BCR82584.1 | Microcystis aeruginosa |

| SEQ ID NO | Remarks | Amino Acid Sequence | Accession | Organism |
|---|---|---|---|---|
| 7 | | MTTLLQRGKEIYYIGIHKQIFEIKNFYPLDIFDSFVTQIETTSENCYLESSCKIERDKLYPARFGIGFTLKNLKQLNVVYEFFQKVESRI DVQINYSLIQQFFGEDFDFNKMTEFMVGVDARPELAESKLKIALTIKDYPEKIKTAIELNGGLDKNIYNLLVSNSLHIGFDLSLDGRSE IELYPYIRNQEFQIFDIQQRLATVLLPQSLQFLPICSRICVGLSKANADKVVYFYLKNLNDFLNYFTVNDTARRVHAYYQQQPMDEM CVAVQEKELLGGTIEKMNLYYLI | WP_ 168465419.1 | Aphanizomenon sp. UHCC 0183 |
| 8 | | MTTLLLQRGKEIYYIGIHKQIFEIKNFYPLDIFDSFVTQIETTSENCYLESSCKIERDKLYPARFGIGFTLKNLKQLNVVYEFFQKVESRI DVQINYSLIQQFFGEDFDFNKMTEFMVGVDARPELAESKLKIALTIKDYPEKIKTAIELNGGLDKNIYNLLVSNSLHIGFDLSLDGRSE IELYPYIRNQEFQIFDIQQRLATVLLPQSLPICSRICVGLSKANADKVVYFYLKNLNDFLNYFTVNDTARRVHAYYQQQPMREMCVA VQEKQLLGGTIEKMNLYYLI | MBO1Q57683.1 | Dolichospermum sp. JUN01 |
| 9 | | MFRIKELQFYIYLESSCKIERDKLYPARFSIGFTLKNLKQLNVVYEFFQKVESRIDVQINYSLIQQFFGEDFDFNKMTEFMVGVDAR PELAESKLKIALTIKDYPEKIKTAIELNGGLDKNIYNLLVSNSLHIGFDLSLDGRSEIELYPYIRNQEFQIFDIQQRLATVLLPQSLQFLPI CSRICVGLSKANADKVVYFYLKNLNDFLNYFTVNDTARRVHAYYQQQPMDEMCVAVQEKELLGGTIEKMNLYYLI | MBD2217104.1 | Apharazomenon flos-aquae FACHB-1040 |
| 10 | | MATSLESSKKNYYISTHKQAFDVENFYPLEIWEKFVEKIEKTSEKCFLESSCKIDQGQFYAARFGIGFDLQNLQQLNAVYNFCQE VESRVGVRVDYSLIKQFLGDDFDFSKMTEFLIGVDARRELAESKLKIALTINDYPEKLKTAIYLNGGLDETIEKLIVSNSLHLGFDLSL NGISEIELYPYIGKQDFQRIDIQQRLATVLSPQALRPLAACRRICVGLSKGNTEKILYYYLEDIKDFLNYFTPNDTARRVHAYYQKQPI SEMCVAAPESQFIAEKIEKMNLYYLF | WP_ 002767616.1 | Microcystis aeru-ginosa |
| 11 | | MTKKNIRPQQVAPVERETTSTAKDQSGQVTAQWCIDYPKKIKTAIELNGGLDKNIYNLLVSNSLHIGFDLSLDGRSEIELYPYIRNQE FQIFDIQQRLATVLLPQSLQFLPICSRICVGLSKANADKVVYFYLKNLNDFLNYFTVNDTARRVHAYYQQQPMREMCVAVQEKQLL GGTIEKMNLYYLI | MBS9395133.1 | Dolichospermum sp. OL01 |
| 12 | | MVLKSNKKLYYISAHKHAFEIDNLYPLNLFEGFVERIEKIEKTENCVLESSCKIDHDKLYPVRFNIGFPNNSIKQLHAVMDFFRRVES RVDVKLNLSLFQQFIGNDFKLDKMTDLMLGIDLRRDLSDSRLKIGLTIEDYPEKQKAAVILNNNIDEVTSNLLISNRLHIGFDFYLNGR SEMELYPHIMQQDFQKLDVQQRLSKVLSPPALQVVPACTRICVGISKANRDKIIYYYLENMGDFLNYFTVNDTARKVHAYYLKQP VVEMCVALPESELLAGTTIKNLNLYYLL | KXS89939.1 | Microcystis aeru-ginosa NIES-88 |
| 13 | LimF-H172L | MKKRKSSKVFKSTIAPEEKLRYIGNHKQAFDIEPLYPLALFEEFVATTGDCIIECSGKIKQDQLYPARIDLQFSDKHHFHNIHTSIDFL KRAASRTDVNLNLDILATFLAGNFDYSKVQNILAGIDLRQNLGESKLKLFIRIGDYPAKMAVAKHLCNITPESEAMLRSDTLLIGFDFY LDGRSAIELYPELKKDEFNHPFIYNQLKTILSPEALKPLPLCNLFGIGLSPANEANVLYYHLENIEDFLSYFPINDTARRVHDFYLQQ EGSRRMWVALSESEMKAGRINNVNLYYSKAFTSQNP | - | - |

The prenyltransferase in the prenylation process preferably includes an enzyme including an amino acid sequence corresponding to the following (1), (2), or (3) and more preferably includes an enzyme having an amino acid sequence corresponding to the following (1), (2), or (3). In this aspect, these enzymes preferably have prenyl group transfer activity.

(1) Amino acid sequence represented by any of SEQ ID NOS: 1 to 12 listed in the above Table 1
(2) An amino acid sequence that has one or more amino acids deleted, replaced, or added in an amino acid sequence represented by any of SEQ ID NOS: 1 to 12
(3) An amino acid sequence that is 80% or more homologous to the amino acid sequence represented by SEQ ID NO: 1

In the present specification, when "having an amino acid sequence in which one or multiple amino acids are deleted, replaced, or added" is used, the number of amino acids deleted, replaced, or added is not particularly limited insofar as the resulting prenyltransferase retains its function. Furthermore, "a plurality of" means an integer of 2 or more, preferably 2 to 15, more preferably 2 to 10, further preferably 2 to 5, and even more preferably 2, 3, or 4. The position of deletion, replacement, or addition in each prenyltransferase is not particularly limited insofar as the resulting prenyltransferase retains its function, and may be N-terminal, C-terminal, or within in each prenyltransferase.

Furthermore, in the present specification, "Y% or more homologous to the amino acid sequence represented by SEQ ID NO: X" means that when the amino acid sequences of the two polypeptides are aligned so as to maximize the identity, the ratio of the number of common amino acid residues to the total number of amino acids in SEQ ID NO: X is Y% or more. In the present specification, when "Y% or more homologous" is used, this can also be rephrased as "Y% or more identical."

The amino acid sequence of the above (2) is an amino acid sequence derived from any of SEQ ID NOS: 1 to 12 by deletion, replacement, or addition of preferably 1 or more and 7 or less, more preferably 1 or more and 5 or less, further preferably 1 or more and 3 or less, and even more preferably 1 or 2 amino acids.

The amino acid sequence of the above (3) is homologous to the amino acid sequence represented by SEQ ID NO: 1, preferably 85% or more, more preferably 90% or more, further preferably 92% or more, even more preferably 95% or more, still more preferably 98% or more, and particularly preferably 99% or more.

The prenyltransferase in the prenylation process preferably includes an enzyme including an amino acid sequence corresponding to the following (4) and also preferably includes an enzyme having an amino acid sequence corresponding to the following (4). In this aspect, these enzymes preferably have prenyl group transfer activity.

(4) An amino acid sequence that is 80% or more homology with the amino acid sequence represented by SEQ ID NO: 2

The amino acid sequence of the above (4) is homologous to the amino acid sequence represented by SEQ ID NO: 2, preferably 85% or more, more preferably 90% or more, further preferably 92% or more, even more preferably 95% or more, still more preferably 98% or more, and particularly preferably 99% or more.

The prenyltransferase preferably contains a prenyltransferase derived from *Limnothrix* sp. or *Symploca* sp. or an enzyme homologous thereto. The enzyme homologous to the prenyltransferase derived from *Limnothrix* sp. or *Symploca* sp. is not particularly limited, but examples include enzymes having an amino acid sequence at least 40%, 50% or more, 60% or more, 70% or more, 75% or more, 80% or more, 85% or more, 90% or more, 92% or more, 95% or more, 98% or more, or 99% or more homology with the amino acid sequence of the prenyltransferase derived from *Limnothrix* sp. or *Symploca* sp. (for example, the amino acid sequence represented by SEQ ID NO: 1 or 2).

The prenyltransferase preferably includes an enzyme including an amino acid sequence selected from the group consisting of the amino acid sequence represented by SEQ ID NO: 1, the amino acid sequence represented by SEQ ID NO: 2, and amino acid sequences that have one or a plurality of amino acids deleted, replaced, or added in these amino acid sequences. It is particularly preferable that the prenyltransferase has an amino acid sequence selected from the group consisting of the amino acid sequence represented by SEQ ID NO: 1, the amino acid sequence represented by SEQ ID NO: 2, and amino acid sequences that have one or a plurality of amino acids deleted, replaced, or added in these amino acid sequences. An amino acid sequence that has one or a plurality of amino acids deleted, replaced, or added in the amino acid sequence represented by SEQ ID NO: 1 may be the amino acid sequence represented by SEQ ID NO: 13.

When the substrate of the prenylation process is a compound that does not have a structure represented by the above formula (IV) and having at least one structure represented by the above formula (III), and the prenyltransferase contains an enzyme that includes an amino acid sequence corresponding to the following (2') or (3), or that has any of these amino acid sequences, it is preferable that Glu at position 54 and His at position 172 in the amino acid sequence represented by SEQ ID NO: 1 in these enzymes is not deleted or replaced, and it is more preferable that Glu at position 54, His at position 172, Tyr at position 188, Tyr at position 237, and Tyr at position 292 in these enzymes are not deleted or replaced. Furthermore, the Asp at position 70 in the amino acid sequence represented by SEQ ID NO: 1 may have

been deleted or replaced, and when replaced, may have been replaced by Ala.

(2') An amino acid sequence that has one or more amino acids deleted, replaced, or added in an amino acid sequence represented by SEQ ID NO: 1

(3) An amino acid sequence that is 80% or more homology with the amino acid sequence represented by SEQ ID NO: 1

When the substrate of the prenylation process is a compound having no structure represented by the above formula (IV) and at least one structure represented by the above formula (III) and the prenyltransferase contains an enzyme including an amino acid sequence represented by the above (2) or (4) or having an amino acid sequence represented by the above (2) or (4), when the amino acid sequence of these enzymes is aligned with the amino acid sequence represented by SEQ ID NO: 1, the amino acids corresponding to Glu at position 54 and His at position 172 of SEQ ID NO: 1 are preferably not deleted or replaced, and the amino acids corresponding to Glu at position 54, His at position 172, Tyr at position 188, Tyr at position 237, and Tyr at position 292 of SEQ ID NO: 1 are more preferably not replaced. Furthermore, the amino acid corresponding to the Asp at position 70 in SEQ ID NO: 1 may be deleted or replaced, and when replaced, may be replaced by Ala.

Furthermore, when the substrate of the prenylation process is a compound having at least one structure represented by (IV), and the prenyltransferase contains an enzyme that includes an amino acid sequence corresponding to the above (2') or (3), or that has any of these amino acid sequences, it is preferable that Glu at position 54 in the amino acid sequence represented by SEQ ID NO: 1 in these enzymes is not deleted or replaced, and it is more preferable that Glu at position 54, Tyr at position 188, Tyr at position 237, and Tyr at position 292 in these enzymes are not deleted or replaced. In this aspect, His at position 172 in the amino acid sequence represented by SEQ ID NO: 1 may have been deleted or replaced, and when replaced, is preferably replaced by an amino acid having an aliphatic residue (for example, Leu, Met, Ile, or Val), and is more preferably replaced with Leu. Furthermore, the Asp at position 70 in the amino acid sequence represented by SEQ ID NO: 1 may have been deleted or replaced, and when replaced, may have been replaced by Ala.

Furthermore, when the substrate of the prenylation process is a compound having at least one structure represented by the above formula (IV) and the prenyltransferase contains an enzyme including an amino acid sequence represented by the above (2) or (4) or having an amino acid sequence represented by the above (2) or (4), when the amino acid sequence of these enzymes is aligned with the amino acid sequence represented by SEQ ID NO: 1, the amino acid corresponding to Glu at position 54 of SEQ ID NO: 1 is preferably not deleted or replaced, and the amino acids corresponding to Glu at position 54, Tyr at position 188, Tyr at position 237, and Tyr at position 292 are more preferably not replaced. Furthermore, the amino acid corresponding to His at position 172 of SEQ ID NO: 1 may be deleted or replaced, and when replaced, is preferably replaced by an amino acid having an aliphatic residue (for example, Leu, Met, Ile, or Val), and is more preferably replaced by Leu. Furthermore, the amino acid corresponding to the Asp at position 70 in SEQ ID NO: 1 may be deleted or replaced, and when replaced, may be replaced by Ala.

When the amino acid sequence represented by SEQ ID NO: 1 has a deletion or replacement, the deleted or replaced amino acid may be at least one of I at position 52, D at position 70, Q at position 72, L at position 122, H at position 172, E at position 190, L at position 222, G at position 224, H at position 239, and W at position 273. In the amino acid sequence represented by SEQ ID NO: 1, one or a plurality of amino acids may be further deleted, replaced, or added for an amino acid residue other than E at position 54, Y at position 188, Y at position 237, and Y at position 292.

Furthermore, when a deletion or replacement is performed in an amino acid sequence represented by any of SEQ ID NOS: 2 to 12, the amino acid that has been deleted or replaced may be at least one amino acid corresponding to I at position 52, D at position 70, Q at position 72, L at position 122, H at position 172, E at position 190, L at position 222, G at position 224, H at position 239, and W at position 273 when the amino acid sequence is aligned with the amino acid sequence represented by SEQ ID NO: 1. In the amino acid sequence represented by any of SEQ ID NOS: 2 to 12, one or a plurality of amino acids may be deleted, replaced, or added for an amino acid other than the amino acids corresponding to E at position 54, Y at position 188, Y at position 237, and Y at position 292 when aligned with the amino acid sequence represented by SEQ ID NO: 1.

The results of aligning SEQ ID NOS: 2 to 13 with SEQ ID NO: 1 are shown in FIGS. 14-1 and 14-2. In the figure, arrows indicate amino acids corresponding to a site to which a prenyl group donor binds (is contained) in the enzyme LimF having an amino acid sequence represented by SEQ ID NO: 1.

In the production method of the present embodiment, the prenyltransferase may be suitably selected according to the substrate to be prenylated and the type of compound that can provide a prenyl group.

For example, when the substrate to be prenylated is a peptide or a protein and prenylation of His or a derivative thereof is performed, when an amino acid adjacent to the N-terminal side of His or a derivative thereof has a positive charge or has much steric hindrance, an enzyme may be used that includes or has an amino acid sequence in which the I at position 52 is replaced in the amino acid sequence represented by SEQ ID NO: 1 or an amino acid sequence in which an amino acid corresponding to the I at position 52 in SEQ ID NO: 1 is replaced in an amino acid sequence represented by any of SEQ ID NOS: 2 to 12 when aligned with the amino acid sequence represented by SEQ ID NO:

1. Here, the amino acid to be replaced is not particularly limited, but may, for example, be replaced by an amino acid with less steric hindrance and is preferably replaced by Ala.

Furthermore, when the prenyl group introduced into the substrate in the production method of the present embodiment is a dimethylallyl group, an enzyme may be used that includes or has an amino acid sequence in which the G at position 224 of SEQ ID NO: 1 is replaced in the amino acid sequence represented by SEQ ID NO: 1, or an amino acid sequence in which an amino acid corresponding to G at position 224 of SEQ ID NO: 1 is replaced in an amino acid sequence represented by any of SEQ ID NO: 2 to 12 when aligned with the amino acid sequence represented by SEQ ID NO: 1. Here, the amino acid to be replaced may, for example, be replaced by an amino acid that is bulkier than Gly, is preferably replaced by Cys, Met, Gln, Leu, Thr, Val, or His, is more preferably replaced by Met or His, and is further preferably replaced by Met.

Furthermore, when the prenyl group introduced into the substrate in the production method of the present embodiment is a relatively large group such as a farnesyl group, geranylgeranyl group, geranylfarnesyl group, hexaprenyl group, octaprenyl group, or decaprenyl group, and particularly when such is a farnesyl group, an enzyme may be used that includes or has an amino acid sequence in which the L at position 222 or the W at position 273 of SEQ ID NO: 1 is replaced in the amino acid sequence represented by SEQ ID NO: 1, or an amino acid sequence in which an amino acid corresponding to the L at position 222 or the W at position 273 of SEQ ID NO: 1 is replaced in an amino acid sequence represented by any of SEQ ID NOs: 2 to 12 when aligned with the amino acid sequence of SEQ ID NO: 1. Here, the W at position 273 or the amino acid corresponding to W at position 273 may be replaced, for example, by a neutral amino acid or an amino acid with less steric hindrance, preferably by a neutral amino acid with less steric hindrance, more preferably by Asn, Ser, Thr, Val, Ile, Cys, Gly or Ala, and further preferably by Asn or Thr.

When using an enzyme that includes or has an amino acid sequence in which W at position 273 of SEQ ID NO: 1 is replaced in the amino acid sequence represented by SEQ ID NO: 1 or an amino acid sequence in which an amino acid corresponding to W at position 273 of SEQ ID NO: 1 is replaced in an amino acid sequence represented by any of SEQ ID NOS: 2 to 12 when aligned with the amino acid sequence represented by SEQ ID NO: 1, H at position 239 of SEQ ID NO: 1 or the amino acid corresponding to H at position 239 of SEQ ID NO: 1 when aligned with the amino acid sequence represented by SEQ ID NO: 1 or any of SEQ ID NOS: 2 to 12 may be further replaced. Here, the replaced amino acid may, for example, be replaced by Gln, Gly, Arg, Val, Cys, or Ala, and is preferably replaced by Gly or Ala.

In the production method of the present embodiment, when the prenyl group introduced into the substrate is a relatively large group such as a farnesyl group, a geranylgeranyl group, a geranylfarnesyl group, a hexaprenyl group, an octaprenyl group, or a decaprenyl group, the H239G/W237T variant and the H239G/W237N variant of the enzyme having the amino acid sequence represented by SEQ ID NO: 1, and an enzyme including or having an amino acid sequence in which mutations corresponding to the H239G/W237T mutation and the H239G/W237N mutation of the amino acid sequence represented by SEQ ID NO: 1 are present in the amino acid sequence represented by any of SEQ ID NOS: 2 to 12 when aligned with the amino acid sequence represented by SEQ ID NO: 1 may be used.

Furthermore, when the prenyl group introduced into the substrate in the production method of the present embodiment is a geranyl group, an enzyme may be used that includes or has: (i) the amino acid sequence represented by SEQ ID NO: 1; (ii) an amino acid sequence in which one or multiple amino acids other than E at position 54, Y at position 188, Y at position 237, and Y at position 292, preferably one or multiple amino acids other than E at position 54, H at position 172, Y at position 188, Y at position 237, and Y at position 292, or at least one amino acid from among I at position 52, D at position 70, Q at position 72, L at position 122, H at position 172, E at position 190, L at position 222, G at position 224, H at position 239, and W at position 273, more preferably at least one amino acid from among I at position 52, D at position 70, Q at position 72, L at position 122, E at position 190, L at position 222, G at position 224, H at position 239, and W at position 273 in the amino acid sequence represented by SEQ ID NO: 1 is deleted or replaced; or (iii) an amino acid sequence in which one or multiple amino acids other than those corresponding to E at position 54, Y at position 188, Y at position 237, and Y at position 292, preferably one or multiple amino acids other than E at position 54, H at position 172, Y at position 188, Y at position 237, and Y at position 292, or at least one amino acid from among I at position 52, D at position 70, Q at position 72, L at position 122, H at position 172, E at position 190, L at position 222, G at position 224, H at position 239, and W at position 273, more preferably at least one amino acid from among I at position 52, D at position 70, Q at position 72, L at position 122, E at position 190, L at position 222, G at position 224, H at position 239, and W at position 273 in SEQ ID NO: 1 is replaced in an amino acid sequence represented by any of SEQ ID NOS: 2 to 12 when alight with the amino acid sequence represented by SEQ ID NO: 1.

Here, I at position 52, D at position 70, Q at position 72, L at position 122, E at position 190, L at position 222, and H at position 239 may be replaced by any amino acid, but may, for example, be replaced by Ala. H at position 172 may be replaced by any amino acid, but may, for example, be replaced by Leu, Phe, or Ala. G at position 224 may be replaced by any amino acid, but may, for example, be replaced by Phe or Tyr. W at position 273 may be replaced by any amino acid, but may, for example, be replaced by Asn, Ser, Thr, Val, Ile, Cys, Phe, Met, Gly, or Ala.

Note that, among the enzymes listed above as preferable enzymes for when a comparatively large group (particularly, a farnesyl group) such as a dimethylallyl group, a geranyl group, a farnesyl group, a geranylgeranyl group,

a geranylfarnesyl group, a hexaprenyl group, an octaprenyl group, or a decaprenyl group is introduced, enzymes listed in duplicate can be preferably used in any case. For example, when an enzyme listed above as a preferable enzyme for when a geranyl group is introduced is also described as an enzyme preferable for introducing a comparatively large group (particularly, a farnesyl group) such as a farnesyl group, a geranylgeranyl group, a geranylfarnesyl group, a geranylfarnesyl group, a hexaprenyl group, an octaprenyl group, and a decaprenyl group, this enzyme is preferably used when introducing a geranyl group as well as when introducing a comparatively large group (particularly, a farnesyl group) such as a farnesyl group, a geranylgeranyl group, a geranylfarnesyl group, a geranylfarnesyl group, a hexaprenyl group, an octaprenyl group, and a decaprenyl group.

Furthermore, in the enzymes listed above as enzymes that can be used or are preferably used when the substrate of the prenylation process is a compound that does not have structure represented by the above formula (IV) and that has at least one structure represented by the above formula (III), when the substrate of the prenylation process is a compound having at least one structure represented by (IV), and when the substrate to be prenylated is a peptide or a protein and His or a derivative thereof is prenylated, a mutation may be introduced that is listed above as being introduced in an enzyme listed above as an enzyme used when introducing a comparatively large group (particularly a farnesyl group) such as a dimethylallyl group, a geranyl group, a farnesyl group, a geranylgeranyl group, a geranylfarnesyl group, a hexaprenyl group, an octaprenyl group, and a decaprenyl group according to the type of prenyl group introduced into the substrate. Furthermore, in addition to the mutations described above, one or multiple amino acids may be deleted, replaced, or added. The prenyltransferase may include an enzyme including or having an amino acid sequence that is at least 40% or more, 50% or more, 60% or more, 70% or more, 75% or more, 80% or more, 85% or more, 90% or more, 92% or more, 95% or more, 98% or more, or 99% or more homologous to an enzyme listed above or a variant thereof.

In the production method of the present embodiment, the prenyltransferase may be selected prior to the prenylation process according to the substrate to be prenylated and the type of compound that can provide a prenyl group.

Any of the prenyltransferases described above may be used in a while carried by a solid-phase carrier such as magnetic beads. Therefore, the aspect of an enzyme carried on a solid-phase carrier is included in the prenyltransferase used in the prenylation process. Furthermore, the prenyltransferase may have additional sequences such as a His tag, glutathione-S-transferase (GST), maltose-binding protein (MBP), and the like.

The above prenyltransferase may be subjected to the prenylation process using independently or two or more in combination.

(Prenylation Process)

The prenylation process is a process for bringing any of the above substrates or a combination thereof into contact with any of the above prenyltransferases or a combination thereof to obtain a compound having at least one structure represented by the following formula (I) or (II).

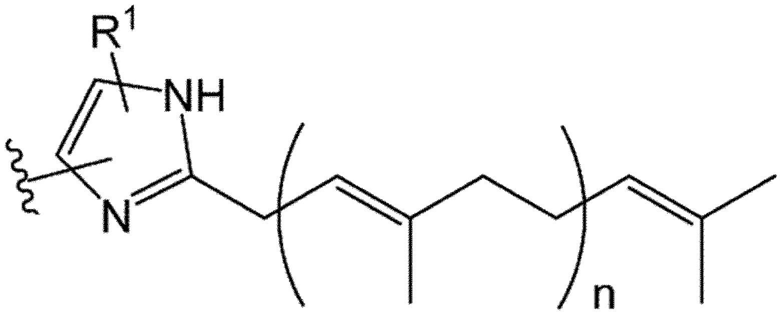

( I )

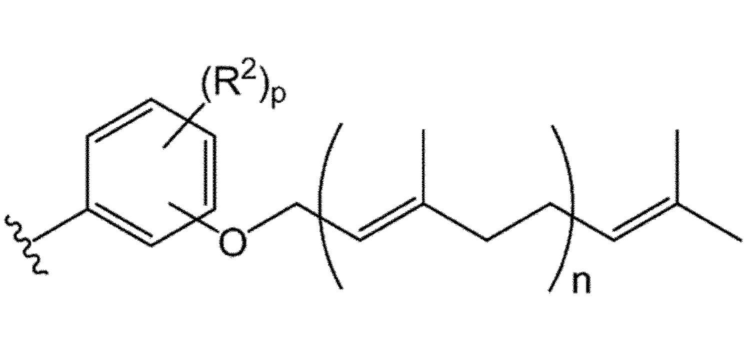

( I I )

Note that in formula (I), $R^1$ is a hydrogen atom or optional substituent, and n represents an integer of 0 to 11, and in formula (II) above, each $R^2$ is independently optional substituent, where p represents an integer of 0 to 4 and n represents an integer of 0 to 11.

In the structure represented by the above formula (I), the bonding position in the imidazole ring of $R^1$ is not particularly limited, that is, the structure represented by the above formula (I) may have a structure that corresponds to the above formulas (III-1) and (III-2) in the above formula (III).

In the structure represented by the above formula (II), the bonding position in the benzene ring for the oxygen atom may be any of the ortho position, the meta position, or the para position, that is, the structure represented by the above formula (II) may have a structure corresponding to the above formulas (IV-1), (IV-2), and (IV-3) in the structure represented by the above formula (IV). In the structure represented by the above formula (II), the bonding position of the oxygen atom in the benzene ring is preferably the para position.

Furthermore, examples, preferable aspects, and the like of $R^1$ in the above formula (I) are the same as for those of $R^1$ in the above formula (III). Furthermore, examples, preferable aspects, and the like of $R^2$ and p in the above formula (II) are the same as for those of $R^2$ and p in the above formula (IV).

In the above formulas (I) and (II), n represents an integer of 0 to 11 and depends on the type of prenyl group donor and the like described later that is used. In the above formulas (I) and (II), n is, for example, 0 or more and 8 or less, 0 or more and 6 or less, 0 or more and 5 or less, 0 or more and 3 or less, 0 or more and 2 or less, or 0, 1, or 2.

The compound obtained by the prenylation process corresponds to the substrate subjected to the prenylation process. Any of the above substrates can provide a compound having the structure of the above formula (I) or (II), but other $R^1$s in the above formula (I), $R^2$ and p in formula (I), and other structures can provide structures that correspond to the substrate used.

Note that when the substrate has two or more structures represented by the above formula (III) or (IV), at least one or more from among a plurality of moieties having this structure must be prenylated, one moiety alone may be prenylated, or all moieties may be prenylated.

Furthermore, similarly to when the substrate has both a structure represented by the above formula (III) and a structure represented by the above formula (IV), either the structure represented by the above formula (III) or the structure represented by the above formula (IV) may be prenylated, the structure represented by the above formula (III) alone may be prenylated, the structure represented by the above formula (IV) alone may be prenylated, and both the structure represented by the above formula (III) and the structure represented by the above formula (IV) may be prenylated.

The prenylation process may be, for example, a process for reacting at an appropriate reaction temperature and reaction time in the presence of any of the substrates described above or a combination thereof, any of the prenyltransferase described above or a combination thereof, and any compound capable of providing a prenyl group (hereinafter referred to as a "prenyl group donor") or a combination thereof.

The prenyl group donor is not particularly limited insofar as it is a compound having a prenyl group or a functional group that can be converted to a prenyl group. The prenyl group donor is, for example, dimethylallyl diphosphate (DMAPP) (dimethylallyl pyrophosphate). Examples of prenyl diphosphates include isoprenyl diphosphate (IPP), geranyl diphosphate (GPP), farnesyl diphosphate (FPP), geranylgeranyl diphosphate (GGPP), and phytyl diphosphate (PDP). These prenyl group donors may be subjected to the prenylation process using independently or two or more in combination. The prenyl group donor may be suitably changed according to the type of prenyltransferase.

The prenylation process may be carried out in a reaction system in which the substrate, prenyltransferase, and prenyl group donor are all added to a solvent, or may be carried out by bringing a reaction system in which any of the substrate, prenyltransferase, and prenyl group donor are carried on a solid-phase carrier such as magnetic beads into contact with a solution to which the remainder are added.

When the prenyltransferase is present in a solution, the concentration of the prenyltransferase may be suitably changed according to the expression and purification conditions of the enzyme and is not particularly limited. The concentration of the prenyltransferase may be, for example, 0.050 $\mu$M to 1.0 mM or 0.10 to 500 $\mu$M.

When the substrate is present in the solution, the concentration thereof is not particularly limited but may be, for example, 0.010 $\mu$M to 50 mM or 0.10 $\mu$M to 10 mM.

When the prenyl group donor is present in the solution, the concentration thereof is not particularly limited but may be, for example, 0.20 to 100 times or 1.0 to 50 times the concentration of the substrate, and specifically may be 0.010 $\mu$M to 100 mM or 0.10 to 50 mM.

The reaction temperature (incubate temperature) in the prenylation process is not particularly limited but is preferably within a range of $\pm$20°C from the optimum temperature of the prenyltransferase to be used. The reaction temperature is, for example, 0 to 50°C, preferably 0 to 40°C.

The reaction time in the prenylation process may be suitably adjusted according to the reaction temperature and the concentration and the like of the substrate, prenyltransferase, and prenyl group donor. The reaction time is, for example, 30 minutes to 100 hours, 1.0 to 80 hours, 3.0 to 50 hours, or 5.0 to 40 hours.

The prenylation process may contain ingredients other than the substrate, prenyltransferase, and prenyl group

donor in the reaction system. Such ingredients are not particularly limited, and examples include ingredients used in preparing any of the substrate, prenyltransferase, and prenyl group donor, as well as cofactors that raise the catalytic activity of the prenyltransferase, and the like.

The cofactors are not particularly limited, but examples include magnesium ions, copper ions, iron ions, manganese ions, molybdenum ions, nickel ions, selenium ions, zinc ions, and the like. One of these cofactors may be present alone or in a combination of two or more in the reaction system. Of these, magnesium ions are preferably present.

Whether or not a cofactor is used in the prenylation process may be appropriately selected based on various conditions such as the type of prenyltransferase to be used.

One preferable aspect of the prenylation process is a process in which a peptide or protein having an amino acid sequence containing at least one His, Tyr, or derivative thereof is brought into contact with any of the above prenyltransferases or a combination thereof to introduce a prenyl group into at least one His residue, Tyr residue or a derivative residue thereof.

According to such an aspect, a prenyl group can be introduced into a peptide or protein which generally tends to have low cell membrane permeability and/or bioavailability, and a potentially promising drug can be obtained.

(Other Processes)

The method for producing the compound of the present embodiment may include processes other than the above prenylation process. Examples of such processes include a process for preparing at least one of a substrate, a prenyltransferase, and a prenyl group donor to be subjected to the prenylation process, a process for separating and purifying the prenylated compound through the prenylation process, and a process for analyzing physical properties of the purified prenylated compound.

The process for preparing a substrate to be subjected to the prenylation process (hereinafter referred to as the "substrate preparation process") is a process for preparing a compound having at least one structure represented by the above formula (III) or (IV). The substrate preparation process may be a process for producing such a compound by a conventionally known production method or may be a process for purchasing a commercially available compound and optionally preparing such through purification or the like.

In the substrate preparation process, one type or two or more types of substrates as described above may be prepared. When there are two or more types substrates prepared in the substrate preparation process, the substrate preparation process is a process for preparing a library of compounds having at least one structure represented by the above formula (III) or (IV). By implementing the method for producing the compound of the present embodiment using such a compound library, a library of compounds having at least one structure represented by the above formula (I) or (II) can be produced. That is, one aspect of the production method of the present embodiment is a method for producing a compound library having at least one structure represented by the above formula (I) or (II).

Furthermore, when two or more types of compounds containing peptides or proteins having an amino acid sequence that includes at least one His, Tyr, or derivative thereof are prepared in the substrate preparation process, the substrate preparation process is a process for preparing a library of such peptides or proteins. The library of peptides or proteins having prenyl groups in at least one His residue, Tyr residue, or derivative residue thereof can be produced by carrying out a method for producing the compound of the present embodiment using such a peptide or protein library. That is, one aspect of the production method of the present embodiment is a method for producing a peptide or protein library having a prenyl group in at least one His residue, Tyr residue, or derivative residue thereof. The method for producing a prenylated compound library of the present embodiment described below may be used as reference for the method for producing a peptide or protein library.

A conventionally known chemical synthesis method may be used as the method for producing the substrate in the substrate preparation process, and particularly when the substrate is a peptide or a protein, a synthesis method using a translation synthesis system may be used. Examples of such translation synthesis systems include cell translation systems and cell-free translation systems. When producing a peptide or protein library, it is preferable to use a synthesis method using a cell-free translation system from perspectives such as improving the diversity of the library. A method for producing a peptide or protein using a cell-free translation system will be described later.

The process for preparing a prenyltransferase (hereinafter referred to as the "prenyltransferase preparation process") is a process for preparing LimF or an enzyme that is homologous thereto as described above. In the prenyltransferase preparation process, the enzyme may be isolated from a naturally occurring organism (for example, *Limnothrix* sp., *Symploca* sp., and the like) or may be obtained by heterogeneously expressing a gene encoding the target enzyme in an appropriate host (for example, *Escherichia coli*). Alternatively, this can be obtained by a cell-free translation system or a chemical synthesis method. In the prenyltransferase preparation process, the prenyltransferase may be selected according to the substrate to be prenylated and the kind of the compound capable of donating the prenyl group.

A process for separating and/or purifying the prenylated compound (hereinafter referred to as the "separation/purification process") is a process for separating a compound having at least one structure represented by the above

formula (I) or (II) obtained by the production method of the present embodiment from a raw material and/or a byproduct, and/or, when a plurality of target compounds is obtained, purifying each of the target compounds. A well-known separation method such as liquid chromatography or the like may be used as the separation/purification process.

A process for analyzing the physical properties of the purified prenylated compound (hereinafter referred to as the "analysis process") is a process for analyzing the target compound obtained in the prenylation process or the target compound isolated in the separation/purification process. The analysis process may include identification of the obtained compound, calculation of prenylation efficiency, measurement of the dissociation constant for a desired target substance, and the like. Specifically, the analysis process may include an analysis method for a compound described in the examples.

[Method for Producing Prenylated Compound Library]

In the method for producing the compound of the present embodiment, a library of compounds containing a prenylated peptide or protein can be produced by using a library containing two or more compounds containing a peptide or protein having an amino acid sequence including at least one His, Tyr or a derivative thereof as the substrate.

That is, the method for producing a compound library of the present embodiment is a method for producing a compound library containing a prenylated peptide or protein, the method including a process wherein a compound library containing a peptide or protein having an amino acid sequence containing at least one His, Tyr or a derivative thereof is brought into contact with a prenyltransferase and a prenyl group is introduced into at least one His residue, Tyr residue, or derivative residue thereof, wherein the prenyltransferase is LimF or its homologous enzyme.

According to the method for producing a compound library of the present embodiment, a library of compounds containing highly hydrophobic peptides or proteins can be produced by prenylating part of at least one His, Tyr, or derivative thereof using a prescribed prenyltransferase. Furthermore, because LimF or an enzyme homologous thereto is used as the prenyltransferase in the method for producing a compound library of the present embodiment, a library that has high substrate tolerance for the prenyltransferase and contains various compounds having prenyl groups can be produced. As described above, according to the method for producing the compound library of the present embodiment, prenyl groups can be efficiently introduced into various compounds, and a compound having potentially high cell membrane affinity and/or high cell membrane permeability can be produced.

In the present specification, a "compound library" refers to a compound group containing at least two compounds. A compound library obtained by the method for producing a compound library of the present embodiment is a library of compounds containing a peptide or protein in which at least one His residue, Tyr residue, or derivative residue thereof has been prenylated. The compounds contained in the library may be peptides or proteins, or compounds in which a structure other than an amino acid is bound to a peptide or a protein by a covalent bond or a non-covalent bond. Examples of moieties other than a peptide or a protein in the compound in which a structure other than an amino acid is bound to a peptide or a protein include nucleic acid molecules such as linkers, DNA, RNA, and the like, and labels (fluorescent substances, radioactive substances, metal nanoparticles, quantum dots, enzymes, and the like) for detecting a peptide or a protein.

The method for producing a compound library of the present embodiment can be implemented in a manner similar to the method for producing a compound of the present embodiment described in detail above other than using a library that includes two or more compounds including a peptide or a protein having an amino acid sequence that includes at least one His, Tyr, or derivative thereof as a substrate.

A process for producing a library used as a substrate (hereinafter referred to as the "library preparation process") will be described in detail below. Note that the compound library may be prepared by purchasing a commercially available library.

(Library Preparation Process)

The library preparation process is not particularly limited insofar as it is a process that can prepare a library containing two or more types of compounds containing a peptide or protein having an amino acid sequence containing at least one His, Tyr, or derivative thereof. The compounds in the library may be synthesized by a solid-phase or liquid-phase chemical synthesis method or may be prepared by a method using a translation synthesis system, but preferably by a method using a cell-free translation system.

The library preparation process is preferably a process in which an mRNA library is translated by a cell-free translation system to prepare a compound library containing a peptide or protein having an amino acid sequence containing at least one His, Tyr or a derivative thereof.

In the present specification, "mRNA library" refers to an mRNA group including two or more types of mRNA having a plurality of N1N2N3 codons.

Furthermore, in the present specification, "N1N2N3" means a codon that designates a given amino acid, and N1, N2, and N3 are each independently selected from adenine (A), guanine (G), cytosine (C), and uracil (U). One mRNA

includes a plurality of N1N2N3, but each of N1, N2, and N3 is independently selected. Therefore, for example, when -N1N2N3-N1N2N3- is included in the mRNA, the two of each of N1, N2, and N3 may be the same as or different from each other.

A process for preparing a compound library containing peptides using the mRNA library will be described below for convenience, but a process for preparing a compound library containing proteins using the mRNA library can also be similarly implemented by adjusting the number of bases in the mRNA. Note that the library preparation process is more preferably a process in which an mRNA library is translated by a cell-free translation system to prepare a compound library containing a peptide having an amino acid sequence containing at least one His, Tyr or a derivative thereof.

In one aspect of the library preparation process, first, an mRNA library containing mRNA encoding peptides contained in each compound in the compound library is prepared (hereinafter referred to as the "mRNA preparation process").

The sequences of mRNAs encoding the peptides contained in each compound of the compound library is determined according to the amino acid sequence of a desired peptide. Such an mRNA library may be prepared by, for example, synthesizing a DNA library encoding the mRNA library and transcribing the DNA library.

In the mRNA preparation process, any amino acid may be re-assigned to the N1N2N3 codon. In the re-assignment, a relationship that differs from the relationship between the codon and the amino acid in the natural genetic code table may be assigned, or the same relationship may be assigned.

In the present specification, "natural genetic code table" refers to a table indicating amino acids represented by genetic code made up of triplets of mRNA *in vivo.* In the natural genetic code table, N1N2N3 encodes the following amino acids.

[Table 2]

| | U | C | A | G | |
|---|---|---|---|---|---|
| **U** | **Phe** | **Ser** | **Tyr** | **Cys** | **U** |
| | **Phe** | **Set** | **Tyr** | **Cys** | **C** |
| | **Leu** | **Ser** | **STOP** | **STOP** | **A** |
| | **Leu** | **Ser** | **STOP** | **Trp** | **G** |
| **C** | **Leu** | **Pro** | **His** | **Arg** | **U** |
| | **Leu** | **Pro** | **His** | **Arg** | **C** |
| | **Leu** | **Pro** | **Gln** | **Arg** | **A** |
| | **Leu** | **Pro** | **Gln** | **Arg** | **G** |
| **A** | **Ile** | **Thr** | **Asn** | **Ser** | **U** |
| | **Ile** | **Thr** | **Asn** | **Ser** | **C** |
| | **Ile** | **Thr** | **Lys** | **Arg** | **A** |
| | **Met** | **Thr** | **Lys** | **Arg** | **G** |
| **G** | **Val** | **Ala** | **Asp** | **Gly** | **U** |
| | **Val** | **Ala** | **Asp** | **Gly** | **C** |
| | **Val** | **Ala** | **Glu** | **Gly** | **A** |
| | **Val** | **Ala** | **Glu** | **Gly** | **G** |

More specifically, in the mRNA preparation process, for example, Leu may be assigned to the UUG codon as in the natural genetic code table described above, or an amino acid other than Leu may be assigned by reassigning the amino acid. "Assigning an amino acid to a codon" means rewriting genetic code such that a codon encodes that amino acid. In the present specification, "assigning an amino acid to a codon" and "reassigning a codon" are used synonymously.

The assignment of an amino acid different from the natural genetic code table to each codon is realized by, for example, codon reassignment using an artificial aminoacylated RNA catalyst flexizyme (flexizyme). According to the flexizyme, a desired amino acid can be bound to tRNA having a given anticodon, and thus a given amino acid can be assigned to a given codon. For example, a known flexizyme disclosed in H. Murakami, H. Saito, and H. Suga, (2003), Chem. Biol., Vol. 10, 655-662 and the like may be used.

In the present specification, binding an amino acid to tRNA involves charging the amino acid to tRNA, aminoacylating tRNA, or acylating tRNA with the amino acid.

The plurality of N1N2N3 in each mRNA is not particularly limited and may be completely random; for example, a portion of bases may be fixed to a specific base, such as in a plurality of N1N2K, a plurality of N1N2S, a plurality of N1N2M, a plurality of N1N2W, a plurality of N1N2A, a plurality of N1N2U, a plurality of N1N2C, or a plurality of N1N2G. In the above, N1 and N2 have the same meaning as N1 and N2 in N1N2N3, each K is each independently either of uracil (U) and guanine (G), each S is independently either of cytosine (C) and guanine (G), each M is independently either of adenine (A) and cytosine (C), and each W is independently either of adenine (A) and uracil (U).

The mRNA preparation process will be described below for convenience by citing as an example a case where an N1N2K library contains a plurality of mRNA containing a plurality of N1N2K codons, that is, a case where each mRNA in the mRNA library contains a plurality of mRNA codons, but even in a case where another mRNA library is used, the same implementation is possible insofar as the peptides contained in the translated compound library are prenylated. In the natural genetic code table, N1N2K indicates 20 amino acids for which the right column of the above Table 2 is G or U.

In the mRNA preparation process, any amino acid can be assigned to the "N1N2K" codon, and not only proteinogenic amino acids, but also, for example, non-proteinogenic amino acids may be assigned. For example, when an amino acid containing a cyclic structure and/or an N-alkylamino acid is used as the non-proteinogenic amino acid, there is a tendency to be able to obtain a compound library having increased resistance to proteolysis, increased cell membrane permeability, and/or increased rigidity of conformation. Such compound library is useful for screening for compounds targeting a disease-related molecule in a cell and/or compounds targeting a molecule having a protease activity.

In the mRNA preparation process, all of the plurality of N1N2K may be assigned to non-proteinogenic amino acids, or a portion thereof may be assigned to non-proteinogenic amino acids.

The mRNA library preferably includes a start codon, a plurality of N1N2K, and a stop codon in this order in each mRNA. Here, at least one set of the plurality of N1N2K is a codon to which His or Tyr is assigned. When using the natural genetic code table, each mRNA includes, for example, a sequence of AUG-(N1N2K)n-UAG. Here, N1 and N2 are each independently selected from adenine (A), guanine (G), cytosine (C) and uracil (U), and each K is independently either uracil (U) or guanine (G). Furthermore, n is an integer of 1 or more and 49 or less. n may be 2 or more, 3 or more, 4 or more, 5 or more, or 9 or more, and may be 44 or less, 39 or less, 29 or less, or 19 or less within the above range.

In the library preparation process, when an mRNA library encoding a peptide having an amino acid sequence containing at least one His, Tyr, or derivative thereof is prepared by using the natural genetic code table, it is preferable that each mRNA contains a start codon, a plurality of N1N2K, and a stop codon in this order and that at least one set of the plurality of N1N2K is any of CAU, CAC, UAU and UAC (that is, any codon to which His or Tyr is assigned). In this case, for example, to describe an example that includes CAU, each mRNA includes a sequence of AUG-(N1N2K)n1-CAU-(N1N2K)n2-UAG. Here, N1, N2, and K have the same meaning as above, and n1 and n2 are each an integer of 0 or more and 48 or less. The sum of n1 and n2 may be defined such that their sum is n-1 (where n is the same meaning as above).

In the description of the substrate of the method for producing the compound of the present embodiment, it is described that, in the amino acid sequence of the peptide or protein to be the substrate, a particularly preferable amino acid on the N-terminal side adjacent to His, Tyr, or a derivative thereof is Thr, Ala, Gly, Ser, Val, Leu, Asn, Met, Pro, or Ile. From this perspective, each mRNA in the mRNA library preferably includes, for example, a sequence shown in Table 3 below (in the sequences shown in Table 3, RBU is an example and may be replaced with a codon encoding any of Thr, Ala, Gly, Ser, Val, Leu, Asn, Met, Pro, or Ile). In Table 3, N1, N2, and K have the same meaning as described above, R is either adenine (A) or guanine (G), B is either guanine (G), cytosine (C), or uracil (U), and m1 and m2 are each an integer of 0 or more and 47 or less. m1 and m2 may be selected within a range such that the sum of m1 and m2 is 0 or more and 47 or less. The sum of m1 and m2 may be 1 or more, 2 or more, 3 or more, or 7 or more, and may be 42 or less, 37 or less, 27 or less, or 17 or less.

Note that in Table 3, the CAU codon may be replaced by other codons that encode His or Tyr (for example, CAC, UAU, UAC, and the like).

[Table 3]

| | | | | | |
|---|---|---|---|---|---|
| **mRNA lib:** | **AUG $(N1N2K)_{m1}$** | **RBU** | **CAU** | **$(N1N2K)_{m2}$** | **UAG** |
| **Peptide:** | **Met** | **A/I/G/T/V/S** | **His** | | **Stop** |

When the library preparation process is a process for preparing a library of a compound containing a cyclic peptide, for example, an mRNA library containing an mRNA encoding a peptide containing an amino acid having a functional group 1 shown in Table 4 below and an amino acid having a corresponding functional group 2 may be used.

[Table 4]

| | Functional Group 1 | Functional Group 2 |
|---|---|---|
| (A) | 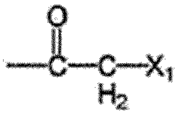 (A-1) | HS-(A-2) |
| (B) | 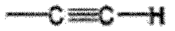 (B-1) | $N_3$-(B-2) |
| (C) | -Ar-$CH_2NH_2$ (C-1) | 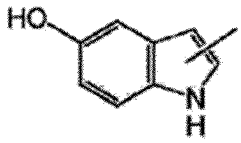 (C-2) |
| (D) | -C≡C-$CH_2$-$X_1$ (D-1) | HS-(D-2) |
| (E) | -Ar-$CH_2$-$X_1$ (E-1) | HS-(B-2) |

In the chemical structure formulas in the above Table 4, $X_1$ is a leaving group and Ar is an optionally substituted aromatic ring. Examples of the leaving group include halogen atoms such as Cl, Br, and I.

When such an mRNA library is used, a library of compounds containing cyclic peptides obtained by cyclizing amino acids having functional groups 1 and amino acids having corresponding functional groups 2 can be obtained. For example, when the library preparation process is performed using an mRNA library that encodes peptides containing an amino acid having a functional group of (A-1) and an amino acid having a functional group of (A-2), a cyclic peptide containing a cyclic structure formed by an N-CO-$CH_2$-S structure can be prepared.

Either of the functional groups 1 and 2 may be on the N-terminal side, these may be disposed on the N-terminal and the C-terminal, one may be a terminal amino acid and the other a non-terminal amino acid, and both may be non-terminal amino acids. The bond formed by the functional group 1 and the functional group 2 can be called a chemical cross-linking structure for forming a molecular cyclic structure in a cyclic peptide.

For example, a chloroacetylated amino acid may be used as the amino acid having the functional group of (A-1). Examples of chloroacetylated amino acids include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophan, β-N-chloroacetyl-L-diaminopropanoic acid, γ-N-chloroacetyl-L-diaminobutyric acid, δ-N-chloroacetyl-L-ornithine, ε-N-chloroacetyl-L-lysine, and D-amino acid derivatives and the like corresponding thereto.

N-chloroacetyl-L-tyrosine and N-chloroacetyl-D-tyrosine are preferably used as the amino acid having the functional group of (A-1).

Examples of amino acids having the functional group of (A-2) include cysteine, homocysteine, mercaptonorvaline, mercaptonorleucine, 2-amino-7-mercaptoheptanoic acid, 2-amino-8-mercaptooctanoic acid, and the like.

Cysteine is preferably used as the amino acid having the functional group of (A-2).

Examples of cyclization methods using an amino acid having the functional group of (A-1) and an amino acid having the functional group of (A-2) include the methods taught in: Kawakami, T. et al., Nat. Chem. Biol. 5, 888-890 (2009), Yamagishi, Y et al., ChemBioChem 10, 1469-1472 (2009), Sako, Y et al., J. Am. Chem. Soc. 130, 7932-7934 (2008), Goto, Y et al., ACS Chem. Biol. 3, 120-129 (2008), Kawakami T. et al, Chem. Biol. 15, 32-42 (2008), and WO 2008/117833 and the like.

Examples of amino acids having the functional group of (B-1) include propargylglycine, homopropargylglycine, 2-amino-6-heptynoic acid, 2-amino-7-octynoic acid, 2-amino-8-nonynoic acid, and the like.

An amino acid that has been 4-pentynoylated or 5-hexynoylated may be used.

Examples of 4-pentynoylated amino acids include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, α-N-(4-pentenoyl)-L-ornithine, ε-N-(4-pentenoyl)-L-lysine, and D-amino acid derivatives and the like corresponding thereto.

Examples of 5-hexynoylated amino acids include amino acids in which a 4-pentynoyl group is replaced with a 5-hexynoyl group in a compound exemplified as a 4-pentynoylated amino acid.

Examples of amino acids having the functional group of (B-2) include azidoalanine, 2-amino-4-azidobutanoic acid, azidoptonorvaline, azidonorleucine, 2-amino-7-azidoheptanoic acid, 2-amino-8-azidooctanoic acid, and the like.

It is also possible to use an amino acid that has been azidoacetylated or 3-azidopentanoylated.

Examples of azidoacetylated amino acids include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine, ε-N-azidoacetyl-L-lysine, and D-amino acid derivatives corresponding thereto.

Examples of 3-azidopentanoylated amino acids include amino acid in which an azidoacetyl group is replaced with a 3-azidopentanoyl group in a compound exemplified as an azidoacetylated amino acid.

Examples of cyclization methods using an amino acid having the functional group of (B-1) and an amino acid having the functional group of (B-2) include methods taught in Sako, Y et al., J. Am. Chem. Soc. 130, 7932-7934 (2008), WO 2008/117833, and the like.

Examples of an amino acid having the functional group of (C-1) include N-(4-aminomethyl-benzoyl)-phenylalanine (AMBF), 3-aminomethyltyrosine, and the like.

Examples of amino acids having the functional group of (C-2) include 5-hydroxytryptophan (WOH) and the like.

Examples of cyclization methods using an amino acid having the functional group of (C-1) and an amino acid having the functional group of (C-2) include the methods taught in Yamagishi, Y et al., Chembiochem 10, 1469-1472 (2009), WO 2008/117833, and the like.

Examples of amino acids having the functional group of (D-1) include 2-amino-6-chloro-hexynoic acid, 2-amino-7-chloro-heptynoic acid, 2-amino-8-chloro-octynoic acid, and the like.

Examples of amino acids having the functional group of (D-2) include cysteine, homocysteine, mercaptonorvaline, mercaptonorleucine, 2-amino-7-mercaptoheptanoic acid, 2-amino-8-mercaptooctanoic acid, and the like.

Examples of cyclization methods using an amino acid having the functional group of (D-1) and an amino acid having the functional group of (D-2) include the methods taught described in WO 2012/074129 and the like.

Examples of the amino acid of (E-1) include N-3-chloromethylbenzoyl-L-phenylalanine, N-3-chloromethylbenzoyl-L-tyrosine, N-3-chloromethylbenzoyl-L-tryptophan, and D-amino acid derivatives and the like corresponding thereto.

Examples of the amino acid of (E-2) include cysteine, homocysteine, mercaptonorvaline, mercaptonorleucine, 2-amino-7-mercaptoheptanoic acid, 2-amino-8-mercaptooctanoic acid, and the like.

A cyclization method using an amino acid having the functional group of (E-1) and an amino acid having the functional group of (E-2) may be performed, for example, by referring to the cyclization method of (A-1) and (A-2) or the cyclization method of (D-1) and (D-2).

When the library preparation process is a process for preparing a library of compounds containing cyclic peptides, each mRNA in the mRNA library preferably contains, for example, a sequence shown in Table 5 below. In Table 5 below, N1, N2, K, R, and B have the same meaning as Table 3 above, m3 and m4 are each integer of 0 or more and 46 or less, and (linker) is a given sequence. m3 and m4 may be selected within a range such that the sum of m3 and m4 is 0 or more and 46 or less. The sum of m1 and m2 may be 1 or more, 2 or more, 3 or more, or 6 or more, and may be 41 or less, 36 or less, 26 or less, or 16 or less.

[Table 5]

| | | | | | | |
|---|---|---|---|---|---|---|
| **mRNA lib:** | **AUG** | **$(N1N2K)_{m3}$** | **RBU** | **CAU $(N1N2K)_{m4}$** | **UGU (linker)** | **UAG** |
| **Peptide:** | **ClAc-$^{D/L}$Y/W/F** | | **A/I/G/T/V/S** | **His** | **Cys** | **Stop** |

Note that in the above table, an N-chloroacetyl amino acid is assigned to the AUG codon, and a cyclic peptide is formed by a chloroacetyl group of this amino acid and a mercapto group of Cys present downstream. In the above table, D-, L-Tyr, Trp, or Phe is assigned to the AUG codon, but the amino acid to which such is assigned is not limited to Tyr, Trp, or Phe.

For example, the flexizyme described above may be used to assign an N-chloroacetyl amino acid to the AUG codon.

In the library preparation process, the mRNA library prepared in the mRNA preparation process is subsequently translated (hereinafter referred to as the "translation process"). The translation process may be performed, for example, in a cell-free translation system including tRNA having an anticodon corresponding to any of the N1N2N3 codons and having an amino acid assigned to the codon charged thereto. There may, for example, be 20 types of the N1N2N3 codon and tRNA contained in the cell-free translation system.

The cell-free translation system may be a translation system obtained by freely removing constituent factors of an existing translation system in accordance with a target or by freely adding other constituent factors and reconstructing only the necessary components. For example, when a translation system from which a specific amino acid has been

removed is reconstructed, a codon corresponding to the amino acid becomes a vacant codon that does not encode any amino acid. Therefore, when a given amino acid is charged to tRNA having an anticodon complementary to the empty codon by using flexizyme or the like and translation is performed by adding such, the given amino acid is encoded by that codon. Thus, a peptide having the given amino acid introduced therein can be prepared instead of the removed amino acid.

The tRNA in the cell-free translation system may be wild type tRNA derived from an organism (for example, *Escherichia coli*) or may be artificial tRNA prepared by transcribing *in vitro.*

The 20 types of N1N2N3 corresponding to the 20 types of tRNA codons used in the cell-free translation system may have the same sequence except for the anticodon loop portion. According to such an aspect, the reactivity of a specific tRNA is not increased or decreased, each tRNA has uniform reactivity, and it becomes possible to express a desired peptide with high reproducibility.

Note that "cell-free translation system" in the present specification refers to a translation system that does not contain cells, and an *Escherichia coli* extract solution, a wheat embryo extract solution, a rabbit erythrocyte extract solution, an insect cell extract solution, or the like may be used as the cell-free translation system. Furthermore, a reconstitutable cell-free translation system may be used that is constructed by reconstructing ribosome proteins, aminoacyl tRNA synthase (aaRS), ribosome RNA, amino acids, rRNA, GTP, ATP, translation initiation factor (IF) elongation factor (EF), release factor (RF), ribosome regeneration factor (RRF), and other factors necessary for translation.

A cell-free translation system containing RNA polymerase may be used from the viewpoint of performing transcription from DNA as well.

Examples of commercially available cell-free translation systems include RTS-100 (registered trademark) from Roche Diagnostics, PURESYSTEM (registered trademark) from PGI, PUREfrex from Frontiersmen, and PURExpress In Vitro Protein Synthesis Kit from New England BioLabs, which are commercially available cell-free translation system derived from *Escherichia coli,* and those from ZOEGENE or CellFree Sciences, which are systems using a wheat embryo extract solution.

Furthermore, for example, the art described in the following documents is known as systems that use ribosomes of *Escherichia coli*: H. F. Kung et al., 1977. The J. Biol. Chem. Vol. 252, No. 19, 6889-6894; M. C. Gonza et al., 1985, Proc. Natl. Acad. Sci. U.S.A. Vol. 82, 1648-1652; M. Y Pavlov and M. Ehrenberg, 1996, Arch. Biochem. Biophys. Vol. 328, No. 1, 9-16; Y Shimizu et al., 2001, Nat. Biotechnol. Vol. 19, No. 8, 751-755; H. Ohashi et al., 2007, Biochem. Biophys. Res. Commun. Vol. 352, No. 1, 270-276.

Cell-free translation systems include tRNA for extension and may further include start tRNA. The present inventors have to this point constructed a translation system in which N1N2N3 encodes a given amino acid by means of, for example, the reassignment of codons using a flexizyme.

In a natural translation system, tRNA having an anticodon corresponding to each amino acid is present, and each tRNA has a unique sequence in a region other than the anticodon loop, but when using a flexizyme to reassign any amino acid to all of N1N2N3, all tRNA may be artificial. In this case, the elongation tRNA corresponding to each N1N2N3 included in the translation system may be that composed of a base sequence for which 80% or more, 85% or more, 88% or more, or 90% or more of the total length is identical. That is, of the sequence excluding the anticodon, an elongation tRNA group having substantially the same sequence can be used. The elongation tRNA group may have entirely the same base sequence other than the anticodon loop. The elongation tRNA corresponding to each N1N2N3 added to the translation system may be that in which 85% or more, 88% or more, 90% or more, 93% or more, 95% or more, 98% or more, or 99% or more of portions other than the anticodon loop is identical.

In the present specification, "anticodon loop" indicates a single-stranded loop portion that includes an anticodon in tRNA. It is possible for a person skilled in the art to appropriately determine the sequence of the anticodon loop so as to augment interaction between the codon and anticodon.

According to the method using a cell-free translation system as described above, there is a tendency to be able to obtain an expression product in a highly pure form without purification. Furthermore, based on the diversity of N1N2N3 in the mRNA, a library containing $1\times10^{12}$ to $1\times10^{13}$ different peptides or more can be produced.

The library preparation process may be a process for preparing a compound library containing a genotype-bound peptide or protein through a display method, particularly through an mRNA display method.

Display method refers to a system that enables a phenotype to be displayed in a genotype encoding the sequence thereof and activated species to be concentrated and amplified (that is, selected) using a replication system reconstructed in a test tube by linking the phenotype and the genotype by a covalent or non-covalent bond.

Examples of the display method include a phage display using *Escherichia coli* as a replication career, a yeast display, or the like. Furthermore, the display method includes *in vitro* display that does not use a prokaryotic or eukaryotic organism as a career, and in particular, according to the *in vitro* display, it is possible to search for a wider variety of libraries than with the phage display.

Examples of the *in vitro* display include a ribosome display, a cDNA display, an mRNA display, and the like.

It is preferable that the process for preparing the compound library comprising proteins or peptides bound to

a genotype by an mRNA display method includes a process for preparing an mRNA library of respective mRNA that encode the peptides or proteins having an amino acid sequence that contains at least one His, Tyr, or derivative thereof, a process for binding puromycin to the 3' terminal of the respective mRNA in the mRNA library and producing a puromycin-bound mRNA library, and a process for translating the puromycin-bound mRNA library through the cell-free translation system.

The process of preparing the mRNA library and the process of translating the puromycin-bound mRNA library through the cell-free translation system may be performed in the same manner as the mRNA preparation process and the translation process each described above.

The process for producing the puromycin-bound mRNA library may be carried out by binding puromycin to the downstream region of the open leading frame (ORF) of each mRNA when preparing the mRNA library in the method for producing the peptide library described above. The puromycin may be bound to mRNA via a linker comprising a peptide or a nucleic acid. By binding puromycin to the ORF on the downstream region of mRNA, ribosomes which translated it take in puromycin, and then ribosomes form a complex of an mRNA and a peptide. Such a peptide-mRNA complex can connect genotypes with phenotypes and can be applied to *in vitro* displays.

(Compound Library)

One aspect of the present embodiment is a compound library produced by the production method described above. The compound library of the present embodiment includes the library of compounds containing peptides or proteins described above, and a library of complexes of the compounds containing peptides or proteins and mRNA.

[Screening Method]

One aspect of the present embodiment is a method for screening compounds to identify a compound that binds to a target substance, producing a compound library by any of the production methods described above, preferably contacting a library of complexes of compounds containing peptides or proteins and mRNA to the target substance and selecting a compound that binds to the target substance.

The process for contacting the compound to the target substance may be, for example, a method of incubating a system in which the compound and the target substance coexist, a method of fixing the target substance to a solid-phase carrier and bringing a liquid phase containing the compound into contact with the solid-phase carrier, or the like. The contact may be performed in a suitably selected buffer solution and interact by adjusting the pH, temperature, contact time, and the like.

In the present specification, the "solid-phase carrier" is not particularly limited so long as it is a carrier that can fix a target substance, and examples include microtiter plates made of glass, metal, resin, or the like; substrates; beads; nitrocellulose membranes; nylon membranes; PVDF membranes; and the like. The target substance may be fixed to these solid-phase carriers by a known method.

In the present specification, the "target substance" is not particularly limited and may be a low molecular weight compound, a high molecular weight compound, a nucleic acid, a peptide, a protein, a sugar, a lipid, or the like. In particular, the above compound library may be used when the target substance has protease activity or is an intracellular molecule.

The process of selecting a compound that binds to the target substance may be carried out by, for example, labeling the compound in a detectable manner according to a known method, washing the surface of the solid-phase carrier with a buffer solution after the process of bringing the above compound into contact with the target substance, and detecting the compound that binds to the target substance.

Examples of the detectable label include enzymes such as peroxidases, alkaline phosphatase, and the like; radioactive substances such as $^{131}I$, $^{35}S$, $^{18}F$, $^{3}H$, and the like; fluorescent substances such as fluorescein isothiocyanate, rhodamine, dansyl chloride, phycoerythrin, tetramethyl rhodamine isothiocyanate, near-infrared fluorescent materials, and the like; light-emitting substances such as luciferase, luciferin, aequorin, and the like; and nanoparticles such as gold colloid, quantum dots, and the like. When an enzyme is used as a label, detection may also be performed by contacting a substrate with the enzyme to develop color. Alternatively, detection may be performed by binding biotin to the compound and binding avidin or streptavidin labeled with an enzyme or the like.

Moreover, it is possible to not only detect and measure the presence or absence or strength of binding, but also to measure the acceleration or inhibition of the activity of the target substance and identify a compound having such an acceleration or inhibition activity. Such a method also makes it possible to identify a compound having physiological activity that is useful as a medicine.

In the screening method of the present embodiment, a display method may be applied to the process for selecting a compound that binds to the target substance. In the screening method of the present embodiment, when the library of compounds containing mRNA is used, an mRNA display method may be applied to the process for selecting

a compound that binds to the target substance.

In the mRNA display method, a cDNA library is obtained by performing reverse transcription on the library of compounds containing mRNA. Each cDNA in the cDNA library encodes a peptide or a protein that binds to the target molecule.

The mRNA library may be obtained once more by amplifying the cDNA library and translating such. In the mRNA library obtained once more, the concentration of a molecule that binds to a target molecule is higher than that in the original mRNAlibrary. Therefore, by repeating the process described above a plurality of times, molecules that bind to the target molecule can be gradually concentrated.

Since the amino acid sequence of the concentrated peptide or protein can be identified by analyzing the sequence of cDNA, a compound having a high affinity for the target molecule can be easily selected based on the sequence information.

The RaPID system (Yamagishi, Y et al., Chem. Biol., 2011, 18(12), 1562-70) is an example of a screening system combining an FIT system used in an example described below with the mRNA display method.

(Screening Kit)

Another aspect of the present embodiment is a compound screening kit. One aspect of the screening kit of the present embodiment includes a compound library produced by the production method described above.

In addition to the above compound library, the screening kit may contain a reagent and device necessary for detecting binding between the target substance and the compound. Examples of such reagents and devices include solid-phase carriers, buffer solutions, labeling reagents, enzymes, enzyme reaction stopping solutions, and microplate readers; however, the present invention is not limited thereto.

Aspects described in the present specification and aspects described in the present specification as preferred embodiments and the like may be arbitrarily combined in any of the above embodiments described in the present specification.

Examples

The present invention will be described in further detail below using examples and comparative examples. The present invention is not intended to be limited in any way by the following examples.

[Materials]

Unless otherwise specified, the reagents and the like used in the examples were purchased from Nacalai Tesque, Wako Pure Chemical Industries, Sigma-Aldrich Japan, Kanto Chemical, or Watanabe Chemical Industries, and used without being purified. Geranyl pyrophosphate was purchased from Isopronoid, LC. Farnesyl pyrophosphate was purchased from Sigma-Aldrich Japan. All oligo primers were purchased from Eurofins Genomics (OPC purified grade).

[General Experimental Procedure]

Liquid chromatography-mass spectrometry (LC-MS) experiments for the analysis of synthetic substrates and enzyme reaction products were performed using a Waters Xevo G2-XS QToF device equipped with an Acquity I-Class UPLC system. Injected samples were separated by an Acquity UPLC Petide BEH C18 column (300Å, 1.7 μm, 2.1 mm×150 mm) using the following gradients: 1% B×2 minutes; 1 to 61% B×6 minutes; 95% B×1 minute; 1% B×3 minutes (total runtime: 12 minutes) or 1% B×2 minutes; 1 to 81% B×16 minutes; 95% B×1 minute; 1% B×3 minutes (total runtime: 22 minutes) (mobile phase A: 0.1% (v/v) formic acid aqueous solution; and B: 0.1% (v/v) formic acid acetonitrile solution). The samples were then introduced directly into the Xevo QTof MS.

All MS analyses were performed in positive mode with a capillary voltage of 0.7 kV, and nitrogen was used as a cone gas (50 L/min) and a desolvation gas (1,000 L/min). The temperature of the ionization source was 120°C, and the desolvation gas was 400°C. In the MS/MS analysis, spectra were obtained with a scan time of 0.3 seconds and selected ions were subjected to a lamp of 15 to 30 or 25 to 40 eV collision energy. The LC-MS data was analyzed using MassLynx 4.1, and MS/MS spectral assignment was performed manually. The m/z allowable error was set to 0.10 when generating an extracted ion chromatogram (EIC) having selected ions. In the purification of the synthetic substrate and the enzyme reaction product, an LC-20AP device (Shimadzu) equipped with a Chromolith Prep column (100×25 mm, C18 phase, Merck) was used together with a mobile phase A: 0.1% (v/v) trifluoroacetic acid (TFA) aqueous solution and a B: 0.1% (v/v) TFA acetonitrile solution.

[Method for Expressing and Purifying Prenyltransferase]

A LimF synthetic gene optimized for codons was purchased from Eurofins Genomics and a pET32 -LimF-SMS plasmid was produced by using an In-Fusion HD Cloning kit (TaKaRa Clontech) to introduce cDNA encoding SUMOstar-LimF into the pET32 vector (Novagen).

In order to express the LimF-SUMO fusion protein in *Escherichia coli,* the pET32-LimF-SMS plasmid was transformed into *E. coli* BL21 Gold (DE3) using 100 mg/L ampicillin sodium as a selection marker. The obtained transformant was cultured in a 2xYT medium containing 100 mg/L ampicillin sodium. The culture was incubated at 37°C while shaking at 160 rpm until OD600 reached 0.8. Next, the culture was cooled on ice, and immediately isopropyl-β-D-1-thiogalactopyranoside (IPTG) and ethanol were added at the final concentrations of 0.2 mM and 3% (v/v), respectively. After inducing IPTG, the culture was incubated for 20 hours at 20°C and 180 rpm, and cell pellets were then recovered by centrifugation at 4°C (6,000×g, 15 minutes). The pellets were re-suspended in 100 mL of lysis buffer (40 mM $K_2HPO_4$, 10 mM $KH_2PO_4$, 500 mM NaCl, 20 mM imidazole, 1 mM DTT, 0.1 mM PMSF, 0.5% triton X-100, pH 7.5) and then destroyed by ultrasonic treatment on ice. After centrifugation (20,000×g, 30 minutes) at 4°C, filtering was performed, and a soluble fraction was collected

The collected soluble fraction was injected into a Histrp HP 5 mL column (Cytiva) that was pre-equilibrated using a buffer solution A (40 mM $K_2HPO_4$, 10 mM $KH_2PO_4$, 500 mM NaCl, 20 mM imidazole, 1 mM DTT, pH 7.5). A His tag LimF-SUMO fusion protein was eluted using a buffer solution A containing 200 mM imidazole. Next, the protein was dialyzed for 6 hours at 4°C in relation to a buffer solution B (20 mM Tris-HCl, pH 7.5, 150 mM NaCl, 1 mM DTT). Following this, SUMOstar protease was added to the dialysis membrane and dialyzed for 6 hours at 4°C to remove the SUMO tag. Next, tag-free LimF was injected into a Hitrap SP 5 mL column (Cytiva) that was pre-equilibrated using a buffer solution C (10 mM Hepes pH 7.5, 150 mM NaCl, 1 mM DTT), and eluted using a buffer solution C containing 250 mM NaCl. Next, the purified fraction was injected into a HiLoad 16/60 Superdex 200 prepacked gel filtration column (Cytiva) at 4°C and eluted in buffer solution C. Next, the tag-free LimF fraction was concentrated using an Amicon centrifugal filter (Merck). The purity of the protein was confirmed by SDS-PAGE analysis, and the protein concentration was measured using an absorbance of 280 nm calculated by the ExPASy ProtParam tool. Next, the protein was rapidly frozen in liquid nitrogen and preserved at - 80°C.

[Method for Chemically Synthesizing Peptide]

Cyclic peptides bcLimE1, E2, and E3 used in the examples described below were synthesized using a standard Fmoc Solid-phase peptide synthesis (SPPS) protocol on a Syro I automatic synthesis device (Biotage). 2-chlorotrityl chloride resin was pre-charged with Fmoc-Tyr(tBu)-OH (0.877 mmol/g loading), and the peptides were synthesized as C-terminal acids using a resin of 0.025 mmol. The following combinations of side-chain protecting groups were used: Asp (OtBu), Glu (OtBu), His (Boc), Lys (Boc), Asn (Trt), Gin (Trt), Arg (Pbf), Ser (tBu), Thr (tBu), and Tyr (tBu).

A peptide coupling reaction was carried out using 2-(1H-benzotriazol-1-yl)-1,1,3,3-tetramethyluronium hexafluorophosphate (HBTU) as an activator and diisopropylethylamine (DIPEA) as a base. Fmoc-deprotection was performed using a 20% (v/v) piperidine DMF solution. Unless otherwise described, all of the reagents were dissolved in dimethylformamide (DMF), and reactions were performed in DMF.

Following SPPS, the resin was washed four times with dichloromethane (DCM) and dried in a vacuum. A fully protected linear precursor peptide was released from the resin by reacting the resin for three hours at room temperature in a 20% 1,1,1,3,3,3-hexafluoro-2-propanol (HFIP) DCM solution containing 5 mL of 2.5% triisopropylsilane (TIS). 40mL of ice-cooled diethyl ether was added to a solution containing the released peptide, and then the peptide was precipitated by being allowed to stand for 30 minutes at -20°C. Peptide pellets were collected by manual centrifugation and then washed four times using 10 mL of ice-cooled diethyl ether. The washed pellets were dried overnight in a draft, and the residual diethyl ether was removed.

Each linear precursor peptide pellet was dissolved in 10 mL of DMF. 1.1 equivalents of benzotriazole-1-yloxytripyrrolidinophosphonium hexafluorophosphate (PyBOP) and 2.2 equivalents of DIPEA were added, and a cyclization reaction was performed for 6 hours at room temperature. After cyclization, 10 mL of DCM was added and extracted twice in 100 mL of water. The organic phase containing the peptide was collected, and the solvent was removed by a rotary evaporator.

Next, the side-chain protecting group was deprotected by reacting at room temperature for 3 hours using 5 mL of TFA/water/TIS (95:2.5:2.5, v/v) solution. The deprotected peptide was precipitated using diethyl ether as described above, and then further washed with diethyl ether. The dried pellets were dissolved in 5 mL of dimethyl sulfoxide (DMSO) containing 0.1% TFA, filtered, and purified by preparative reverse phase HPLC. A fraction containing the desired product was recovered, lyophilized, and then redissolved in DMSO to obtain a peptide solution.

For synthesis of short peptides, C-terminal amidated peptides were synthesized by the same SPPS method as described above using NovaPEG Rink Amide resin (0.44 mmol/g loading). After extending the peptide chain, the N-terminal was acetylated by reacting the resin for 60 minutes at room temperature in NMP 2 mL containing 0.5 M $Ac_2O$ and 0.25 M DIPEA. Peptides were released from the resin by washing the resin three times with DMF and four times with DCM, and then reacting for three hours at room temperature in a TFA/water/TIS solution. A purified peptide was obtained by the same post-treatment and purification procedure as described above. A previously reported method was used to synthesize thioether cyclic peptides (Passioura, T.; Liu, W.; Dunkelmann, D.; Higuchi, T.; Suga, H. Display selection of exotic macrocyclic peptides expressed under a radically reprogrammed 23 amino acid genetic code. J. Am. Chem. Soc., 2018, 140 (37), 11551-11555).

[Method for Preparing Flexizymes and tRNA]\

dFx, eFx, $tRNA^{GluE2}_{GUG}$, and $tRNA^{fMet}_{CAU}$ were prepared as previously reported by *in vitro* transcription using T7 RNA polymerase (Goto, Y; Katoh, T.; Suga, Nat. Protoc., Flexizymes for genetic code reprogramming. 2011, 6 (6), 779-790.)

[Method for Preparing Aminoacylated tRNA Using Flexizymes]

$tRNA^{fMet}_{CAU}$ was charged with ClAc-D-Phe and ClAc-D-Tyr using eFx; $tRNA^{GluE2}_{GUG}$ was charged with 3-Me-L-His, 1-Me-L-His, L-Ala(2-Thi), L-Ala(3-Thi), and $\alpha$-Me-D/L-His using eFx; and $tRNA^{GluE2}_{GUG}$ was charged with L-His, D-His, and L-Ala(4-Thz) using dFx. An aminoacylation reaction was performed in a solution containing 25 $\mu$M flexizymes (eFx or dFx), 25 $\mu$M tRNA, 5 mM activated amino acid donor (eFx reaction: cyanomethyl ester, dFx reaction: 3,5-dinitrobenzyl ester), 100 mM HEPES-KOH pH 7.5, 600 mM MgClz, and 20% DMSO. The activated amino acids were prepared as previously reported (Goto, Y; Katoh, T.; Suga, Nat. Protoc., Flexizymes for genetic code reprogramming. 2011, 6 (6), 779-790.; Passioura, T.; Liu, W.; Dunkelmann, D.; Higuchi, T.; Suga, H. J. Am. Chem. Soc., Display selection of exotic macrocyclic peptides expressed under a radically reprogrammed 23 amino acid genetic code. 2018, 140 (37), 11551-11555).

Each aminoacylation reaction mixture was incubated on ice for 2 hours (ClAc-D-Phe and ClAc-D-Tyr) or 6 hours (other amino acids), and then 4 times the amount of 0.3 M sodium acetate (pH 5.2) and 10 times the amount of EtOH were added. Next, centrifugation (15,300$\times$g) was performed for 15 minutes at room temperature, and the supernatant was discarded. Aminoacyl-tRNA pellets were washed twice with 70% EtOH containing 0.1 M sodium acetate (pH 5.2) and once with 70% EtOH. The obtained aminoacyl-tRNA was dried for 10 minutes. The aminoacyl-tRNA was dissolved in 1 mM sodium acetate (pH 5.2) immediately before being added to the translation mixture to produce a 250 $\mu$M solution.

[Method for Preparing DNA Template for Substrate]

The template DNA used in the following examples was prepared using the primers shown in Tables 6 (Tables 6-1 and 6-2) and 7 (Tables 7-1 to 7-4) below. Straight chain double-stranded DNA having a T7 promoter upstream of a sequence encoding a thioether ring-closing cyclic peptide (hereinafter referred to as "teMP") was prepared using Taq DNA polymerase in the same way as previously reported (Vinogradov, A. A.; Shimomura, M.; Goto, Y; Ozaki, T.; Asamizu, S.; Sugai, Y; Suga, H.; Onaka, Nat. Commun., Minimal lactazole scaffold for in vitro thiopeptide bioengineering. 2020, 11(1), 1-13).

[Table 6-1]

| Name | Extension | | PCR | |
|---|---|---|---|---|
| | Forward primer | Reverse primer | Forward primer | Reverse primer |
| teLimE2 | T7exF43 | teLimE2-R1 | T7pcrF22 | teLimE2-R2 |
| teLimE2(P-1A) | T7exF43 | teLimE2(P-1A)-R1 | T7pcrF22 | teLimE2-R2 |
| teLimE2(P-1D) | T7exF43 | teLimE2(P-1D)-R1 | T7pcrF22 | teLimE2-R2 |
| teLimE2(P-1E) | T7exF43 | teLimE2(P-1E)-R1 | T7pcrF22 | teLimE2-R2 |
| teLimE2(P-1F) | T7exF43 | teLimE2(P-1F)-R1 | T7pcrF22 | teLimE2-R2 |
| teLimE2(P-1G) | T7exF43 | teLimE2(P-1G)-R1 | T7pcrF22 | teLimE2-R2 |
| teLimE2(P-1H) | T7exF43 | teLimE2(P-1H)-R1 | T7pcrF22 | teLimE2-R2 |
| teLimE2(P-1I) | T7exF43 | teLimE2(P-1I)-R1 | T7pcrF22 | teLimE2-R2 |
| teLimE2(P-1K) | T7exF43 | teLimE2(P-1K)-R1 | T7pcrF22 | teLimE2-R2 |
| teLimE2(P-1L) | T7exF43 | teLimE2(P-1L)-R1 | T7pcrF22 | teLimE2-R2 |
| teLimE2(P-1M) | T7exF43 | teLimE2(P-1M)-R1 | T7pcrF22 | teLimE2-R2 |
| teLimE2(P-1N) | T7exF43 | teLimE2(P-1N)-R1 | T7pcrF22 | teLimE2-R2 |
| teLimE2(P-1Q) | T7exF43 | teLimE2(P-1Q)-R1 | T7pcrF22 | teLimE2-R2 |
| teLimE2(P-1R) | T7exF43 | teLimE2(P-1R)-R1 | T7pcrF22 | teLimE2-R2 |
| teLimE2(P-1S) | T7exF43 | teLimE2(P-1S)-R1 | T7pcrF22 | teLimE2-R2 |
| teLimE2(P-1T) | T7exF43 | teLimE2(P-1T)-R1 | T7pcrF22 | teLimE2-R2 |
| teLimE2(P-1V) | T7exF43 | teLimE2(P-1V)-R1 | T7pcrF22 | teLimE2-R2 |
| teLimE2(P-1W) | T7exF43 | teLimE2(P-1W)-R1 | T7pcrF22 | teLimE2-R2 |
| teLimE2(P-1Y) | T7exF43 | teLimE2(P-1Y)-R1 | T7pcrF22 | teLimE2-R2 |
| teLimE2(E+1A) | T7exF43 | teLimE2(E+1A)-R1 | T7pcrF22 | teLimE2(E+1A)-R2 |
| teLimE2(E+1D) | T7exF43 | teLimE2(E+1D)-R1 | T7pcrF22 | teLimE2(E+1D)-R2 |
| teLimE2(E+1F) | T7exF43 | teLimE2(E+1F)-R1 | T7pcrF22 | teLimE2(E+1F)-R2 |
| teLimE2(E+1G) | T7exF43 | teLimE2(E+1G)-R1 | T7pcrF22 | teLimE2(E+1G)-R2 |
| teLimE2(E+1H) | T7exF43 | teLimE2(E+1H)-R1 | T7pcrF22 | teLimE2(E+1H)-R2 |
| teLimE2(E+1I) | T7exF43 | teLimE2(E+1I)-R1 | T7pcrF22 | teLimE2(E+1I)-R2 |
| teLimE2(E+1K) | T7exF43 | teLimE2(E+1K)-R1 | T7pcrF22 | teLimE2(E+1K)-R2 |
| teLimE2(E+1L) | T7exF43 | teLimE2(E+1L)-R1 | T7pcrF22 | teLimE2(E+1L)-R2 |
| teLimE2(E+1M) | T7exF43 | teLimE2(E+1M)-R1 | T7pcrF22 | teLimE2(E+1M)-R2 |
| teLimE2(E+1N) | T7exF43 | teLimE2(E+1N)-R1 | T7pcrF22 | teLimE2(E+1N)-R2 |
| teLimE2(E+1P) | T7exF43 | teLimE2(E+1P)-R1 | T7pcrF22 | teLimE2(E+1P)-R2 |
| teLimE2(E+1Q) | T7exF43 | teLimE2(E+1Q)-R1 | T7pcrF22 | teLimE2(E+1Q)-R2 |
| teLimE2(E+1R) | T7exF43 | teLimE2(E+1R)-R1 | T7pcrF22 | teLimE2(E+1R)-R2 |
| teLimE2(E+1S) | T7exF43 | teLimE2(E+1S)-R1 | T7pcrF22 | teLimE2(E+1S)-R2 |
| teLimE2(E+1T) | T7exF43 | teLimE2(E+1T)-R1 | T7pcrF22 | teLimE2(E+1T)-R2 |
| teLimE2(E+1V) | T7exF43 | teLimE2(E+1V)-R1 | T7pcrF22 | teLimE2(E+1V)-R2 |
| teLimE2(E+1W) | T7exF43 | teLimE2(E+1W)-R1 | T7pcrF22 | teLimE2(E+1W)-R2 |
| teLimE2(E+1Y) | T7exF43 | teLimE2(E+1Y)-R1 | T7pcrF22 | teLimE2(E+1Y)-R2 |
| teMP(3aa) | T7exF43 | teMP(3aa)-R1 | T7pcrF22 | teMP(3aa)-R2 |
| teMP(4aa) | T7exF43 | teMP(4aa)-R1 | T7pcrF22 | teMP(4aa)-R2 |
| teMP(5aa) | T7exF43 | teMP(5aa)-R1 | T7pcrF22 | teMP(5aa)-R2 |
| teMP(6aa) | T7exF43 | teMP(6aa)-R1 | T7pcrF22 | teMP(6aa)-R2 |

[Table 6-2]

| Name | Extension | | PCR | |
|---|---|---|---|---|
| | Forward primer | Reverse primer | Forward primer | Reverse primer |
| teMP(7aa) | T7exF43 | teMP(7aa)-R1 | T7pcrF22 | teMP(7aa)-R2 |
| teMP(8aa) | T7exF43 | teMP(8aa)-R1 | T7pcrF22 | teMP(8aa)-R2 |
| teMP(9aa) | T7exF43 | teMP(9aa)-R1 | T7pcrF22 | teMP(9aa)-R2 |
| teMP(10aa) | T7exF43 | teMP(10aa)-R1 | T7pcrF22 | teMP(10aa)-R2 |
| teMP(11aa) | T7exF43 | teMP(11aa)-R1 | T7pcrF22 | teMP(11aa)-R2 |
| teMP(12aa) | T7exF43 | teMP(12aa)-R1 | T7pcrF22 | teMP(12aa)-R2 |
| teMP(13aa) | T7exF43 | teMP(13aa)-R1 | T7pcrF22 | teMP(13aa)-R2 |
| teMP(14aa) | T7exF43 | teMP(14aa)-R1 | T7pcrF22 | teMP(14aa)-R2 |
| teMP(16aa) | T7exF43 | teMP(16aa)-R1 | T7pcrF22 | teMP(16aa)-R2 |
| teMP(18aa) | T7exF43 | teMP(18aa)-R1 | T7pcrF22 | teMP(18aa)-R2 |
| teR1 | T7exF43 | teR1-R1 | T7pcrF22 | teR1-R2 |
| teR2 | T7exF43 | teR2-R1 | T7pcrF22 | teR2-R2 |
| teR3 | T7exF43 | teR3-R1 | T7pcrF22 | teR3-R2 |
| teR4 | T7exF43 | teR4-R1 | T7pcrF22 | teR4-R2 |
| teR5 | T7exF43 | teR5-R1 | T7pcrF22 | teR5-R2 |
| teR1(P-1K) | T7exF43 | teR1(P-1K)-R1 | T7pcrF22 | teR1(P-1K)-R2 |
| teR2(W+1P) | T7exF43 | teR2(W+1P)-R1 | T7pcrF22 | teR2(W+1P)-R2 |
| teR3(E-1S) | T7exF43 | teR3-R1 | T7pcrF22 | teR3(E-1S)-R2 |
| teR4(W-1A) | T7exF43 | teR4-R1 | T7pcrF22 | teR4(W-1A)-R2 |
| teR5(P+1A) | T7exF43 | teR5(P+1A)-R1 | T7pcrF22 | teR5(P+1A)-R2 |
| teLimE2-CAC | T7exF43 | teLimE2-CAC-R1 | T7pcrF22 | teLimE2-CAC-R2 |
| teR6 | T7exF43 | teR6-R1 | T7pcrF22 | teR6-R2 |
| teR6-H5A | T7exF43 | teR6-H5A-R1 | T7pcrF22 | teR6-H5A-R2 |
| teR6-H5A(T-1A) | T7exF43 | teR6-H5A(T-1A)-R1 | T7pcrF22 | teR6-H5A(T-1A)-R2 |
| teR6-H5A(T-1D) | T7exF43 | teR6-H5A(T-1D)-R1 | T7pcrF22 | teR6-H5A(T-1D)-R2 |
| teR6-H5A(T-1E) | T7exF43 | teR6-H5A(T-1E)-R1 | T7pcrF22 | teR6-H5A(T-1E)-R2 |
| teR6-H5A(T-1F) | T7exF43 | teR6-H5A(T-1F)-R1 | T7pcrF22 | teR6-H5A(T-1F)-R2 |
| teR6-H5A(T-1G) | T7exF43 | teR6-H5A(T-1G)-R1 | T7pcrF22 | teR6-H5A(T-1G)-R2 |
| teR6-H5A(T-1H) | T7exF43 | teR6-H5A(T-1H)-R1 | T7pcrF22 | teR6-H5A(T-1H)-R2 |
| teR6-H5A(T-1I) | T7exF43 | teR6-H5A(T-1I)-R1 | T7pcrF22 | teR6-H5A(T-1I)-R2 |
| teR6-H5A(T-1K) | T7exF43 | teR6-H5A(T-1K)-R1 | T7pcrF22 | teR6-H5A(T-1K)-R2 |
| teR6-H5A(T-1L) | T7exF43 | teR6-H5A(T-1L)-R1 | T7pcrF22 | teR6-H5A(T-1L)-R2 |
| teR6-H5A(T-1M) | T7exF43 | teR6-H5A(T-1M)-R1 | T7pcrF22 | teR6-H5A(T-1M)-R2 |
| teR6-H5A(T-1N) | T7exF43 | teR6-H5A(T-1N)-R1 | T7pcrF22 | teR6-H5A(T-1N)-R2 |
| teR6-H5A(T-1P) | T7exF43 | teR6-H5A(T-1P)-R1 | T7pcrF22 | teR6-H5A(T-1P)-R2 |
| teR6-H5A(T-1Q) | T7exF43 | teR6-H5A(T-1Q)-R1 | T7pcrF22 | teR6-H5A(T-1Q)-R2 |
| teR6-H5A(T-1R) | T7exF43 | teR6-H5A(T-1R)-R1 | T7pcrF22 | teR6-H5A(T-1R)-R2 |
| teR6-H5A(T-1S) | T7exF43 | teR6-H5A(T-1S)-R1 | T7pcrF22 | teR6-H5A(T-1S)-R2 |
| teR6-H5A(T-1V) | T7exF43 | teR6-H5A(T-1V)-R1 | T7pcrF22 | teR6-H5A(T-1V)-R2 |
| teR6-H5A(T-1W) | T7exF43 | teR6-H5A(T-1W)-R1 | T7pcrF22 | teR6-H5A(T-1W)-R2 |
| teR6-H5A(T-1Y) | T7exF43 | teR6-H5A(T-1Y)-R1 | T7pcrF22 | teR6-H5A(T-1Y)-R2 |

[Table 7-1]

| Name | Base Sequence **(5' to 3')** | SEQ ID NO |
|---|---|---|
| T7pcrF22 | GGCGTAATACGACTCACTATAG | 14 |
| T7exF43 | TAATACGACTCACTATAGGGTTAACTTTAACAAGGAGAAAAAC | 15 |

(continued)

| Name | Base Sequence **(5' to 3')** | SEQ ID NO |
|---|---|---|
| teLimE2(P-1A)-R1 | ACACGGAATTTCTTCATGTGCATAACGCATGTTTTTCTCCTTGTTAAAG | 16 |
| teLimE2(P-1D)-R1 | ACACGGAATTTCTTCATGGTCATAACGCATGTTTTTCTCCTTGTTAAAG | 17 |
| teLimE2(P-1E)-R1 | ACACGGAATTTCTTCATGTTCATAACGCATGTTTTTCTCCTTGTTAAAG | 18 |
| teLimE2(P-1F)-R1 | ACACGGAATTTCTTCATGAAAATAACGCATGTTTTTCTCCTTGTTAAAG | 19 |
| teLimE2(P-1G)-R1 | ACACGGAATTTCTTCATGACCATAACGCATGTTTTTCTCCTTGTTAAAG | 20 |
| teLimE2(P-1H)-R1 | ACACGGAATTTCTTCATGATGATAACGCATGTTTTTCTCCTTGTTAAAG | 21 |
| teLimE2(P-1I)-R1 | ACACGGAATTTCTTCATGAATATARCGCATGTTTTTCTCCTTGTTAAAG | 22 |
| teLimE2(P-1K)-R1 | ACACGGAATTTCTTCATGCTTATAACGCATGTTTTTCTCCTTGTTAAAG | 23 |
| teLimE2(P-1L)-R1 | ACACGGAATTTCTTCATGCAGATAACGCATGTTTTTCTCCTTGTTAAAG | 24 |
| teLimE2(P-1M)-R1 | ACACGGAATTTCTTCATGCATATAACGCATGTTTTTCTCCTTGTTAAAG | 25 |
| teLimE2(P-1N)-R1 | ACACGGAATTTCTTCATGATTATAACGCATGTTTTTCTCCTTGTTAAAG | 26 |
| teLimE2-R1 | ACACGGAATTTCTTCATGCGGATAACGCATGTTTTTCTCCTTGTTAAAG | 27 |
| teLimE2(P-1Q)-R1 | ACACGGAATTTCTTCATGTTGATAACGCATGTTTTTCTCCTTGTTAAAG | 28 |
| teLimE2(P-1R)-R1 | ACACGGAATTTCTTCATGACGATAACGCATGTTTTTCTCCTTGTTAAAG | 29 |
| teLimE2(P-1S)-R1 | ACACGGAATTTCTTCATGAGAATAACGCATGTTTTTCTCCTTGTTAAAG | 30 |
| teLimE2(P-1T)-R1 | ACACGGAATTTCTTCATGGGTATAACGCATGTTTTTCTCCTTGTTAAAG | 31 |
| teLimE2(P-1V)-R1 | ACACGGAATTTCTTCATGAACATAACGCATGTTTTTCTCCTTGTTAAAG | 32 |
| teLimE2(P-1W)-R1 | ACACGGAATTTCTTCATGGCAATAACGCATGTTTTTCTCCTTGTTAAAG | 33 |
| teLimE2(P-1Y)-R1 | ACACGGAATTTCTTCATGATAATAACGCATGTTTTTCTCCTTGTTAAAG | 34 |
| teLimE2-R2 | CGAAGCTTAACACGGAATTTCTTCATG | 35 |
| teLimE2(E+1A)-R1 | TGCATGCGGATAACGCATGTTTTTCTCCTTGTTAAAG | 36 |
| teLimE2(E+1D)-R | GTCATGCGGATAACGCATGTTTTTCTCCTTGTTAAAG | 37 |
| teLimE2(E+1F)-R1 | AAAATGCGGATAACGCATGTTTTTCTCCTTGTTAAAG | 38 |
| teLimE2(E+1G)-R1 | ACCATGCGGATAACGCATGTTTTTCTCCTTGTTAAAG | 39 |
| teLimE2(E+1H)-R1 | ATGATGCGGATAACGCATGTTTTTCTCCTTGTTAAAG | 40 |
| teLimE2(E+1I)-R1 | AATATGCGGATAACGCATGTTTTTCTCCTTGTTAAAG | 41 |
| teLimE2(E+1K)-R1 | CTTATGCGGATAACGCATGTTTTTCTCCTTGTTAAAG | 42 |
| teLimE2(E+1L)-R1 | CAGATGCGGATAACGCATGTTTTTCTCCTTGTTAAAG | 43 |
| teLimE2(E+1M)-R1 | CATATGCGGATAACGCATGTTTTTCTCCTTGTTAAAG | 44 |
| teLimE2(E+1N)-R | ATTATGCGGATAACGCATGTTTTTCTCCTTGTTAAAG | 45 |
| teLimE2(E+1P)-R1 | CGGATGCGGATAACGCATGTTTTTCTCCTTGTTAAAG | 46 |
| teLimE2(E+1Q)-R1 | TTGATGCGGATAACGCATGTTTTTCTCCTTGTTAAAG | 47 |
| teLimE2(E+1R)-R1 | ACGATGCGGATAACGCATGTTTTTCTCCTTGTTAAAG | 48 |
| teLimE2(E+1S)-R1 | AGAATGCGGATAACGCATGTTTTTCTCCTTGTTAAAG | 49 |
| teLimE2(E+1T)-R1 | GGTATGCGGATAACGCATGTTTTTCTCCTTGTTAAAG | 50 |
| teLimE2(E+1V)-R1 | AACATGCGGATAACGCATGTTTTTCTCCTTGTTAAAG | 51 |
| teLimE2(E+1W)-R | GCAATGCGGATAACGCATGTTTTTCTCCTTGTTAAAG | 52 |
| teLimE2(E+1Y)-R1 | ATAATGCGGATAACGCATGTTTTTCTCCTTGTTAAAG | 53 |

[Table 7-2]

| Name | Base Sequence **(5' to 3')** | SEQ ID NO |
|---|---|---|
| teLimE2(E+1A)-R2 | CGAAGCTTAACACGGAATTTCTGCATGCGGATAACGCATG | 54 |
| teLimE2(E+1D)-R2 | CGAAGCTTAACACGGAATTTCGTCATGCGGATAACGCATG | 55 |
| teLimE2(E+1F)-R2 | CGAAGCTTAACACGGAATTTCAAAATGCGGATAACGCATG | 56 |
| teLimE2(E+1G)-R2 | CGAAGCTTAACACGGAATTTCACCATGCGGATAACGCATG | 57 |
| teLimE2(E+1H)-R2 | CGAAGCTTAACACGGAATTTCATGATGCGGATAACGCATG | 58 |
| teLimE2(E+1I)-R2 | CGAAGCTTAACACGGAATTTCAATATGCGGATAACGCATG | 59 |
| teLimE2(E+1K)-R2 | CGAAGCTTAACACGGAATTTCCTTATGCGGATAACGCATG | 60 |
| teLimE2(E+1L)-R2 | CGAAGCTTAACACGGAATTTCCAGATGCGGATAACGCATG | 61 |
| teLimE2(E+1M)-R2 | CGAAGCTTAACACGGAATTTCCATATGCGGATAACGCATG | 62 |
| teLimE2(E+1N)-R2 | CGAAGCTTAACACGGAATTTCATTATGCGGATAACGCATG | 63 |
| teLimE2(E+1P)-R2 | CGAAGCTTAACACGGAATTTCCGGATGCGGATAACGCATG | 64 |
| teLimE2(E+1Q)-R2 | CGAAGCTTAACACGGAATTTCTTGATGCGGATAACGCATG | 65 |
| teLimE2(E+1R)-R2 | CGAAGCTTAACACGGAATTTCACGATGCGGATAACGCATG | 66 |
| teLimE2(E+1S)-R2 | CGAAGCTTAACACGGAATTTCAGAATGCGGATAACGCATG | 67 |
| teLimE2(E+1T)-R2 | CGAAGCTTAACACGGAATTTCGGTATGCGGATAACGCATG | 68 |
| teLimE2(E+1V)-R2 | CGAAGCTTAACACGGAATTTCAACATGCGGATAACGCATG | 69 |
| teLimE2(E+1W)-R2 | CGAAGCTTAACACGGAATTTCGCAATGCGGATAACGCATG | 70 |
| teLimE2(E+1Y)-R2 | CGAAGCTTAACACGGAATTTCATAATGCGGATAACGCATG | 71 |
| teMP(18aa)-R1 | CCGGAATTTCTGCATGAGAATATGCTTGACCAACACGCATGTTTTTCTCCTTGTTAAAG | 72 |
| teMP(16aa)-R1 | CCGGAATTTCTGCATGAGAATATGCTTGAACACGCATGTTTTTCTCCTTGTTAAAG | 73 |
| teMP(14aa)-R1 | CTGCATGAGAATATGCTTGACGCATGTTTTTCTCCTTGTTAAAG | 74 |
| teMP(13aa)-R1 | CTGCATGAGAATATGCTTGACGCATGTTTTTCTCCTTGTTAAAG | 75 |
| teMP(12aa)-R1 | TTTCTGCATGAGAATATGCACGCATGTTTTTCTCCTTGTTAAAG | 76 |
| teMP(11aa)-R1 | TTTCTGCATGAGAATATGCACGCATGTTTTTCTCCTTGTTAAAG | 77 |
| teMP(10aa)-R1 | GAATTTCTGCATGAGAATAACGCATGTTTTTCTCCTTGTTAAAG | 78 |
| teMP(9aa)-R1 | GAATTTCTGCATGAGAACGCATGTTTTTCTCCTTGTTAAAG | 79 |
| teMP(8aa)-R1 | AATTTCTGCATGAGAACGCATGTTTTTCTCCTTGTTAAAG | 80 |
| teMP(7aa)-R1 | ACATTCTGCATGAGAACGCATGTTTTTCTCCTTGTTAAAG | 81 |
| teMP(6aa)-R1 | TTAACATGCATGAGAACGCATGTTTTTCTCCTTGTTAAAG | 82 |
| teMP(5aa)-R1 | CGAAGCTTAACATGCATGAGACATGTTTTTCTCCTTGTTAAAG | 83 |
| teMP(4aa)-R1 | CGAAGCTTAACAATGAGACATGTTTTTCTCCTTGTTAAAG | 84 |
| teMP(3aa)-R1 | CGAAGCTTAACAATGCATGTTTTTCTCCTTGTTAAAG | 85 |
| teMP(18aa)-R2 | CGAAGCTTAACAACGTTCTGCTTCCGGAATTTCTGCATGAGAAT | 86 |

(continued)

| Name | Base Sequence **(5' to 3')** | SEQ ID NO |
|---|---|---|
| teMP(16aa)-R2 | CGAAGCTTAACAACGTGCTTCCGGAATTTCTGCATGAGAATATG | 87 |
| teMP(14aa)-R2 | CGAAGCTTAACATGCTTCCGGAATTTCTGCATGAGAATATGCTT | 88 |
| teMP(13aa)-R2 | CGAAGCTTAACATGCCGGAATTTCTGCATGAGAATATGCTTGAC | 89 |
| teMP(12aa)-R2 | CGAAGCTTAACATGCCGGAATTTCTGCATGAGAATATGCAC | 90 |
| teMP(11aa)-R2 | CGAAGCTTAACACGGAATTTCTGCATGAGAATATGCAC | 91 |
| teMP(10aa)-R2 | CGAAGCTTAACACGGAATTTCTGCATGAGAATAA | 92 |
| teMP(9aa)-R2 | CGAAGCTTAACACGGAATTTCTGCATGAGAACGC | 93 |
| teMP(8aa)-R2 | CGAAGCTTAACAAATTTCTGCATGAGAACGCAT | 94 |
| teMP(7aa)-R2 | CGAAGCTTAACATTCTGCATGAGAACGCATGT | 95 |
| teMP(6aa)-R2 | CGAAGCTTAACATGCATGAGAACGCAT | 96 |
| teMP(5aa)-R2 | CGAAGCTTAACATGCATG | 97 |
| teMP(4aa)-R2 | CGAAGCTTAACAATGAGA | 96 |
| teMP(3aa)-R2 | CGAAGCTTAACAATGCAT | 99 |

[Table 7-3]

| Name | Base Sequence **(5'to 3')** | SEQ ID NO |
|---|---|---|
| teR1-R1 | ATTCATGCGGATAACGCGGAATCATGTTTTTCTCCTTGTTAAAG | 100 |
| teR2-R1 | ACCAATGGGTGTCACCATTCAGCATGTTTTTCTCCTTGTTAAAG | 101 |
| teR3-R1 | AGAAGCATCAAACAAAGACATGTTTTTCTCCTTGTTAAAGTTA | 102 |
| teR4-R1 | AAACTGAGACGAACCATAAAGCAAATACATGTTTTTCTCCTTGTTAAAG | 103 |
| teR5-R1 | ACGGATGCGGCGGAACAATAAGATACATGTTTTTCTCCTTGTTAAAG | 104 |
| teR1-R2 | CGAAGCTTAACATTCATGCGGATAACGCGGA | 105 |
| teR2-R2 | CGAAGCTTAACAACCCGGACCAGACCAATGGGTGTCACCATTC | 106 |
| teR3-R2 | CGAAGCTTAGCAACTATAATTAGCAATATAATGTTCAGAAGCATCAAACAAA | 107 |
| teR4-R2 | CGAAGCTTAGCACTGCGGAATATGCCAAAACTGAGACGAACCATAA | 108 |
| teR5-R2 | CGAAGCTTAGCACTCCCGAGGATACGGATGCGGCGGAACAATAA | 109 |
| teR1(P-1K)-R1 | ATGCTTATAACGCGGAATCATGTTTTTCTCCTTGTTAAAG | 110 |
| teR2 (W+1P)-R1 | ACGGATGGGTGTCACCATTCAGCATGTTTTTCTCCTTGTTAAAG | 111 |
| teRS (P+1A)-R1 | ATGCATGCGGCGGAACAATAAGATACATGTTTTTCTCCTTGTTAAAG | 112 |
| teR1(P-1K)-R2 | CGAAGCTTAACATTCATGCTTATAACGCGGAATC | 113 |
| teR2 (W+1P)-R2 | CGAAGCTTAACAACCCGGACCAGACGGATGGGTGTCACCATTC | 114 |
| teR3(E-1S)-R2 | CGAAGCTTAGCAACTATAATTAGCAATATAATGAGAAGAAGCATCAAACAAA | 115 |
| teR4(W-1A)-R2 | CGAAGCTTAGCACTGCGGAATATGTGCAAACTGAGACGAACCATAAA | 116 |
| teR5 (P+1A)-R2 | CGAAGCTTAGCACTCCCGAGGATATGCATGCGGCGGAACAATAA | 117 |
| teLimE2-CAC-R1 | GAATTTCTTGGTGCGGATAACGCATGTTTTTCTCCTTGTTAAAG | 118 |
| teLimE2-CAC-R2 | CGAAGCTTAACACGGAATTTCTTCGTGCGGAT | 119 |
| teR6-R1 | AAGCTTAACACGGAATATAGGTATGATTACCACGCATGTTTTTCTCCTTGTTAAAG | 120 |
| teR6-H5A-R1 | GAATATAGGTTGCATTACCACGCATGTTTTTCTCCTTGTTAAAG | 121 |
| teR6-H5A(T-1A)-R1 | GAATATATGCTGCATTACCACGCATGTTTTTCTCCTTGTTAAAG | 122 |
| teR6-H5A(T-1D)-R1 | GAATATAGTCTGCATTACCACGCATGTTTTTCTCCTTGTTAAAG | 123 |
| teR6-H5A(T-1E)-R1 | GAATATATTCTGCATTACCACGCATGTTTTTCTCCTTGTTAAAG | 124 |
| teR6-H5A(T-1F)-R1 | GAATATAAAATGCATTACCACGCATGTTTTTCTCCTTGTTAAAG | 125 |
| teR6-H5A(T-1G)-R1 | GAATATAACCTGCATTACCACGCATGTTTTTCTCCTTGTTAAAG | 126 |
| teR6-H5A(T-1H)-R1 | GAATATAATGTGCATTACCACGCATGTTTTTCTCCTTGTTAAAG | 127 |
| teR6-H5A(T-1I)-R1 | GAATATAAATTGCATTACCACGCATGTTTTTCTCCTTGTTAAAG | 128 |
| teR6-H5A(T-1K)-R1 | GAATATACTTTGCATTACCACGCATGTTTTTCTCCTTGTTAAAG | 129 |
| teR6-H5A(T-1L)-R1 | GAATATACAGTGCATTACCACGCATGTTTTTCTCCTTGTTAAAG | 130 |

(continued)

| Name | Base Sequence **(5'to 3')** | SEQ ID NO |
|---|---|---|
| teR6-H5A(T-1M)-R1 | GAATATACATTGCATTACCACGCATGTTTTTCTCCTTGTTAAAG | 131 |
| teR6-H5A(T-1N)-R1 | GAATATAATTTGCATTACCACGCATGTTTTTCTCCTTGTTAAAG | 132 |
| teR6-H5A(T-1P)-R1 | GAATATACGGTGCATTACCACGCATGTTTTTCTCCTTGTTAAAG | 133 |
| teR6-H5A(T-1Q)-R1 | GAATATATTGTGCATTACCACGCATGTTTTTCTCCTTGTTAAAG | 134 |
| teR6-H5A(T-1R)-R1 | GAATATAACGTGCATTACCACGCATGTTTTTCTCCTTGTTAAAG | 135 |
| teR6-H5A(T-1S)-R1 | GAATATAAGATGCATTACCACGCATGTTTTTCTCCTTGTTAAAG | 136 |
| teR6-H5A(T-1V)-R1 | GAATATAAACTGCATTACCACGCATGTTTTTCTCCTTGTTAAAG | 137 |
| teR6-H5A(T-1W)-R1 | GAATATACCATGCATTACCACGCATGTTTTTCTCCTTGTTAAAG | 138 |
| teR6-H5A(T-1Y)-R1 | GAATATAATATGCATTACCACGCATGTTTTTCTCCTTGTTAAAG | 139 |

[Table 7-4]

| Name | Base Sequence **(5' to 3')** | SEQ ID NO |
|---|---|---|
| teR6-R2 | CGAAGCTTAACACGGAATATAG | 140 |
| teR6-H5A-R2 | CGAAGCTTAACACGGAATATAGGTTGCATTACC | 141 |
| teR6-H5A(T-1A)-R2 | CGAAGCTTAACACGGAATATATGCTGCATTACC | 142 |
| teR6-H5A(T-1D)-R2 | CGAAGCTTAACACGGAATATAGTCTGCATTACC | 143 |
| teR6-H5A(T-1E)-R2 | CGAAGCTTAACACGGAATATATTCTGCATTACC | 144 |
| teR6-H5A(T-1F)-R2 | CGAAGCTTAACACGGAATATAAAATGCATTACC | 145 |
| teR6-H5A(T-1G)-R2 | CGAAGCTTAACACGGAATATAACCTGCATTACC | 146 |
| teR6-H5A(T-1H)-R2 | CGAAGCTTAACACGGAATATAATGTGCATTACC | 147 |
| teR6-H5A(T-1I)-R2 | CGAAGCTTAACACGGAATATAAATTGCATTACC | 148 |
| teR6-H5A(T-1K)-R2 | CGAAGCTTAACACGGAATATACTTTGCATTACC | 149 |
| teR6-H5A(T-1L)-R2 | CGAAGCTTAACACGGAATATACAGTGCATTACC | 150 |
| teR6-H5A(T-1M)-R2 | CGAAGCTTAACACGGAATATACATTGCATTACC | 151 |
| teR6-H5A(T-1N)-R2 | CGAAGCTTAACACGGAATATAATTTGCATTACC | 152 |
| teR6-H5A(T-1P)-R2 | CGAAGCTTAACACGGAATATACGGTGCATTACC | 153 |
| teR6-H5A(T-1Q)-R2 | CGAAGCTTAACACGGAATATATTGTGCATTACC | 154 |
| teR6-H5A(T-1R)-R2 | CGAAGCTTAACACGGAATATAACGTGCATTACC | 155 |
| teR6-H5A(T-1S)-R2 | CGAAGCTTAACACGGAATATAAGATGCATTACC | 156 |
| teR6-H5A(T-1V)-R2 | CGAAGCTTAACACGGAATATAAACTGCATTACC | 157 |
| teR6-H5A(T-1W)-R2 | CGAAGCTTAACACGGAATATACGATGCATTACC | 158 |
| teR6-H5A(T-1Y)-R2 | CGAAGCTTAACACGGAATATAATATGCATTACC | 159 |

[1. Search for Prenyltransferase]

(Method for Generating Sequence Similarity Network (SSN))

An SSN was generated by an Enzyme Function Initiative-Enzyme Similarity Tool (EFI-ESI) (Zallot, R.; Oberg, N.; Gerlt, The EFI web resource for genomic enzymology tools: leveraging protein, genome, and metagenome databases

to discover novel enzymes and metabolic pathways. Biochemistry, 2019, 58(41), 4169-4182.; Gerlt, J. A.; Bouvier, J. T.; Davidson, D. B.; Imker, H. J.; Sadkhin, B.; Slater, D. R.; Whalen, K. L. Enzyme function initiative-enzyme similarity tool (EFI-EST): a web tool for generating protein sequence similarity networks. BBA. PROTEINS AND PROTEOMICS, 2015, 1854 (8), 1019-1037) using hit sequences obtained by Blastp analysis. Nodes were created by appropriately setting the alignment score, and the resulting network was visualized using Cytoscape 3.8.2 to cluster PTases having different functions. KgpF or LimF was used as the query, as described below.

(Phylogenetic Analysis Method)

A phylogenetic tree was prepared as follows. First, well-known cyanobactin PTase sequences were downloaded from the NCBI database. MEGA 7.0 (Kumar, S.; Stecher, G.; Tamura, MEGA7: molecular evolutionary genetics analysis version 7.0 for bigger datasets. Mol. Biol. Evol., 2016, 33(7), 1870-1874) was used for alignment of the sequences, and then a maximum likelihood phylogenetic tree based on this alignment was generated using 10,000 bootstrap replications.

(Discussion)

In order to search for a potential peptide-PTase having C-prenylation activity, a protein homologous to KgpF, which is the only known cyanobactin C-PTase, was searched using the Basic Local Alignment Search Tool (BLAST). In order to predict the correlation between sequence and function for the obtained hits, a sequence similarity network (SSN) was generated by setting the alignment score to $10^{-86}$. Using the obtained SSN, a cluster having estimated functions including Tyr O-prenylation, terminal N-/O-prenylation, Arg N-prenylation, and Trp N-/C-prenylation, and a cluster having no identified functions were found (FIG. 1a).

Among the unidentified proteins, a protein derived from *Limnothrix* sp. CACIAM 69d that did not belong to any cluster was observed. When this protein was compared to other known cyanobactin PTases and subjected to phylogenetic analysis, it was found that an independent clade was formed between Tyr O-PTase and Trp C-/N-PTase (FIG. 2a). Although this protein was mentioned as a homolog of PirF (Tyr O-PTase) in a previous report (Lima, A.R.J. et al. Insights into Limnothrix sp. Metabolism Based on Comparative Genomics. Front. Microbiol., 9 (2018)), both SSN and phylogenetic analysis suggested that this protein exhibits unique activity. Therefore, this protein was named LimF and further research was conducted.

The limF gene was included in a typical cyanobactin BGC comprising cyanobactin protease (limA) and a macrocyclization enzyme (limG), and peptides estimated to be precursors (limE1 and limE2) (FIG. 2b and Table 8). As a result of manually searching for genes, another precursor candidate (limE3) encoded by a distant locus was also found. The LimE1-3 peptide shared a remarkable similarity with a previously-identified cyanobactin precursor (Donia, M.S., Schmidt, Linking chemistry and genetics in the growing cyanobactin natural products family. Chem. Biol., 18, 508-519 (2011)). LimA and G recognition sequences (RSII and RSIII) were found (FIG. 2c) by the sequence alignment analysis thereof.

[Table 8]

| Protein | Accession | Predicted Function | Closest match | | |
|---|---|---|---|---|---|
| | | | Accession | Identity (%) | Organism |
| LimA | RFP52076.1 | N-Terminal protease | NEQ67225.1 | 75.43 | *Symploca sp. SIO2D2* |
| LimB | RFP52077.1 | Hypothetical protein | MBE5231170.1 | 75.38 | *Microcystis aeruginosa PMC 728.11* |
| LimC | RFP52078.1 | Hypothetical protein | OJJ24754.1 | 60.87 | *Roseofilum reptotaenium AO1-A* |
| LimE1 | RFP52073.1 | Precursor | TRU48195.1 | 62.50 | *Microcystis aeruginosa Ma_QC_Ca_00000000_S207* |
| LimE2 | N/A | Precursor | MBE5231160.1 | 66.67 | *Microcystis aeruginosa PMC 728.11* |
| LimE3 | N/A | Precursor | NCR01836.1 | 60.42 | *Microcystis aeruginosa L211*-11 |
| LimF | RFP52075.1 | Prenyltransferase | NER99685.1 | 68.21 | *Symploca sp. SIO1B1* |

(continued)

| Protein | Accession | Predicted Function | Closest match | | |
|---|---|---|---|---|---|
| | | | Accession | Identity (%) | Organism |
| LimG | RFP52079.1 | C-terminal macrocyclase | NER98176.1 | 71.86 | *Symploca sp. SIO1B1* |

In the general biosynthetic pathway of cyanobactin, A family proteases separate RSII at the N-terminal and then G family macrocyclization enzymes cyclize the core peptide (CP) region while cleaving RSII at the C-terminal to produce a main chain cyclization peptide (Sarkar, S., Gu, W. & Schmidt, Expanding the chemical space of synthetic cyclic peptides using a promiscuous macrocyclase from prenylagaramide biosynthesis. ACS Catal., 10, 7146-7153 (2020)). Generally, the obtained cyclic core peptide is further modified by other modifying enzymes (tailoring enzyme) including PTase to provide a final cyanobactin product. The CP region of the LimE1-3 peptide was identified based on its similarity with other cyanobactin biosynthetic gene clusters and conserved RS motifs. Furthermore, it was hypothesized that a group of peptides in which the main chain was macro-cyclized (bcLimE1, bcLimE2, and bcLimE3. The structures thereof are shown in Table 9 below) could serve as substrates for LimF.

[Table 9]

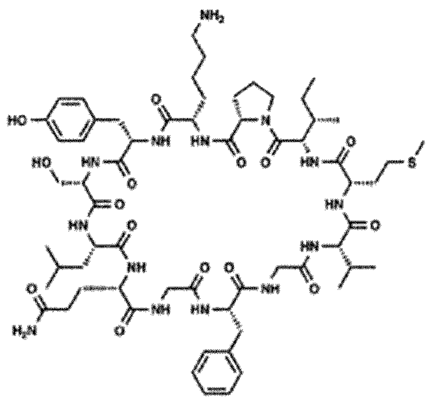

bcLimE1

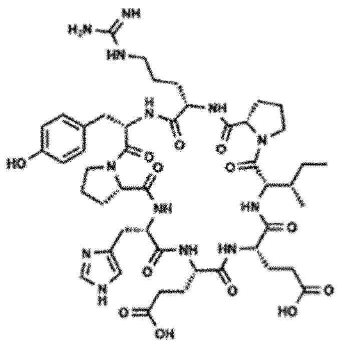

bcLimE2

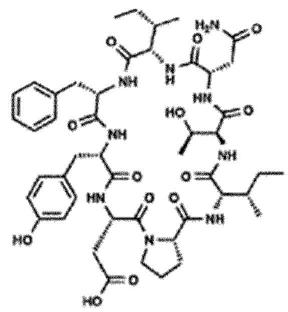

bcLimE3

SSN analysis was also performed on protein sequences that were hits in BLASTp analysis using LimF as a query, with the alignment score set to $10^{-70}$. As a result, 11 new proteins that formed the same cluster as LimF were found (FIG. 1b). The sequences thereof are shown in Table 1 as SEQ ID NOS: 2 to 12.

Based on the above, it was strongly suggested that these 11 types of proteins have the same function as LimF. Among these, the protein of SEQ ID NO: 2 derived from *Symploca sp.* SIO1B1 was estimated to have the highest probability of having the same function as LimF.

[2. Evaluation of Enzymatic Activity of LimF]

In order to examine the enzymatic activity of LimF, LimF was heterologously-expressed in *Escherichia coli* using the method described above and incubated with chemically synthesized bcLimE1, bcLimE2 or bcLimE3 in the presence of MgCh and a prenyl group donor (geranyl pyrophosphate (GPP)). The reaction conditions were as described below. LC-MS analysis of the reaction mixture showed that bcLimE2 was quantitatively converted into a product having a molecular weight increase (+136 Da) corresponding to geranylation (FIG. 3a). MS/MS analysis of the geranylated bcLimE2 suggested that His residue was geranylated by LimF treatment (FIG. 4). On the other hand, bcLimE1 and bcLimE3 were not subjected to geranylation by LimF under these conditions.

LimF-modified bcLimE2 was purified using HPLC and subjected to 1D and 2D NMR analysis including COSY and HMBC. The HMBC correlation observed between the geranyl group and the imidazole C2 carbon indicated a

structure in which the geranylated terminal methylene was bonded to the C2 position of the His side chain (forward geranylation) (FIG. 3b). The structure of limnothamide, which is presumed to be a limBGC product, was identified based on the above. (FIG. 3b). It was also revealed that LimF is a unique PTase that forward geranylates the C2 carbon of His side chains.

In order to examine the biochemical properties of LimF, *in vitro* modification of bcLimE2 facilitated by LimF was performed under various reaction conditions.

First, the same experiment as described above was performed using farnesyl pyrophosphate instead of geranyl pyrophosphate as a prenyl group donor. As a result, a trace amount of farnesylated bcLimE2 was generated, although at a lower efficiency than when GPP was used.

Furthermore, when the reaction was performed under the various conditions described below, LimF showed a clear dependency on $Mg^{2+}$ ions and exhibited relatively wide temperature and pH resistance (FIGS. 5a to c). LimF reaction conditions were optimized based on experiments using various incubation times and GPP/$Mg^{2+}$ concentrations (FIGS. 5d to f).

The optimization conditions were as follows: 1 mM GPP, 40 mM $MgCl_2$, 1 mM dithiothreitol (DTT), pH 7.2, and reaction temperature 25°C. The reaction conditions described above were used below except where otherwise stated.

Under optimal conditions, the steady-state kinetic parameters of the prenylation reaction of bcLimE2 facilitated by LimF were measured by the methods described below. The results were as follows: kcat=13.8±0.6 min $^{-1,}$ Km=0.313±0.039 mM (Table 10, FIG. 5g). This indicates that LimF is one of the most efficient cyanobactin PTases in terms of catalytic turnover.

Interestingly, it was shown that LimF not only prenylates bcLimE2, which is a native substrate, but also His monomers (H-His-OH and Fmoc-His-OH) and His-containing dipeptides (Ac-AH-$NH_2$), respectively, with moderate modification efficiency and high efficiency. The efficiency thereof is shown in the following table.

[Table 10]

| Substrate | Prenyl Group Donor | Modification Efficiency % |
|---|---|---|
| Fmoc-His | GPP | 15.2 ± 0.80/0 |
| Free His | GPP | 34.9 ± 0.7% |
| Ac-AH-$NH_2$ | GPP | >99% |

A reaction was performed for 20 hours with 100 μM LimF.

In order to verify that the geranylated structure of the compounds listed in Table 10 above in which the substrates were geranylated was the same as limnothamide, Ac-AH-$NH_2$ geranylated by LimF was purified and analyzed by NMR. Similarly to bcLimE2, the observed COSY and HMBC correlations supported forward geranylation of the His C2 position. These results suggested that LimF has extremely high substrate sequence tolerance, while also having strict positional selectivity.

Furthermore, when a prenylation reaction facilitated by 20 μM LimF was applied not only to Ac-AH-$NH_2$, but also to various His-containing dipeptides, it became clear that a His prenylation reaction facilitated by LimF progressed for various His-containing dipeptides (FIG. 6).

(Method for Reacting Synthetic Substrate with LimF)

The initial evaluation of LimF using estimated substrates (bcLimE1, bcLimE2, and bcLimE3) was performed as follows. An enzymatic reaction was performed for 12 hours at 37°C under the conditions of LimF (20 μM), peptide substrate (200 μM), prenyl group donor (geranyl pyrophosphate or farnesyl pyrophosphate) (2 mM), HEPES (50 mM, pH 7.5), MgCh (20 mM), and DTT (1 mM). After reacting the substrates bcLimE1, bcLimE2, and bcLimE3 for 12 hours at 37°C, respectively, nine times the amount of 1% TFA was added and centrifuged at 4°C (15300×g, 10 minutes), and a supernatant was recovered. 10 μL of the obtained supernatant was subjected to LC-MS analysis.

(Method of Large-Scale LimF Reaction)

An analyte for NMR analysis was prepared as follows.

Geranylated bcLimE2 was prepared by conducting 100 20 μL scale reactions in parallel at 25° C for 30 hours. Reactions were performed under conditions of LimF (50 μM), bcLimE2 (2 mM), GPP (2.5 mM), HEPES (50 mM, pH 7.5), $MgCl_2$ (100 mM), 1 mM DTT, and DMSO (4%, v/v). After the reactions were completed, the reaction solutions were integrated, 2 mL of methanol (MeOH) was added, and after incubating on ice for 30 minutes, the supernatant was

recovered by centrifugation (15300×g, 10 minutes). Next, the supernatant was diluted with 9 times the amount of water, filtered, and then purified by injecting into preparative reverse phase HPLC. A linear gradient of 1 to 60% buffer solution B over 60 minutes was used for separation. A fraction containing geranylated bcLimE2 (~2.0 mg) was collected, and purity was confirmed by LC-MS. The fraction was then dried and re-suspended in heavy methanol (CDsOD) for NMR analysis.

Enzymatic reactions and purification were performed in the same way as described above when obtaining the geranylated AH dipeptide and ATY tripeptide described above or below.

(Method for Evaluating Biochemical Properties of LimF)

The effects of temperature, pH, and metal ions on a bcLimE2 prenylation reaction facilitated by LimF were examined as follows. The standard reaction conditions were as follows: 1 μM LimF, 1 mM GPP, 40 mM MgClz, 50 mM HEPES pH 7.5, DMSO (4%, v/v), 1 mM DTT, 0.1 mM substrate, reaction temperature 30°C, and reaction time 60 minutes. The reaction temperature, pH, and type and concentration of metal ions were appropriately changed. After reacting, nine times the amount of 1% TFA was added, and the supernatant was then subjected to LC-MS analysis.

In a time-course experiment of LimF, the reaction was incubated at 25°C, and at each point in time, the reaction was stopped by adding 9 times the amount of 1% TFA. Prenylation efficiency was calculated as (peak area of the product / (peak area of the product + peak area of the substrate))×100(%), unless otherwise stated.

The concentration dependence of GPP and $MgCl_2$ in bcLimE2 prenylation reactions facilitated by LimF was measured in reaction systems containing 1 μM LimF, 1 mM or a suitably modified concentration of GPP, 50 mM HEPES pH 7.2, 0.4 mM substrate, 1 mM DTT, and 40 mM or a suitably modified concentration of MgCh. A reaction solution containing components other than GPP was prepared and preheated to 25°C, and the reaction was then started by adding GPP. After incubating at 25°C, the reaction was stopped at each point and analyzed by LC-MS. Next, the initial rate was calculated as the slope of increase in the amount of prenylation product produced in the linear region. Three separate reactions were carried out under each condition and the mean and standard deviation of the results were used for the analysis.

For GPP concentration dependence and peptide substrate concentration dependence, the kinetic parameters were estimated by fitting the initial rate and GPP substrate concentration or peptide substrate concentration to the Michaelis-Menten model in Graphpad Prism 8.4.2 (GraphPad Prism Software Inc., San Diego, California) software.

For MgCh concentration dependence ($K_{Mg}$), the kinetic parameters were estimated by fitting the initial rate and MgCh concentration to the following formula:

[Math. 1]

$$v = V_{max}[Mg^{2+}]/(K_{Mg} + [Mg^{2+}])$$.

Regarding the evaluation of the Tyr prenylation activity of LimF described below, a similar reaction was performed with 5 μM LimF using teR6-H5A as a substrate.

(Method for Measuring Steady State Kinetic Parameters of LimF)

Kinetic parameters of the His-prenylation reaction facilitated by LimF were determined based on reactions under optimal conditions using substrates of different concentrations. For the Tyr-prenylation described below, the kinetic parameters were measured under the same conditions except that LimF was set to 5 μM and the reaction temperature was set to 16°C. In order to estimate the kinetic parameters, the initial rate and substrate concentration were fitted to the Michaelis-Menten model in Graphpad Prism 8.4.2 software.

[3. *In vitro* Biosynthesis of Various Prenylation teMPs by FIT-LimF System]

Thioether ring-closing cyclic peptides (teMP) have been used in the development of new peptide ligands for desired target proteins, and the usefulness thereof has been proven by remarkable results (Huang, Y, Wiedmann, M.M. & Suga, RNA display methods for the discovery of bioactive macrocycles. Chem. Rev., 119, 10360-10391 (2018).; Goto, Y et al. Reprogramming the translation initiation for the synthesis of physiologically stable cyclic peptides. ACS Chem. Biol., 3, 120-129 (2008)).

Therefore, next, an examination was conducted as to whether LimF can modify His residue on a non-natural teMP backbone.

First, teMP (teLimE2) mimicking the sequence of bcLimE2 was designed and expressed by genetic code reprogramming using customized cell-free translation, and the so-called flexible *in vitro* translation (FIT) system (Goto, Y, Katoh, T. & Suga, Flexizymes for genetic code reprogramming. Nat. Protoc., 6, 779-790 (2011)). Specifically, the AUG start codon was reprogrammed with N-chloroacetyl-D-phenylalanine ($^{ClAc}$D-F), a linear precursor having a chloroacetyl group (ClAc group) at the N-terminal was expressed, and a thioether bond was formed by a spontaneous ring-closing $S_N2$ reaction with a downstream Cys thiol (FIG. 7a). Note that the FIT system had the composition described below.

teLimE2 expressed *in vitro* was incubated for 16 hours at 25°C in the presence of 100 μM LimF and analyzed by LC-MS. Chromatograms showed peaks corresponding to geranylation products (teLimE2-$H^{Ger}$), indicating successful prenylation of teLimE2 by LimF (FIG. 7a). The *in vitro* translation system conjugated with LimF will be referred to hereinafter as the FIT-LimF system.

The FIT-LimF system can easily prepare various substrates, and subsequently, prenylation facilitated by LimF can be carried out by the one-pot method. Therefore, with the FIT-LimF system, it is easy to examine the broad substrate tolerance of LimF. Since RiPP enzymes may be affected by the local sequence environment near the modification site, the efficiency of prenylation by LimF was investigated in regard to various substrates wherein residue near the prenylation site was changed. Note that the FIT-LimF system was prepared as described below.

38 types of teLimE2 variants in which the His-1 and His+1 positions were substituted with 19 types of proteinogenic amino acid other than Cys were designed and then expressed and modified in the FIT-LimF system. The reaction results were analyzed by LC-MS. In the present experiment, the prenylation process was performed under intentionally poor modification efficiency conditions by using a low LimF concentration (10 μM). This allowed for more accurate investigation of the influence of His-1 and His+1 amino acids.

The His-1 variant series suggested that LimF tends to have weak selectivity for this position. Specifically, substrates having relatively small or hydrophobic residues such as Thr/Ala/Met/Pro/Ser/Val at the -1 position underwent prenylation by LimF with extremely high efficiency, but substrates having bulky or charged residues at the -1 position underwent prenylation by LimF with relatively low efficiency (FIG. 7b). On the other hand, only Pro had low prenylation efficiency at the +1 position, and the amino acid at the +1 position was extremely variable (FIG. 7c). The same trend was confirmed in cyclic peptides having different sequences (FIG. 7f). As a result, findings on differences in the efficiency of modification by LimF in substrates having different His forward and reverse sequences were obtained.

The following series of experiments examined whether teMPs of various ring sizes were tolerated by LimF. Thirteen teMP variants with different ring sizes (4 to 18 residues) and a preferred local sequence motif (Ser-His-Ala/Cys) were designed. One-pot expression and modification of these teMPs by the FIT-LimF system (LimF concentration: 10 μM) showed that all of these underwent quantitative prenylation (FIG. 7d). The above results demonstrate that LimF can prenylate teMPs regardless of ring size.

Next, it was shown that LimF can modify teMPs having a variety of random sequences. Five teMPs composed of random sequences were expressed and modified using the FIT-LimF system (LimF concentration: 10 μM) (FIG. 7e). Of the five teMPs tested, two teMPs (teR1 and teR2) having -1 and +1 residues, which have an advantage in LimF prenylation, underwent quantitative prenylation. As expected, the efficiency of the LimF modification was suppressed in substrates in which the adjacent residues of these teMPs (-1 or +1 residues) were mutated to unfavorable amino acids (teR1(P-1K) and teR2(W+1P)). On the other hand, while the prenylation efficiency of teR3, teR4, and teR5 peptides was moderate or low, modification efficiency by LimF was greatly improved by substituting the residue adjacent to His with a preferred side chain (teR3(E-1S), teR4(W-1A), and teR5(P+1A)).

The above results indicate that LimF can prenylate a variety of teMPs, regardless of the length and overall sequence construction thereof, mainly depending on the local sequence environment near the His residue. A similar trend was confirmed for the prenylation of cyclic peptides with different sequences using the FIT-LimF system (LimF concentration: 20 μM) (FIG. 8a).

Furthermore, it was demonstrated that a plurality of prenyl groups were introduced when the substrate included a moiety accepting a plurality of prenylations. Five teMPs composed of random sequences were expressed and modified using the FIT-LimF system (LimF concentration: 20 μM) (FIG. 8b). Major monoprenylated products and secondary diprenylated products were obtained in cyclic peptides having two His residues. Additionally, major diprenylated products and secondary monoprenylated products were obtained in cyclic peptides having three His residues. The above results demonstrate that the FIT-LimF system can produce cyclic peptides having multiple prenyl groups.

(FIT-LimF System)

The transcription-coupled *in vitro* translation system (transcription-coupled *in vitro* translation system) contained the following components: each proteinogenic amino acid (excluding methionine) of 500 μM, 50 mM HEPES-KOH (pH 7.6), 12 mM Mg $(OAc)_2$, 100 mM potassium acetate, 2 mM spermidine, 1 mM DTT, 20 mM phosphocreatine (Roche), 2 mM ATP, 2 mM GTP, 1 mM CTP, 1 mM UTP, 0.1 mM 10-formyl-5,6,7,8-tetrahydrofolic acid, 1.5 mg/mL *E. coli* total tRNA (Roche), 0.73 μM AlaRS, 0.03 μM ArgRS, 0.38 μM AsnRS, 0.13 μM AspRS, 0.02 μM CysRS, 0.06 μM GlnRS,

0.23 μM GluRS, 0.09 μM GlyRS, 0.02 μM HisRS, 0.4 μM IleRS, 0.04 μM LeuRS, 0.11 μM LysRS, 0.03 μM MetRS, 0.68 μM PheRS, 0.16 μM ProRS, 0.04 μM SerRS, 0.09 μM ThrRS, 0.03 μM TrpRS, 0.02 μM TyrRS, 0.02 μM ValRS, 0.6 μM MTF, 2.7 μM IF1, 0.4 μM IF2, 1.5 μM IF3, 0.26 μM EF-G, 10 μM EF-Tu, 0.66 μM EF-Ts, 0.25 μM RF2, 0.17 μM RF3, 0.5 μM RRF, 0.1 μM T7 RNA polymerase, 0.1 μM nucleoside diphosphate kinase, 4 μg/mL creatine kinase, 3 μg/mL myokinase, 0.1 μM pyrophosphatase, and 1.2 μM ribosomes.

$tRNA^{fMet}_{CAU}$ (ClAc-D-Phe-$tRNA^{fMet}_{CAU}$ or ClAc-D-Tyr-$tRNA^{fMet}_{CAU}$) charged with a DNA template and chloroacetylamino acid was added to the above compositions at final concentrations of 0.04 μM and 50 μM, respectively. Next, the mixture was incubated for 1 hour at 37°C, and translation and spontaneous thioether ring formation were performed.

Similar *in vitro* translation reactions were performed without His for the substrates incorporating non-proteinogenic amino acid derivatives of histidine described below, and $tRNA^{GluE2}_{GUG}$ (50 μM) charged with His analogues was added.

Reactions facilitated by LimF were performed as follows in the FIT-LimF system of the present example, except where otherwise stated.

After reacting for 1 hour at 37°C, the translation mixtures were mixed with the LimF enzyme mixtures, respectively, and a reaction mixture containing 10 μM LimF and 1 mM GPP was prepared. After incubating for 16 hours at 25°C, the reaction was stopped by adding 45 μL of a 1% TFA aqueous solution, and the mixture was then incubated on ice for 10 minutes. The precipitated fraction was separated by centrifugation at 4°C (15200×g, 10 minutes), and 10 μL of supernatant was subjected to LC-MS analysis.

[4. Prenylation of Peptide Containing His Derivative by FIT-LimF System]

Next, an examination was conducted as to whether prenylation by LimF proceeded when prenylated His residue was replaced by a similar side chain (His derivative). Using genetic code reprogramming by the FIT system, a teLimE2 derivative in which the His residue was substituted with various non-proteinogenic amino acids was expressed (FIG. 8c). Specifically, cyclic peptides were synthesized in which L-His, which is a proteinogenic amino acid, was substituted with Nτ- and Nπ-methylated His analogs ($_L$His(1-Me) and $_L$His(3-Me)); an amino acid having a thiophene or thiazole side chain ($_L$Ala(2-Thi), $_L$Ala(3-Thi), and $_L$Ala(4-Thz)); D-His; or α-methyl-His. Each synthesized teLimE2 derivative was modified using the FIT-LimF system (LimF concentration: 100 μM).

teLimE2 expressed by genetic code reprogramming using L-His was used as a control. The teLimE2 was prenylated by LimF with the same efficiency as the teLimE2 translated by the original genetic code, thereby confirming that the reprogrammed FIT-LimF system functions well.

As illustrated in FIG. 8c, teLimE2 derivatives containing D-His and α-methyl-His ($_D$His and $\alpha(Me)_{D/L}$His) were favorable substrates for LimF. However, Nτ- and Nπ-methylated His analogs ($_L$His(1-Me) and $_L$His(3-Me)) incorporated into the LimE2 sequence were not prenylated at all by LimF. Furthermore, artificial residues having a thiophene or thiazole side chain ($_L$Ala(2-Thi), $_L$Ala(3-Thi), and $_L$Ala(4-Thz)) were not prenylated by LimF, either. This suggests that NH in imidazole may be involved in LimF recognition.

These results suggest that the imidazole moiety of His is involved in recognition by LimF, and that a main chain structure including α-substitution and its molecular configuration is not an important determining factor. As a result, the usefulness of the FIT-LimF system, which is capable of producing teMPs having various non-natural prenylation residues, was demonstrated.

[5. Tyrosine O-Prenylation by LimF]

In the process of evaluating teMPs with various random sequences, a teMP (teR6) that provides two prenylation products after LimF treatment was discovered by chance (FIG. 9a). A prenylated product was also provided in a variant (teR6-H5A) in which the His of the peptide was substituted with Ala. This shows that LimF adds prenyl groups to residues other than His. In the mass spectrometry results for peptides that underwent prenylation at a site other than His, a peak corresponding to a fragment in which the prenyl moiety ($C_{10}H_{17}$) was dissociated was observed (FIG. 9b). This suggests that prenyl groups are present on heteroatoms (McIntosh, J.A., Donia, M.S., Nair, S.K. & Schmidt, Enzymatic basis of ribosomal peptide prenylation in cyanobacteria. J. Am. Chem. Soc., 133, 13698-13705 (2011).; Donia, M.S., Schmidt, Linking chemistry and genetics in the growing cyanobactin natural products family. Chem. Biol., 18, 508-519 (2011)).

MS/MS analysis of the prenylated peptides identified that Tyr at the seventh position was prenylated by LimF (FIG. 9a). For simple NMR analysis, model tripeptide Ac-ATY-$NH_2$ was synthesized and modified with LimF to obtain the corresponding Tyr-prenylation products. The correlation between the product COSY and HMBC determined that the prenylation site was a phenolic OH group (FIG. 9c). The above results indicate that LimF can catalyze two different prenylation modes: His C-prenylation and Tyr O-prenylation.

In order to study the local sequence selectivity of Tyr-prenylation by LimF, a series of Tyr-1 variants of teR6-H5Awere tested in the presence of 10 μM LimF under reaction conditions of 16 hours at 25°C. The results showed a preference for the -1 position similar to His-prenylation (FIG. 9d).

In order to further evaluate the two different prenylation modes by LimF, two teMPs (teR6-H5A/Y7H and teR6-H5A) that undergo efficient His-prenylation and Tyr-prenylation, respectively, were designed and chemically synthesized. Kinetic analysis was carried out according to the above method using these peptides. The Km and kcat values for His-prenylation of the obtained teR6-H5A/Y7H were equivalent to those of bcLimE2, suggesting that the sequence of the closed ring backbone had no significant effect on catalytic efficiency (Table 11, Items 1 and 2).

Furthermore, by changing the GPP or $Mg^{2+}$ concentration and performing LimF modification of teR6-H5A using the method above, it was revealed that the GPP and $Mg^{2+}$ concentrations required to demonstrate half of the maximum activity are similar for Tyr-prenylation and His-prenylation (FIG. 10a to e). This suggests that the two prenylation modes may be catalyzed at similar binding sites. However, the kcat and Km values for Tyr-prenylation were lower than for His-prenylation (Table 11, Items 2 and 3, FIG. 10f), suggesting that His-prenylation is the main function of LimF. However, the catalytic turnover rate for Tyr-prenylation by LimF (kcat=1.0 $min^{-1}$) was equal to or greater than some cyanobactin PTases reported thus far, indicating that Tyr-prenylation is a secondary but significant catalytic activity of LimF (Table 12).

[Table 11]

Table: Steady-State Kinetic Parameters for LimF (Wild-Type) and LimF-H172L Mutant in Different Prenylation Modes

| Item | Enzyme | Substrate | Prenylated Residue | $K_m$ (mM) | $k_{cat}$ ($min^{-1}$) | $k_{cat}/k_{min}$ ($min^{-1}\cdot mM^{-1}$) |
|---|---|---|---|---|---|---|
| **1** | **LimF** | **bcLimE2** | His | 0.31 ± 0.04 | 13.8 ± 0.6 | 44.1 |
| **2** | **LimF** | **teR6-H5A/Y7H** | His | 0.58 ± 0.07 | 11.3 ± 0.6 | 19.4 |
| **3** | **LimF** | **teR6-H5A** | Tyr | 7.3 ± 1.8 | 1.0 ± 0.1 | 0.14 |
| **4** | **LimF-H172L** | **bcLimE2** | His | 1.7 ± 0.4 | 0.05 ± 0.01 | 0.03 |
| **5** | **LimF-H172L** | **teR6-H5A** | Tyr | 1.7 ± 0.4 | 1.2 ± 0.1 | 0.68 |

[Table 12]

| Function | Enzyme Name | Substrate | $K_m$ (mM) | $k_{cat}$ ($min^{-1}$) |
|---|---|---|---|---|
| Tyr C5 *O*-prenylation | PagF | *cyclo*[INPYLYP] | $2.2x10^{-4}$ | 7.4 |
| Tyr C5 *O*-prenylation | PagF | YYY | 0.16 | 5.8 |
| Tyr C5 *O*-prenylation | PagF | N-boc-L-Tyr | 6.2 | 11 |
| Trp C5 *N*-prenylation | AcyF | *cyclo*[TSQIWGSPVP] | 3.32 | 9.50 |
| Tyr C5 *O*-prenylation | LynF | N-boc-L-Tyr | 14.0 | 1.06 |
| Tyr C5 *O*- prenylation | LynF | *cyclo*[APMPPYP] | 4.2 | 0.22 |
| Tyr C10 *O*-prenylation | PirF | *cyclo*[MSGVDYYNP] | 0.116 | 0.70 |
| Tyr C10 *O*-prenylation | PirF | MSGVDYYNPFAGDDAE | 0.118 | 1.10 |
| His C10 *C*-prenylation | **LimF** | *cyclo*[RYPHEEIP] | 0.313 | 13.80 |
| Tyr C10 *O*-prenylation | **LimF** | tecyclol[$_D$FRGNATYIPC] | 7.30 | 1.00 |
| Tyr C10 *O*-prenylation | **LimF-H172L** | tecyclo[$_D$FRGNATYIPC] | 1.74 | 1.19 |
| Aromatic C5 *N*-prenylation | FtmPT2[8] | *12,13-dihydroxyfumitremorgin C* | 0.096 | 1.80 |
| Aromatic C5 *O*-prenylation | Vib-PT[9] | *4-hydroxy-benzenemethanol* | 2.051 | 0.047 |
| Aromatic C10 *O*-prenylation | Fnq26[10] | *4-hydroxy-benzoate* | 5.60 | 0.14 |
| Aromatic C10 O-prenylation | Fnq26[10] | *flaviolin* | 0.021 | 0.013 |

In Table 12 above, 8 indicates an item cited from Grundmann, A.; Kuznetsova, T.; Afiyatullov, S. S.; Li, S.-M., FtmPT2, an N-Prenyltransferase from Aspergillus fumigatus, Catalyses the Last Step in the Biosynthesis of Fumitremorgin B. Chembiochem, 2008, 9 (13), 2059-2063., 9 indicates an item cited from Bai, N.; Li, G.-H.; Luo, S.-L.; Du, L.; Hu, Q.-Y; Xu, H.-K.; Zhang, K.-Q.; Zhao, P.-J., Vib-PT, an aromatic prenyltransferase involved in the biosynthesis of vibralactone from Stereum vibrans. Appl. Environ. Microbiol., 2020, 86(10), e02687-19., and 10 indicates an item cited from Haagen, Y; Unsold, I.; Westrich, L.; Gust, B.; Richard, S. B.; Noel, J. P.; Heide, L., A soluble, magnesium-independent prenyltransferase catalyzes reverse and regular C-prenylations and O-prenylations of aromatic substrates. FEBS Lett., 2007, 581 (16), 2889-2893.

Furthermore, it was confirmed that LimF can O-prenylate Tyr contained in the teMPs of various random sequences. A peptide having a sequence different from that of teR6-H5A/Y7H was expressed in the FIT-LimF system and reacted with 20 μM LimF for 16 hours, and Tyr-prenylation was confirmed. Furthermore, when the amino acid residue at the -1 position of Tyr in the peptide was substituted with Thr, which is favorable for prenylation by LimF, Tyr-prenylation was confirmed to be more efficient.

Table 13 shows the sequences of prenylated teMPs and the prenylation efficiency thereof. In the table, "y" means D-Tyr, and "tecyclo" means having a cyclic structure facilitated by a thioether bond formed between the chloroacetyl group of the amino acid (D-Tyr) at the N-terminal and the thiol group of the downstream Cys. Furthermore, the substrates shown in Table 13 underwent Tyr prenylation as shown by the underline.

[Table 13]

| Substrate | Modification Efficiency % |
|---|---|
| **tecyclo** [ y I T N I T <u>Y</u> D P C] | 2 1 |
| **tecyclo** [ y I T N I A <u>Y</u> D P C] | 1 |
| A reaction was performed for 16 hours with 20 μM LimF. | |

[6. Examination of LimF Variants]

Experiments for introducing site-specific mutation were performed to verify the importance of residues (Glu54, Asp70, and His 172) that were estimated to be located near the substrate imidazole ring from the results of the crystal structure and sequence alignment analysis of PagF, which is one type of cyanobactin PTase. Sequence alignment analyses of cyanobactin PTases showed that Glu54 is highly conserved among F family PTases. On the other hand, it became clear that while Asp70 and His 172 are specific to LimF, other previously reported PTases that modify aromatic side chains have aliphatic residues (Leu, Met, Val) conserved in the His172 position.

Therefore, two Glu54 variants (LimF-E54A and LimF-E54Q), one Asp70 variant (LimF-D70A), and three His172 variants (LimF-H172A, LimF-H172F, and LimF-H172L) were prepared using the method described below, and reacted with the above bcLimE2 to confirm the His-prenylation ability thereof. Specifically, 100 μM bcLimE2 was reacted for 2 hours with 1 μM wt-LimF or each LimF variant under previously optimized standard conditions.

As a result, LimF variants other than LimF-D70A did not produce any or produced almost no prenylated bcLimE2 products (FIG. 11). Thus, it was revealed that Glu54 and His172 play an essential role in His-prenylation by LimF. On the other hand, LimF-D70A exhibited the same His-prenylation ability as the wild type, and it was revealed that this residue did not play an important role in His-prenylation by LimF.

Similarly, modification of the teR6 and teR6-H5A substrates facilitated by wt-LimF and each LimF variant was performed to evaluate the Tyr-prenylation ability thereof. The reaction was carried out in the same manner as the prenylation of bcLimE2, except that teR6 or teR6-H5A was used as the substrate and the reaction was carried out with 5 μM LimF for 24 hours.

The Glu54 variant resulted in the loss of Tyr-prenylation. This result is reasonable considering that Glu54 is strongly conserved in F family PTases. In contrast, LimF-H172L, in which the unique His in LimF is replaced with Leu conserved in Tyr-O-PTases, exhibited more favorable modification efficiency for Tyr-containing substrates than wild-type LimF. A further steady-state kinetic assay of LimF-H172L confirmed a 5-fold increase in kcat/Km over the wild type and confirmed that the Tyr-prenylation activity thereof was enhanced (Table 11, Items 3 and 5).

In contrast to His-prenylation, in which both Glu54 and His172 are essential, His172 does not play an important role in Tyr-prenylation, and rather, mutations to Leu enhance Tyr-prenylation activity.

(Method for Producing Constructed Plasmid for Expressing LimF Variant)

The pET32-LimF-SMS plasmid was used as a template for introducing site-specific mutation based on PCR. The PCR were performed using the In-Fusion (Registered Trademark) HD Cloning Kit (TaKaRa Cloning) based on the

manufacturer's procedures. The sequences of the plasmids were confirmed by Sanger sequencing. Purification of the LimF variant was carried out using a similar protocol as the purification of wild type (wt) LimF. The primers used are shown in Table 14 below.

[Table 14]

| Primer | Sequence **(5' to 3')** | SEQ ID NO |
|---|---|---|
| Lim-E54A-Forward | CATTATTGCGTGTTCAGGCAAGATCAAACAAGACC | 160 |
| Lim-E54A-Reverse | GAACACGCAATAATGCAATCTCCGGTAGTTGCG | 161 |
| Lim-E54Q-Forward | CATTATTCAGTGTTCAGGCAAGATCAAACAAGACC | 162 |
| Lim-E54Q-Reverse | GAACACTGAATAATGCAATCTCCGGTAGTTGCG | 163 |
| Lim-D70A-Forward | CGCATCCTTCTGCAGTTTAGCGATAA | 164 |
| Lim-D70A-Reverse | CTGCAGAAGGATGCGCGCAGGATACAG | 165 |
| Lim-H172L-Forward | TACCTTACTGATCGGCTTTGACTTCTACCTCG | 166 |
| Lim-Hl72L-Reverse | CCGATCAGTAAGGTATCAGAGCGCAGCATGG | 167 |
| Lim-H172F-Forward | TACCTTATTTATCGGCTTTGACTTCTACCTCG | 168 |
| Lim-H172F-Reverse | CCGATAAATAAGGTATCAGAGCGCAGCATGG | 169 |
| Lim-H172A-Forward | TACCTTAGCGATCGGCTTTGACTTCTACCTCG | 170 |
| Lim-H172A-Reverse | CCGATCGCTAAGGTATGAGAGCGCAGCATGG | 171 |

[7. Prenylation of Various Compounds by LimF]

Prenylation by LimF was performed using various low molecular weight compounds having imidazole rings, peptides, and proteins as substrates.

First, it was demonstrated that LimF prenylates not only cyclic peptides, but also linear peptides made up of several to several tens of amino acids. One cyclic peptide and three linear peptides of different lengths, in which the core moiety of the cyclic peptide was conserved, were prepared. Each of the prepared peptides underwent prenylation by LimF (20 μM) with nearly the same efficiency (FIG. 12). These results suggest that prenylation by LimF is not limited to cyclic peptides, but can be applied to a wide range of peptides or proteins.

Next, various low molecular weight compounds, peptides, and proteins, including various compounds that have already been approved as pharmaceuticals, were subjected to His-prenylation facilitated by LimF. Specifically, a 500 μM substrate (for Aviptadil, Pramlintide, GLP-1, Teduglutide, and Elcatonin, a 125 μM substrate was used as an exception) was reacted for 20 hours under previously optimized standard conditions in the presence of 100 μM LimF and 2 mM GPP.

The substrate used, the characteristics of the substrate, the target molecule and use thereof, and His-prenylation efficiency are illustrated in Table 15 below (shown as Tables 15-1 and 15-2).

[Table 15-1]

| Name | Sequence/Structure |
|---|---|
| Cimetidine | 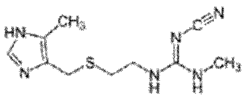 |
| TRH | 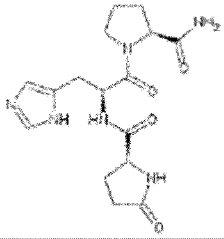 |
| Elcatonin | Cyclo(-Ser-Asn-Leu-Ser-Thr-Asu)-Val-Leu-Gly-Lys-Leu-Ser-Gln-Glu-Leu-His-Lys-Leu-Gln-Thr-Tyr-Pro-Arg-Thr-Asp-Val-Gly-Ala-Gly-Thr-Pro-NH2 |
| Bremelanotide | Ac-Nle-Asp(1)-His-D-Phe-Arg-Trp-Lys(1)-OH |
| ANGIOTENSIN II | H-Asp-Arg-Val-Tyr-Ile-His-Pro-Phe-OH |
| Leoprorelin | H-Pyr-His-Trp-Ser-Tyr-D-Leu-Leu-Arg-Pro-NHEt |
| Histrelin | H-Pyr-His-Trp-Ser-Tyr-D-His(1-Bn)-Leu-Arg-Pro-NHEt |
| GnRH | H-Pyr-His-Trp-Ser-Tyr-Gly-Leu-Arg-Pro-Gly-NH2 |
| α-mating factor | Trp-His-Trp-Leu-Gln-Leu-Lys-Pro-Gly-Gln-Pro-Met-Tyr-OH |
| Lar-D5 | tecyclo[ySLFDASGHYIANYSC] (y: D-Tyr) |
| PBm1 | tecyclo[wAVSNLPPRPHITYIEC]S (w: D-Trp) |

| Name | Sequence |
|---|---|
| Aviptadil | H-His-Ser-Asp-Ala-Val-Phe-Thr-Asp-Asn-Tyr-<br>Thr-Arg-Leu-Arg-Lys-Gln-Met-Ala-Val-Lys-<br>Lys-Tyr-Leu-Asn-Ser-Ile-Leu-Asn-OH |
| Pramlintide | H-Lys-Cys-Asn-Thr-Ala-Thr-Cys-Ala-<br>-Thr-Gln-Arg-Leu-Ala-Asn-Phe-Leu-<br>-Val-His-Ser-Ser-Asn-Asn-Phe-Gly-<br>-Pro-Ile-Leu-Pro-Pro-Thr-Asn-Val-<br>-Gly-Ser-Asn-Thr-Tyr-$NH_2$ |
| GLP-1 | H-His-Ala-Glu-Gly-Thr-Phe-Thr-Ser-Asp-Val-<br>Ser-Ser-Tyr-Leu-Glu-Gly-Gln-Ala-Ala-Lys-<br>Glu-Phe-Ile-Ala-Trp-Leu-Val-Lys-Gly-Arg-$NH_2$ |
| Teduglutide | H-His-Gly-Asp-Gly-Ser-Phe-Ser-Asp-Glu-Met-<br>Asn-Thr-Ile-Leu-Asp-Asn-Leu-Ala-Ala-Arg-<br>Asp-Phe-Ile-Asn-Trp-Leu-Ile-Gln-Thr-Lys-<br>Ile-Thr-Asp-OH |
| Insulin | H-Phe-Val-Asn-Gln-His-Leu-Cys-Gly-Ser-His-<br>Leu-Val-Glu-Ala-Leu-Tyr-Leu-Val-Cys-Gly-<br>Glu-Arg-Gly-Phe-Phe-Tyr-Thr-Pro-Lys-Ala-OH<br>H-Gly-Ile-Val-Glu-Gln-Cys-Cys-Thr-Ser-Ile-<br>Cys-Ser-Leu-Tyr-Gln-Leu-Glu-Asn-Tyr-Cys-<br>Asn-OH |

[Table 15-2]

| Name | % prenylation (LC/MS) | Chemical basis | Route of administration | Moleculuar targets | Approved indications |
|---|---|---|---|---|---|
| Cimetidine | 97% | small molecular drug | oral; intramuscular | histamine H2 receptor antagonist | heartburn and peptic ulcers |
| TRH | 15% | Thyrotropin-releasing hormone (TRH) | N/A | TRH receptor | N/A |
| ANGIOTENSIN II | 1% | Natrual ANGIOTENSIN II | Intravenous | renin-angiotensin system | Hypotension |
| Bremelanotide | 1% | α-MSH analog | Subcutaneous | melanocortin receptors (belong to GPCR familiy) | women hypoactive sexual desire |
| Elcatonin | 5% | Calcitonin analog from eel | Intranasal; subcutaneous | Calcitonin receptor | Osteoporosis |
| Leoprorelin | 99% | GnRH analogue | Subcutaneous; intramuscular | GnRH receptor | Prostate cancer; endometriosis; uterine fibroids; preccoious puberty |
| Histrelin | 99% | GnRH analogue | Subcutaneous; intramuscular | GnRH receptor | Prostate cancer; precocious puberty |
| Aviptadil | 90% | vasoactive intestinal polypeptide analogue | Intracavernous | VIP1 receptor | Erectile dysfunction **(also reported for treating COVID-19 recently)** |
| Pramlintide | 99% | amylin analogue | Subcutaneous | Calcitonin receptor | Type 1 and type 2 diabetes |
| GLP-1 | 75% | Natural GLP-1 | N/A | GLP-1 receptor | N/A |
| Teduglutide | 50% | GLP-2 analgoue | Subcutaneous | GLP-2 receptor | Short bowel syndrome |
| Insulin | 5% | Human Insulin | Subcutaneous; oral | insulin receptor | diabetes |
| GnRH | 99% | Human GnRH | N/A | GnRH receptor | N/A |
| α-mating factor | 39% | cell signaling peptide for yeast | N/A | N/A | N/A |
| Lar-D5 | 89% | RaPID selection hit | N/A | receptor protein tyrosine phosphatases | N/A |
| PBm1 | 99% | RaPID selection hit | N/A | Semaphorin 4D Receptor Plexin B1 | N/A |

From Table 15 above, it is understood that all imidazole-containing compounds and His-containing compounds underwent prenylation by LimF. Interestingly, the N-terminal His of Aviptadil, which is a synthetic vasoactive intestinal polypeptide recently approved to treat COVID-19 causing respiratory failure, was successfully prenylated by LimF with a conversion efficiency of about 90%. In addition, His residue or imidazole C2 carbon was prenylated with very high efficiency in cimetidine, which is a histamine $H_2$ receptor antagonist; Leoprorelin, which is an anticancer agent; Histrelin, which recognizes a gonadotropin-releasing hormone receptor; Pramlintide, which is an analog of amylin; Teduglutide, which is an analog of GLP-1 and GLP-2; gonadotropin-releasing hormone (GnRH); and thioether cyclic peptide PBm1, which was discovered through the RaPID (Random Peptide Integrated Discovery) system and inhibits the interaction of plexin B1 and semaphorin 4D; and Lar-D5, which binds to receptor protein tyrosine phosphatase. In particular, the fact that cimetidine was prenylated by LimF suggests that LimF can tolerate imidazole rings having substituents at the 4- and 5-positions to perform prenylation.

The above results indicate that LimF and enzymes homologous thereto are highly promising as biocatalysts for chemically selective, position-specific prenylation of His in a variety of compounds including low molecular weight compounds, peptides, and proteins.

Moreover, His-prenylation by LimF was also performed for two types of high molecular weight proteins (acetylated bovine-derived albumin (Ac-BSA (Wako Pure Chemical Industries)) and horse-derived glutathione-S-transferase (GST (Sigma-Aldrich Japan))). Specifically, 100 μM Ac-BSA or 100 μM GST was reacted for 20 hours in the presence of 100 μM LimF and 2 mM GPP under previously optimized standard conditions. After desalting the reaction solution after the reaction by solid phase extraction (SPE C-TIP (Nikkyo Technos)), using sinapinic acid as a matrix, analysis was performed using a matrix-assisted laser desorption ionization-time-of-flight mass spectrometer (MALDI-TOF-MS, ultrafleXtreme manufactured by Bruker Daltonics). The obtained mass spectrum is shown in FIG. 13.

From FIG. 13, it is understood that Ac-BSA underwent prenylation by LimF in two locations. GST also underwent

one prenylation by LimF. The above results indicate that LimF and enzymes homologous thereto are very promising as biocatalysts for performing prenylation modification of high molecular weight proteins.

Furthermore, various peptides known as bioactive peptide hormones and artificial cyclic peptide drugs discovered through the RaPID system were subjected to Tyr-prenylation by the LimF-H172L variant. Specifically, a 500 μM substrate was reacted for 20 hours in the presence of 40 μM LimF-H172L and 2 mM GPP under previously optimized standard conditions.

The substrate used, the characteristics of the substrate, the target molecule and use thereof, and Tyr-prenylation efficiency are illustrated in Table 16 below (shown as Tables 16-1 and 16-2).

[Table 16-1]

| Name | Sequence | Name | Sequence |
|---|---|---|---|
| **PBm1** | tecyclo[wAVSNLPPRPHITYIEC]S w: D-Trp | **Lou.Enkephalin** | Tyr-Gly-Gly-Phe-Leu-OH |
| **aMD5** | tecyclo[yWYYAWOOTYKAFPC]G y: D-Tyr | **ANGIOTENSIN II** | H-Asp-Arg-Val-Tyr-Ile-His-Pro-Phe-OH |
| **Guanylin** | Pro-Gly-Thr-Cys-Glu-Ile-Cys-Ala-Tyr-Ala-Ala-Cys-Thr-Gly-Cys (Disulfide bond Cys4-Cys12 and Cys7-Cys15) | **Gastrin-I** | Pyr-Gly-Pro-Trp-Leu-Glu-Glu-Glu-Glu-Glu-Ala-Tyr-Gly-Trp-Met-Asp-Phe-NH2 |
| **α-mating factor** | Trp-His-Trp-Leu-Gln-Leu-Lys-Pro-Gly-Gln-Pro-Met-Tyr-OH | | |

[Table 16-2]

| Name | % prenylation (LC/MS) | Chemical basis | Route of administration | Molecular targets | Approved indications |
|---|---|---|---|---|---|
| PBm1 | 5% | Thioether cyclic peptide which was discovered through the RaPID system and inhibits interaction of plexin B1 and semaphorin 4D | N/A | Semaphorin 4D Receptor Plexin B1 | N/A |
| aMD5 | 1% | Human stem cell growth factor receptor binding thioether cyclic peptide which was discovered through the RaPID system | N/A | Hepatocyte growth factor (HGF) receptor | N/A |
| Guanylin | 1% | Guanylyl cyclase receptor GC-C agonist | N/A | guanylyl cyclase receptor GC-C | N/A |
| α-mating factor | 4% | cell signaling peptide for yeast | N/A | N/A | N/A |
| Leu-Enkephalin | 19% | Endogenous opioid peptide neurotransmitter | N/A | guanylyl cyclase receptor GC-C | N/A |
| ANGIOTENSIN II | 15% | Natrual ANGIOTENSIN II | Intravenous | renin-angiotensin system | Hypotension |
| Gastrin-I | 25% | peptide hormone | N/A | cholecystokinin B receptors | N/A |

From Table 16 above, it is understood that all Tyr-containing compounds underwent Tyr-prenylation by the LimF-H172L variant. In particular, Tyr residues were subjected to prenylation with high efficiency in Leu-Enkephalin, angiotensin II, and gastrin I, which are endogenous opioid peptide neurotransmitters.

The above results indicate that LimF and enzymes homologous thereto are also highly promising as biocatalysts for chemically selective, position-specific prenylation of Tyr in a variety of compounds.

[8. Introduction of Prenyl Group in GLP-1]

(8.1. Introduction of Prenyl Group)

Next, large-scale prenylation was performed on GLP-1 and derivatives thereof, which are known as antidiabetic drug motifs. Although it is difficult to use GLP-1 per se as an antidiabetic drug due to its low stability in the blood, it is known that the stability in the blood can be improved by modification with a fatty acid functional group. A fatty acid-modified GLP-1 derivative that is already in use as an antidiabetic drug is known. GLP-1 is a compound that activates intracellular cAMP production through binding to receptors.

In order to obtain an adequate amount of prenylated GLP-1 derivatives for performing various downstream assays, a prenylation reaction was performed at a 3 mL scale (72 hours at 25°C). The following two compounds were used as substrates. In the following table, "GLP-1" is a moiety of GLP-1 excluding the moiety from the first His to the sixth Arg, and "GLP-1 H7F/K34H" is a moiety wherein the seventh His is replaced by Phe and the 34th Lys is replaced by His.

[Table 17]

| | |
|---|---|
| GLP-1 | H A E G T F T S D V S S Y L E G O A A K E F I A W L V K G R (SEQ ID NO: 197) |
| GLP-1 _H7F/K34H | F A E G T F T S D V S S Y L E G Q A A K E F I A W L V H G R (SEQ ID NO: 204) |

The reaction solution contained LimF (50 μM), GLP-1 or GLP-1_H7F/K34H (1 mM), GPP (2.5 mM), HEPES (50 mM, pH 7.2), MgCh (50 mM), 1 mM DTT, and DMSO (4%, v/v). After the reaction was completed, the reaction mixture was combined and mixed with 2 mL of methanol (MeOH). After incubating for 30 minutes on ice, the supernatant was separated by centrifugation (15300×g, 10 minutes), and diluted with 9 times the amount of 0.1% TFA-aqueous solution. The obtained solution was filtered and subjected to preparative reverse phase HPLC. A linear gradient of 1 to 60% buffer solution B over 60 minutes was used for separation. Fractions containing prenylated GLP-1 were combined and lyophilized. Similarly, fractions containing prenylated GLP-1_H7F/K34H were also lyophilized. The purified peptide was dissolved in DMSO for further assay.

Natural GLP-1 was efficiently prenylated by LimF. Finally, 0.58 mg of prenylated GLP-1 was successfully obtained from 3.2 mg of GLP-1. Furthermore, GLP-1_F[7F/K34FH in which the His position was changed was also able to be efficiently prenylated by LimF. Finally, 0.42 mg of prenylated GLP-1_H7F/K34H was successfully obtained from 3.5 mg of GLP-1 H7F/K34H.

(8.2. Evaluation of Compounds)

A serum stability test, a DPP-4 digestion test, and a cAMP production activation test were performed using GLP-1 and GLP-1_H7F/K34H and the obtained prenylated GLP-1 and GLP-1 H7F/K34H.

The serum stability test was performed by incubating a 50 μM solution of each peptide (GLP-1 and GLP-1_H7F/K34H, and prenylated GLP-1 and GLP-1_H7F/K34H) in human serum at 37°C (100 μL scale). After various incubation times, a sample of 4 μL was taken from the reaction system, and the reaction was stopped by mixing with an equal amount (4 μL) of methanol. After centrifugation at 13,000 rpm for 5 minutes, the supernatant was collected, and after adding 4 times the amount of 1% TFA-aqueous solution, centrifugation was performed for an additional 5 minutes. 1 μL of the obtained supernatant was measured by LC/MS, and the amount of each remaining peptide was quantified.

The DPP-4 digestion test was performed by incubating a 50 μM Tris-Cl (pH 8.0) solution of each peptide (GLP-1 and GLP-1_H7F/K34H, and prenylated GLP-1 and GLP-1_H7F/K34H) in the presence of 2.5 ng/μL DPP-4 at 37°C (100 μL scale). After various incubation times, a sample of 4 μL was taken from the reaction system, and the reaction was stopped by adding 4 times the amount (16 μL) of a 1% TFA-aqueous solution. After centrifugation at 13,000 rpm for 5 minutes, 1 μL of the supernatant was measured by LC/MS, and the amount of each remaining peptide was quantified.

For the cAMP production activation test, each peptide (GLP-1 and GLP-1_H7F/K34H, and prenylated GLP-1 and GLP-1_H7F/K34H) was measured using a cAMP HunterTM eXpress GLP1R CHO-K1 GPCR assay kit purchased from DiscoveRx (Fremont, California, USA) and following the manufacturer's protocol. The concentration range of the used peptides was from 0.33 pM to 20 nM.

The results of each test are shown in FIG. 15.

As illustrated in FIGS. 15 (a) and (b), unmodified GLP-1 and GLP-1_H7F/K34H exhibited rapid degradation; however, the respective prenylated forms thereof exhibited higher stability.
Furthermore, as illustrated in FIG. 15 (c), natural type GLP-1 strongly activated cAMP production; however, cAMP production activation ability was significantly reduced for the His7-prenylated peptide. This is consistent with the fact that His7 is known to be important when GLP-1 binds to receptors. It was also found that GLP-1_H7F/K34H in the His position was replaced maintained activity comparable to that of natural GLP-1. Furthermore, the prenylated form of GLP-1_H7F/K34H was also confirmed to have the same high cAMP production activation ability.
The above results suggest that prenylated GLP-1_H7F/K34H produced by the above method has higher stability in the blood than natural GLP-1 and has the same receptor activating ability as natural GLP-1.

[9. Further Examination of LimF Variants]

(9.1. Change in Substrate Tolerance due to LimF Mutation)

An attempt was made to change the substrate tolerance of LimF shown in FIG. 7 by introducing mutations to LimF. teLimE2 having a mutation introduced at the His -1 position, as shown in FIG. 7 (b), was used as the substrate.
The transcription-coupled *in vitro* translation system contained the following components: each proteinogenic amino acid (excluding methionine) of 500 μM, 50 mM HEPES-KOH (pH 7.6), 12 mM Mg $(OAc)_2$, 100 mM potassium acetate, 2 mM spermidine, 1 mM DTT, 20 mM phosphocreatine (Roche), 2 mM ATP, 2 mM GTP, 1 mM CTP, 1 mM UTP, 0.1 mM 10-formyl-5,6,7,8-tetrahydrofolic acid, 1.5 mg/mL *E. coli* total tRNA (Roche), 0.73 μM AlaRS, 0.03 μM ArgRS, 0.38 μM AsnRS, 0.13 μM AspRS, 0.02 μM CysRS, 0.06 μM GlnRS, 0.23 μM GluRS, 0.09 μM GlyRS, 0.02 μM HisRS, 0.4 μM IleRS, 0.04 μM LeuRS, 0.11 μM LysRS, 0.03 μM MetRS, 0.68 μM PheRS, 0.16 μM ProRS, 0.04 μM SerRS, 0.09 μM ThrRS, 0.03 μM TrpRS, 0.02 μM TyrRS, 0.02 μM ValRS, 0.6 μM MTF, 2.7 μM IF1, 0.4 μM IF2, 1.5 μM IF3, 0.26 μM EF-G, 10 μM EF-Tu, 0.66 μM EF-Ts, 0.25 μM RF2, 0.17 μM RF3, 0.5 μM RRF, 0.1 μM T7 RNApolymerase, 0.1 μM nucleoside diphosphate kinase, 4 μg/mL creatine kinase, 3 μg/mL myokinase, 0.1 μM pyrophosphatase, 1.2 μM ribosomes, and a 0.04 μM mDNA template.
A DNA template and tRNA $^{fMet}{}_{CAU}$ charged with $^{ClAc}{}_{D}$Phe were added to the above composition at final concentrations of 0.04 μM and 50 μM, respectively. Next, the mixture was incubated for 1 hour at 37°C to promote translation and spontaneous thioether ring formation.
After one hour of translation, 3 μL of a translation mixture was separately mixed with 2 μL of an enzyme/GPP mixture solution to form a reaction mixture containing 20 to 100 μM LimF or variant and 1 mM GPP. The same conditions were used for the reaction with teMP subjected to *in vitro* translation to study the substrate tolerance of LimF, with the exception of 10 μM LimF. After incubating for 16 hours at 25°C, the reaction was stopped by adding 45 μL of a 1% TFA aqueous solution. Next, the mixture was incubated on ice for 10 minutes, a precipitate was separated by centrifugation at 4°C (15300×g,10 minutes), and 10 μL of supernatant was subjected to LC-MS analysis.
An amino acid forming a small pocket that stores the -1 position side chain of the substrate in the crystal structure was selected for the introduction site of the LimF mutation. Specifically, the 52nd Ile and 72nd Gln were selected, and variants were prepared by substituting each of these with Ala. The relationship between the amino acid at the -1 position and prenylation efficiency for wild type LimF and each variant is illustrated in FIG. 16. From FIG. 16, it is understood that even a substrate in which a bulky amino acid such as Leu or Phe is present at the -1 position can be prenylated efficiently, particularly in the I52A variant.

(9.2. Change in Prenyl Group Donor Tolerance by Mutation of LimF)

An attempt was made to change the selectivity of LimF with respect to prenyl group donors examined in "2. Evaluation of Enzymatic Activity of LimF" by introducing a mutation into LimF. teLimE2, illustrated in FIG. 7(a), was used as the substrate.
For the assay of LimF variants, 100 μM bcLimE2 was incubated individually at a 5 μL scale with 20 to 100μM LimF variants under conditions of 1 mM GPP or DMAPP, 50 mM HEPES (pH 7.2), 40 mM $MgCl_2$, 1 mM DTT, and DMSO (4%, v/v). After incubating for 16 hours at 25°C, the reaction was stopped by adding 9 times the amount (45 μL) of a 1% TFA aqueous solution. Next, the mixture was incubated on ice for 10 minutes, a precipitate was separated by centrifugation at 4°C (15300×g, 10 minutes), and 1 μL of supernatant was subjected to LC-MS analysis.
An amino acid forming a small pocket that stores a prenyl group in the crystal structure was selected for the introduction site of the LimF mutation. Specifically, Gly at position 224 was selected, and this was substituted with a larger amino acid. Note that, as illustrated in FIG. 17, when a plurality of prenyltransferases were aligned, it was found that the C10 prenyl group (geranyl group) transferases had a characteristic Gly in the position corresponding to the

224th position of LimF. This alignment supports the hypothesis that the 224th Gly contributes to prenyl group selectivity.

The relationship between prenylation efficiency and the prenyl group donor for each variant is illustrated in FIG. 18. From FIG. 18, it is understood that C5 prenylation (dimethylallylation) occurs selectively, particularly in the G224M variant.

Similarly, LimF variants having high selectivity for C15 prenylation (farnesylation) were also investigated.

For the assay of LimF variants, 100 μM bcLimE2 was incubated individually at a 5 μL scale with 20 to 100μM LimF variants under conditions of 1 mM GPP or FPP, 50 mM HEPES (pH 7.2), 5 mM $MgCl_2$, 1 mM DTT, and DMSO (4%, v/v). After incubating for 16 hours at 25°C, the reaction was stopped by adding 9 times the amount (45 μL) of a 1% TFA aqueous solution. Next, the mixture was incubated on ice for 10 minutes, a precipitate was separated by centrifugation at 4°C (15300×g, 10 minutes), and 1 μL of supernatant was subjected to LC-MS analysis.

An amino acid forming a small pocket that stores a prenyl group in the crystal structure was selected for the introduction site of the LimF mutation. Specifically, the amino acids at the positions described in the table in the left column of FIG. 19 were replaced with relatively small Ala. As a result, when the 273rd Trp was substituted, farnesylation activity increased (19%; FIG. 19).

Therefore, substitution of amino acids other than Ala with respect to the W273 position was comprehensively performed (middle column of FIG. 19). As a result, The mutation for W273N and W273T are focused, and in addition to the W273N mutation and the W273T mutation, simultaneous substitution with various amino acids was performed for His at 239, which is an amino acid adjacent to position 273 (right column of FIG. 19). As a result, it was found that C15 prenylation (farnesylation) occurs with high selectivity and high efficiency in the H239G/W237T variant and the H239G/W237N variant.

Thus, the production of a peptide having a wider prenyl group backbone was confirmed.

Next, kinetic parameters were measured for C10 prenylation of wild-type LimF, C5 prenylation of the G224M variant, and C15 prenylation of the H239G/W237T variant.

Specifically, a modification reaction was performed at 25°C with a 10 μM LimF or LimF variant; 1 mM GPP, DMAPP, FPP, or GGPP; 50 mM HEPES (pH 7.2); 5 mM MgCh; 1 mM DTT, DMSO (4%, v/v), and various concentrations of bcLimE2. The initial rate and substrate concentration were fitted to the Michaelis-Menten model in Graphpad Prism 8.4.2 software to estimate the kinetic parameters.

Furthermore, in order to test the concentration dependence of prenyl group donors, a modification reaction was performed with a 1 μM LimF or LimF variant; 1mM or various concentrations of GPP, DMAPP, FPP, or GGPP; 50 mM HEPES (pH 7.2); 0.1 mM bcLimE2, 1 mM DTT, and 5 mM MgCh. After preheating to 25°C without a prenyl group donor, the reaction was initiated by the addition of a prenyl group donor. After incubating at 25°C, the reaction was stopped at each point and analyzed by LC-MS. Next, the initial rate was calculated as the slope of the linear region. Each experiment was repeated three times. The initial rate for the concentration dependence of prenyl group donors was fitted to the Michaelis-Menten model using nonlinear regression analysis in Graphpad Prism 8.4.2 software. The results are shown in the following table. In Table 18, "n.r." indicates that the reaction did not proceed.

[Table 18]

| Enzyme | Prenyl Group Donor | Final Conversion Efficiency | $K_M$ (mM) | $k_{cat}$ ($min^{-1}$) | $k_{cat}/K_M$ ($min^{-1}$ $mM^{-1}$) |
|---|---|---|---|---|---|
| **wt-LimF** | **DMAPP** | **n.r.** | **-** | **-** | **-** |
| | **GPP** | **>99%** | **0.31 ± 0.04** | **13.8 ± 0.6** | **44.1** |
| | **FPP** | **0.0027%** | **-** | **-** | **-** |
| | **GGPP** | **n.r.** | **-** | **-** | **-** |
| **LimF-G224M** | **DMAPP** | **>99%** | | **Not tested** | **-** |
| | **GPP** | **n.r.** | **-** | **-** | **-** |
| | **FPP** | **n.r.** | **-** | **-** | **-** |
| | **GGPP** | **n.r.** | **-** | **-** | **-** |
| **LimF-H239G/W273T** | **DMAPP** | **n.r.** | **-** | **-** | **-** |
| | **GPP** | **16.9%** | **0.58 ± 0.07** | **0.022 ± 0.001** | **0.04** |
| | **FPP** | **>99%** | **0.62 ± 0.06** | **0.63 ± 0.03** | **1.02** |
| | **GGPP** | **n.r.** | **-** | **-** | **-** |

[Table 19]

| | |
|---|---|
| **LimF-H239G/W273T** | **$K_{FPP}$ = 0.014 ± 0.001 mM** |
| | ***Vmax* = 0.849 ± 0.014 min$^{-1}$** |
| **wt-LimF** | **$K_{GPP}$ = 0.11 ± 0.014 mM** |
| | ***Vmax* = 10.03 ± 0.24 min$^{-1}$** |

Next, it was confirmed that the H239G/W237T variant also had high substrate tolerance similar to LimF. Three types of non-natural substrates (cimetidine, GLP-1, and GLP-1_H7F/K34H) were used as the substrate.

Specifically, for the farnesylation of cimetidine, GLP-1 or GLP-1_H7F/K34H, 0.2 mM of each compound was incubated for 20 hours with a 50 μM LimF_H239G/W273T variant and 1 mM FPP under previously optimized standard conditions.

FIG. 20 shows the farnesylation efficiency of each compound. It was confirmed that farnesylation of a non-natural substrate can be achieved by using a H239G/W273T variant.

Finally, it was confirmed that the positional selectivity for prenylation was unchanged from that of wild-type LimF with respect to the C5 prenylation of the G224M variant and the C15 prenylation of the H239G/W237T variant. MS/MS analysis similar to the method described in [2. Evaluation of Enzymatic Activity of LimF] confirmed that the G224M variant and the H239G/W237T variant also dimethylallylated and farnesylated the imidazole C2 carbon, respectively, just as wild-type LimF geranylates the C2 carbon on the imidazole ring of the His side chain.

[10. Additional Notes]

His is one of four proteinogenic amino acids having an aromatic side chain and is characterized by an electron-deficient heteroaromatic imidazole side chain which plays an essential role in protein/peptide functions such as metal bonds, hydrogen bonds, proton transport, nucleophiles, and the like (Liao, S.-M., Du, Q.-S., Meng, J.-Z., Pang, Z.-W. & Huang, The multiple roles of histidine in protein interactions. Chem. Cent. J., 7, 1-12 (2013)). However, His modification rarely occurs. Among these, nucleophilic heteroatom modifications such as N-phosphorylation and alkylation are common in both enzymatic and chemical approaches (Jia, S., He, D. & Chang, C.J. Bioinspired Thiophosphorodichloridate Reagents for Chemoselective Histidine Bioconjugation. Journal of the American Chemical Society 141, 7294-7301 (2019).; Kee, J.-M. & Muir, T.W. Chasing Phosphohistidine, an Elusive Sibling in the Phosphoamino Acid Family. ACS Chem. Biol. 7, 44-51 (2012)).

A series of aromatic PTases are characterized in that various prenylation reactions are performed on aromatic compounds, mainly low molecular weight compounds; however, the prenylation of aromatic residues on peptides is limited to the indole and phenol groups of Trp and Tyr residues, respectively. There are few low molecular weight natural products having a prenylated imidazole structure because the carbon atoms on the imidazole ring are electron-deficient and functionalization is difficult (Larsen, T.O., Frisvad, J.C. & Jensen, Aurantiamine, a diketopiperazine from two varieties of Penicillium aurantiogriseum. Phytochemistry, 31, 1613-1615 (1992).; Kanoh, K. et al. (-)-Phenylahistin: a new mammalian cell cycle inhibitor produced by Aspergillus ustus. Bioorg. Med. Chem. Lett., 7, 2847-2852 (1997)).

Natural products modified at the most electron-deficient C-2 of imidazole are even rarer, and only one compound having a C-2 dimethylallylated imidazole structure has been found thus far (Jan, C., Dippenaar, A. & Holzapfel, Crystal structure of the metal complexes of viridamine. S. Afr. J. Chem., 30, 161-168 (1977)).

So far, enzymes that catalyze the C-alkylation of His have remained unexplored due to the rarity of such structures in nature. As far as we know, LimF is the first structurally-analyzed Ptase to catalyze the C-2 alkylation of His.

In addition to His prenylation activity, LimF also exhibits the function of Tyr prenylation. Although a variety of sequences are tolerated in most cyanobactin Ptases, the sites to be modified are selective. It is unprecedented that a cyanobactin Ptase, like LimF, catalyzes C- and O-prenylation of two different amino acids.

On the other hand, peptide-based molecules have become promising therapeutic methods, particularly for their ability to target for the proteins of interest (POI) in cells.

In regard to the discovery of peptide ligands, the well-established RaPID system using a thioether cyclic peptide library has been proven as a powerful workflow for novel screening peptide ligands for most types of the POI (Taguchi, S. & Suga, Targeting of extracellular protein-protein interactions with macrocyclic peptides. Curr. Opin. Chem. Biol. 62, 82-89 (2021).; Peacock, H. & Suga, Discovery of De Novo Macrocyclic Peptides by Messenger RNADisplay. Trends Pharmacol. Sci., 42, 385-397 (2021)).

Given the fact that LimF has been proven to be able to prenylate such non-natural macrocyclic backbones, the LimF integrated RaPID system will be able to achieve the identification of a variety of prenylated peptide ligands that target desired POIs with improved membrane permeability.

In addition, it is thought that the discovery of LimF will contribute to the expansion of peptide modification methods. Despite considerable advances in the previously reported methods of peptide modification, these are heavily dependent on nucleophilic residues such as Cys and Lys.

Although two recent reports have demonstrated His C-2 alkylation mediated by a radical-mediated approach, direct the late-stage functionalization of His is still in its early stages due to the presence of various interfering functional groups on peptides (Noisier, A.F. et al. Late-Stage Functionalization of Histidine in Unprotected Peptides. Angew. Chem., 58, 19096-19102 (2019).; Chen, X. et al. Histidine-Specific Peptide Modification via Visible-Light-Promoted C-H Alkylation. J. Am. Chem. Soc. 141, 18230-18237 (2019)).

The present specification demonstrates that various compounds (low molecular weight compounds, peptides, and proteins) are selectively prenylated by LimF. The structural analysis of LimF has enabled LimF to be designed with increased versatility, such as by changing substrate interacting residues to adapt to substrates with low prenylation efficiency. Due to its remarkable chemical selectivity and positional selectivity, high substrate tolerance, and mild reaction conditions, LimF is expected to serve as a powerful biocatalyst for chemically-difficult position-selective His C-2 functionalization.

Furthermore, phylogenetic analysis and SSN analysis suggested that 11 types of proteins derived from organisms different from LimF exhibit His C-prenylation activity and/or Tyr O-prenylation activity in a manner similar to LimF.

## Claims

1. A method for producing a compound having at least one of the structures represented by the following formulas (I) or (II):

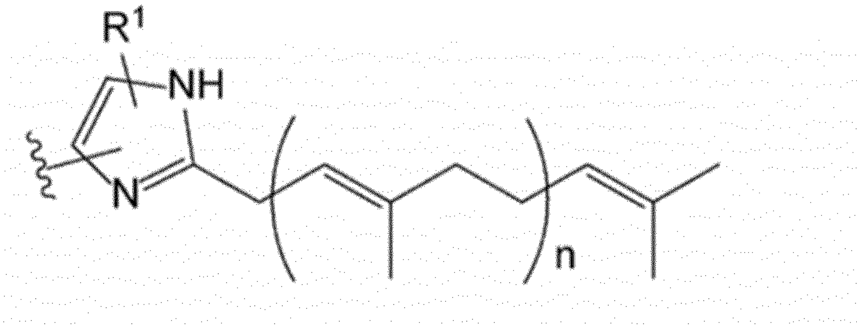

(I)

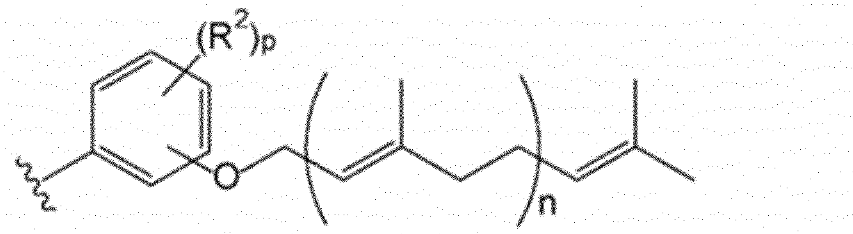

(II)

(in formula (I) above, $R^1$ is a hydrogen atom or optional substituent, and n represents an integer from 0 to 11; in formula (II) above, each $R^2$ is independently optional substituent, p represents an integer of from 0 to 4 and n represents an integer of from 0 to 11),
comprising a step of:
contacting a compound having at least one of the structures represented by the following formula (III) or (IV) with prenyltransferase to introduce a prenyl group into the structure,

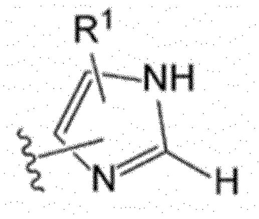

(III)

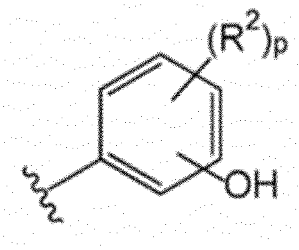

(IV)

(in formula (III) and formula (IV) above, $R^1$, $R^2$, and P are defined as in the foregoing), wherein the prenyltransferase is LimF or an enzyme homologous thereto.

2. The method according to claim 1, wherein a peptide or protein having an amino acid sequence containing at least one His, Tyr, or derivative thereof is contacted with the prenyltransferase, and thereby a prenyl group is introduced into at least one His residue, Tyr residue, or derivative residue thereof.

3. A method for producing a compound library containing prenylated peptides or proteins, comprising a step of: contacting compound library containing peptides or proteins having an amino acid sequence that contains at least one His, Tyr, or derivative thereof with prenyltransferase to introduce a prenyl group to the at least one His residue, Tyr residue, or derivative residue thereof,
wherein the prenyltransferase is LimF or an enzyme homologous thereto.

4. The method according to claim 3, further comprising translating an mRNA library through a cell-free translation system to prepare the compound library containing peptides or proteins having an amino acid sequence that contains at least one His, Tyr, or derivative thereof.

5. The method according to claim 3 or 4, wherein the peptides or the proteins in the compound library are bound to a genotype.

6. The method according to claim 5, further comprising a process for preparing the compound library containing peptides or proteins that are bound to a genotype by an mRNA display method,
wherein the process comprising steps of:

preparing an mRNA library of respective mRNA that encode the peptides or proteins having an amino acid sequence that contains at least one His, Tyr, or derivative thereof;
binding puromycin to the 3' terminal of the respective mRNA in the mRNA library to produce a puromycin-bound mRNA library; and
translating the puromycin-bound mRNA library through the cell-free translation system.

7. The method according to any one of claims 1 to 6, wherein the prenyltransferase comprises prenyltransferase derived from *Limnothrix* sp. or *Symploca* sp. or an enzyme homologous thereto.

8. The method according to any one of claims 1 to 6, wherein the prenyltransferase comprises enzymes containing an amino acid sequence that corresponds to the following (1), (2), or (3).

(1) amino acid sequence represented by any of SEQ ID NOS: 1 to 12
(2) amino acid sequence that has one or more amino acids deleted, substituted, or added in an amino acid sequence represented by any of SEQ ID NOS: 1 to 12
(3) amino acid sequence that has a 80% or more homology with an amino acid sequence represented by SEQ

ID NO: 1

**9.** The method according to any one of claims 1 to 6, wherein the prenyltransferase is at least one selected from a group consisting of enzymes having an amino acid selected from a group consisting of an amino acid sequence represented by SEQ ID NO: 1, an amino acid sequence represented by SEQ ID NO: 2, and an amino acid sequence that has one or more amino acids deleted, substituted, or added in these amino acid sequences.

**10.** The method according to any one of claims 1 to 9, wherein the prenylated compound is a peptide or protein having a cyclic structure formed by four or more amino acids.

**11.** The method according to any one of claims 1 to 10, wherein the compound brought into contact with the prenyltransferase is a peptide or protein, wherein the peptide or protein has a substructure represented by Xaa1-Xaa2, or has an amino acid sequence that has His, Tyr, or a derivative thereof at the N-terminal, wherein Xaa1 is a neutral amino acid and Xaa2 is His, Tyr, or a derivative thereof.

**12.** The production method of any one of claims 1 to 11, wherein the compound brought into contact with the prenyltransferase is a peptide or protein, wherein the peptide or protein has a substructure represented by Xaa2-Xaa3, or has an amino acid sequence that has His, Tyr, or a derivative thereof at the C-terminal, wherein Xaa2 is His, Tyr, or a derivative thereof and Xaa3 is any amino acid other than Pro and a derivative thereof.

**13.** A compound library, produced by the production method according to any one of claims 1 to 12.

**14.** A method for screening compounds to identify a compound that binds to a target substance, comprising a step of: contacting the compound library produced by the production method according to any one of claims 1 to 12 with a target substance; and
selecting a compound that binds to the target substance.

[FIG. 1]

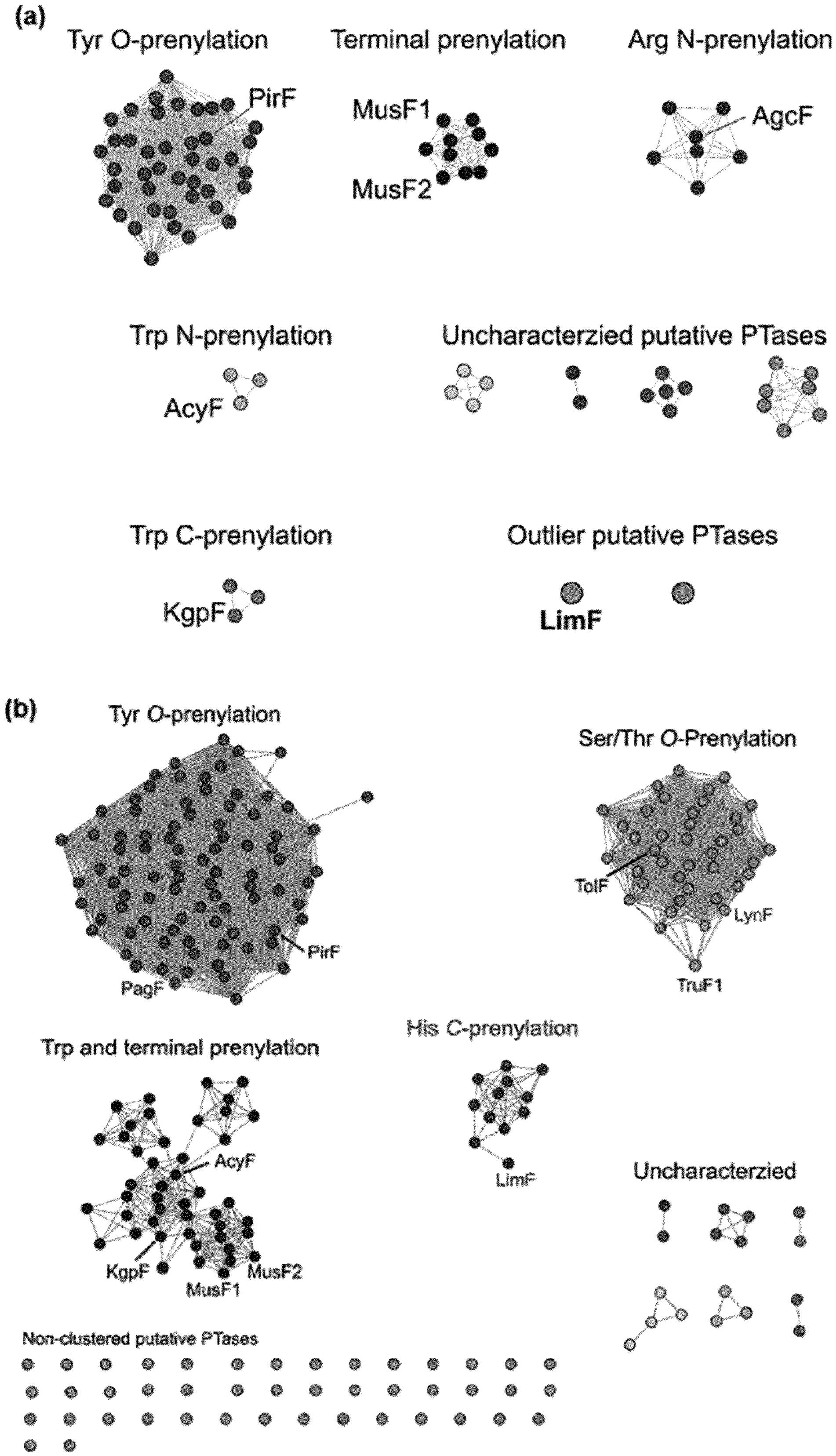
(a)
Tyr O-prenylation
PirF
Terminal prenylation
MusF1
MusF2
Arg N-prenylation
AgcF
Trp N-prenylation
AcyF
Uncharacterzied putative PTases
Trp C-prenylation
KgpF
Outlier putative PTases
LimF
(b)
Tyr O-prenylation
PirF
PagF
Ser/Thr O-Prenylation
TolF
LynF
TruF1
Trp and terminal prenylation
AcyF
KgpF
MusF1
MusF2
His C-prenylation
LimF
Uncharacterzied
Non-clustered putative PTases

[FIG. 2]

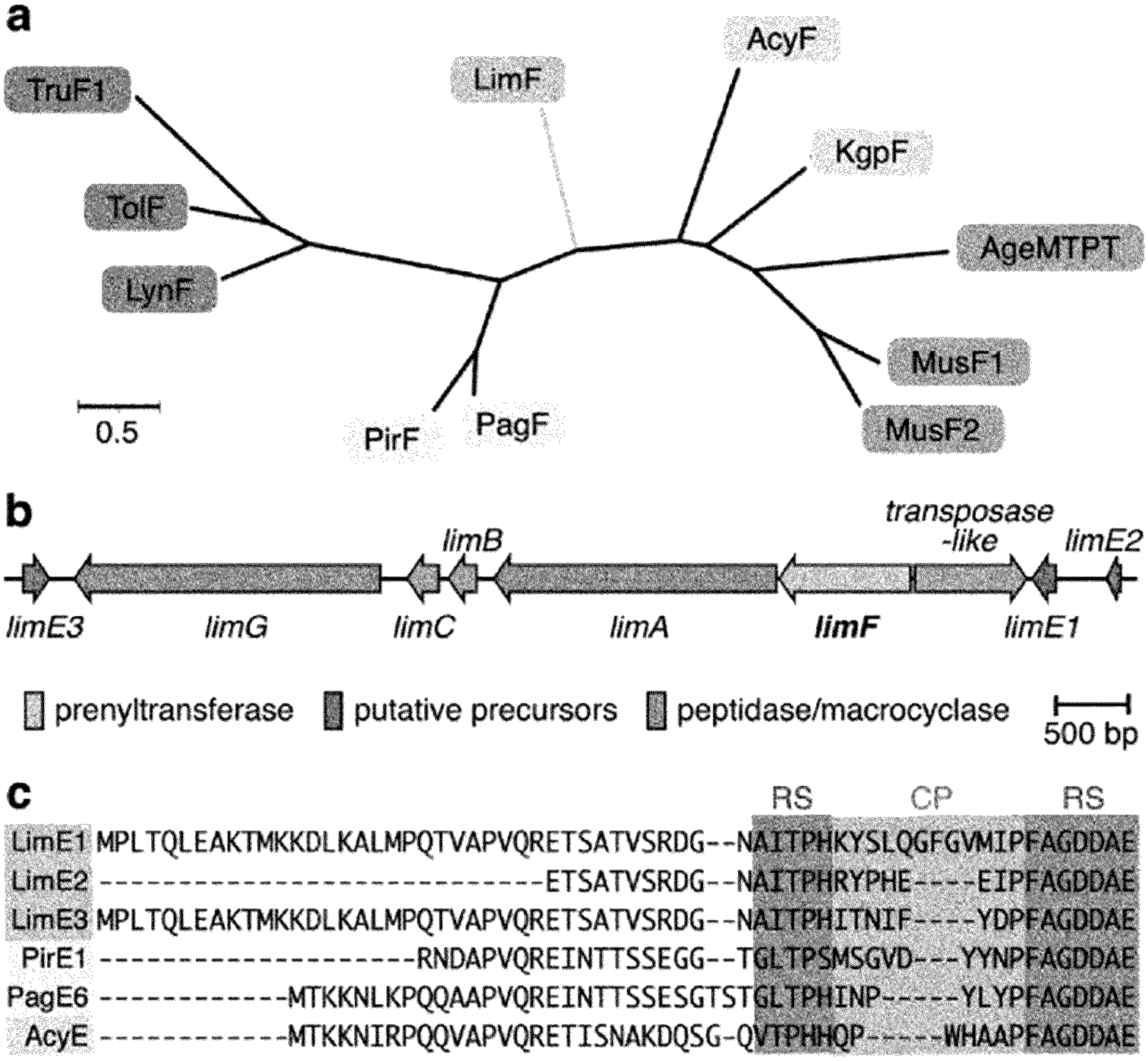
a
TruF1
TolF
LynF
LimF
AcyF
KgpF
AgeMTPT
MusF1
MusF2
PirF
PagF
0.5
b
transposase -like
limB
limE2
limE3
limG
limC
limA
limF
limE1
prenyltransferase
putative precursors
peptidase/macrocyclase
500 bp
c
RS
CP
RS
LimE1 MPLTQLEAKTMKKDLKALMPQTVAPVQRETSATVSRDG--NAITPHKYSLQGFGVMIPFAGDDAE
LimE2 ----------------------------ETSATVSRDG--NAITPHRYPHE----EIPFAGDDAE
LimE3 MPLTQLEAKTMKKDLKALMPQTVAPVQRETSATVSRDG--NAITPHITNIF----YDPFAGDDAE
PirE1 --------------------RNDAPVQREINTTSSEGG--TGLTPSMSGVD---YYNPFAGDDAE
PagE6 ------------MTKKNLKPQQAAPVQREINTTSSESGTSTGLTPHINP-----YLYPFAGDDAE
AcyE ------------MTKKNIRPQQVAPVQRETISNAKDQSG-QVTPHHQP-----WHAAPFAGDDAE

[FIG. 3]

Fig. 3a

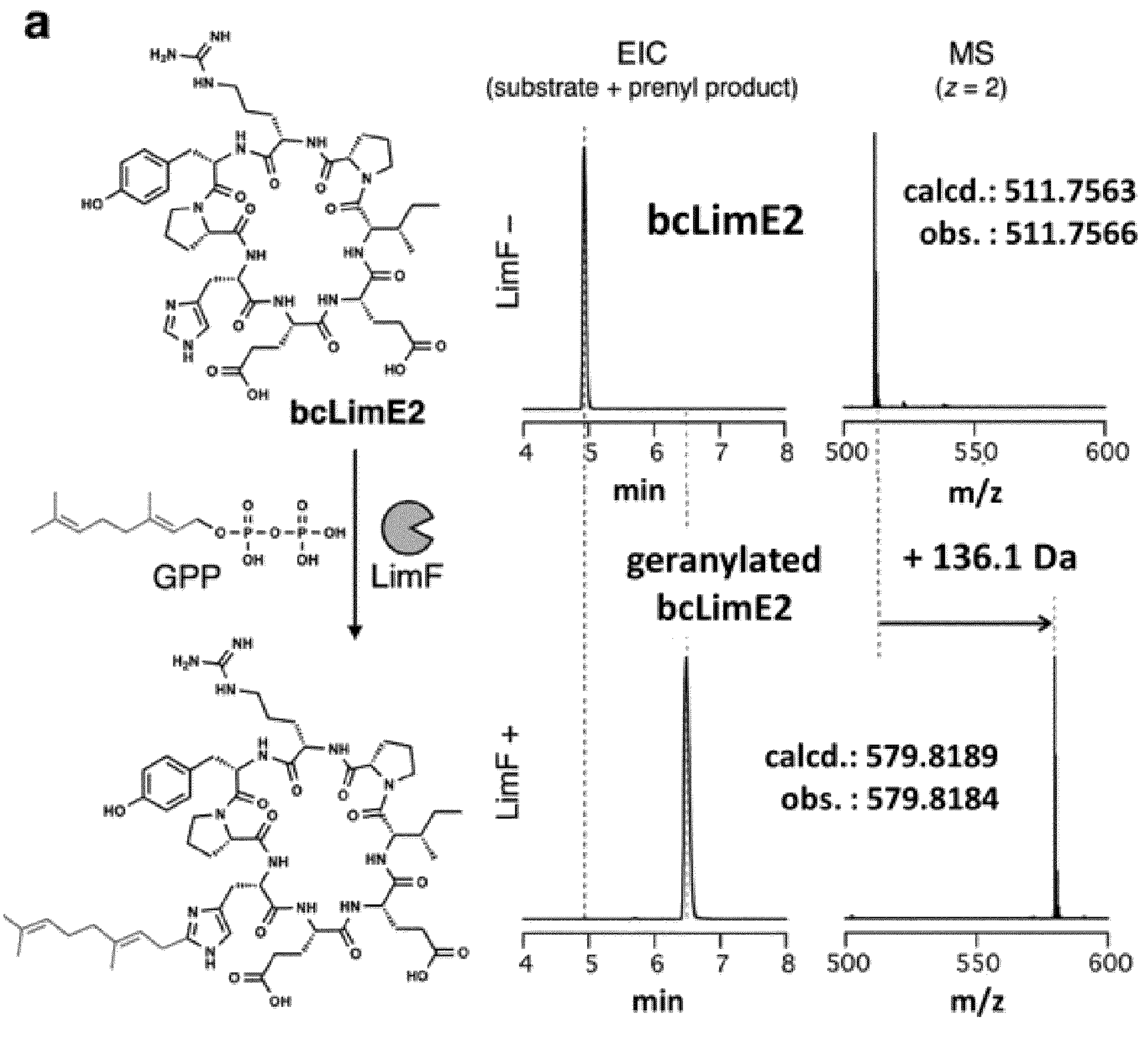
a
bcLimE2
GPP
LimF
EIC
(substrate + prenyl product)
MS
(z = 2)
LimF –
bcLimE2
calcd.: 511.7563
obs. : 511.7566
4 5 6 7 8
min
500 550 600
m/z
geranylated
bcLimE2
+ 136.1 Da
LimF +
calcd.: 579.8189
obs. : 579.8184
4 5 6 7 8
min
500 550 600
m/z

Fig. 3b

b

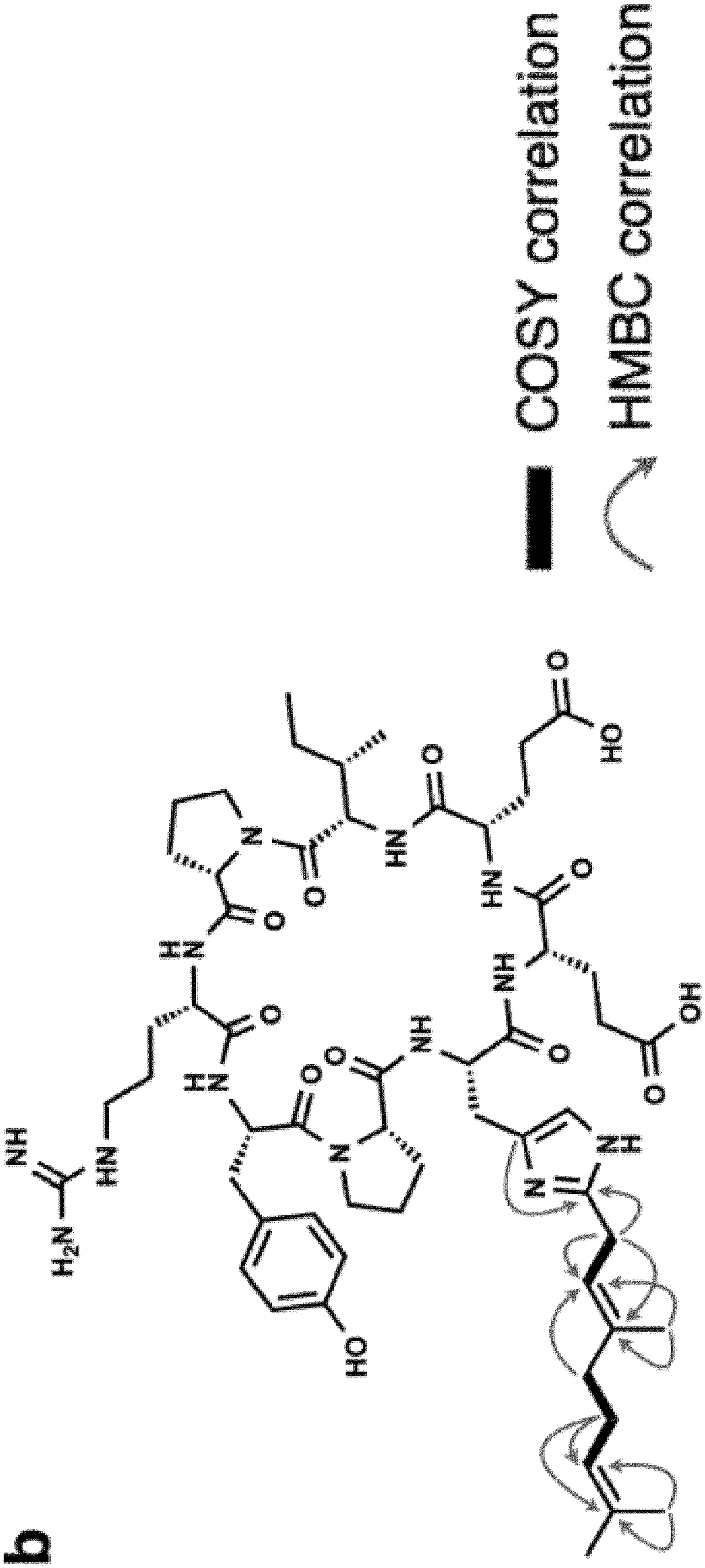


limnothamide

[FIG. 4]

Fig. 4a

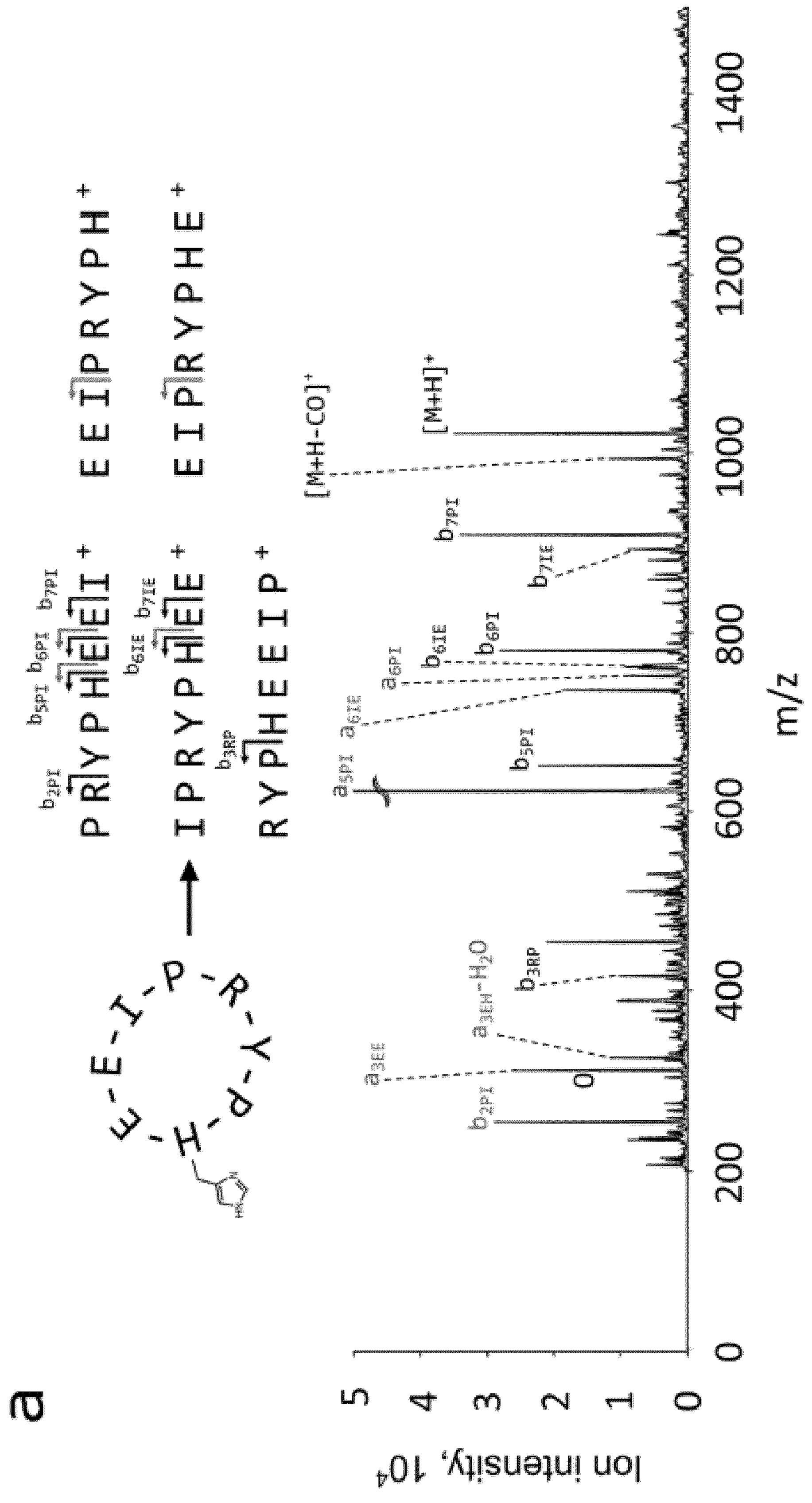
a
Ion intensity, 10^4
m/z
[M+H]+
[M+H-CO]+
E E I P R Y P H +
E I P R Y P H E +
P R Y P H E E I +
I P R Y P H E E +
R Y P H E E I P +

Fig. 4b

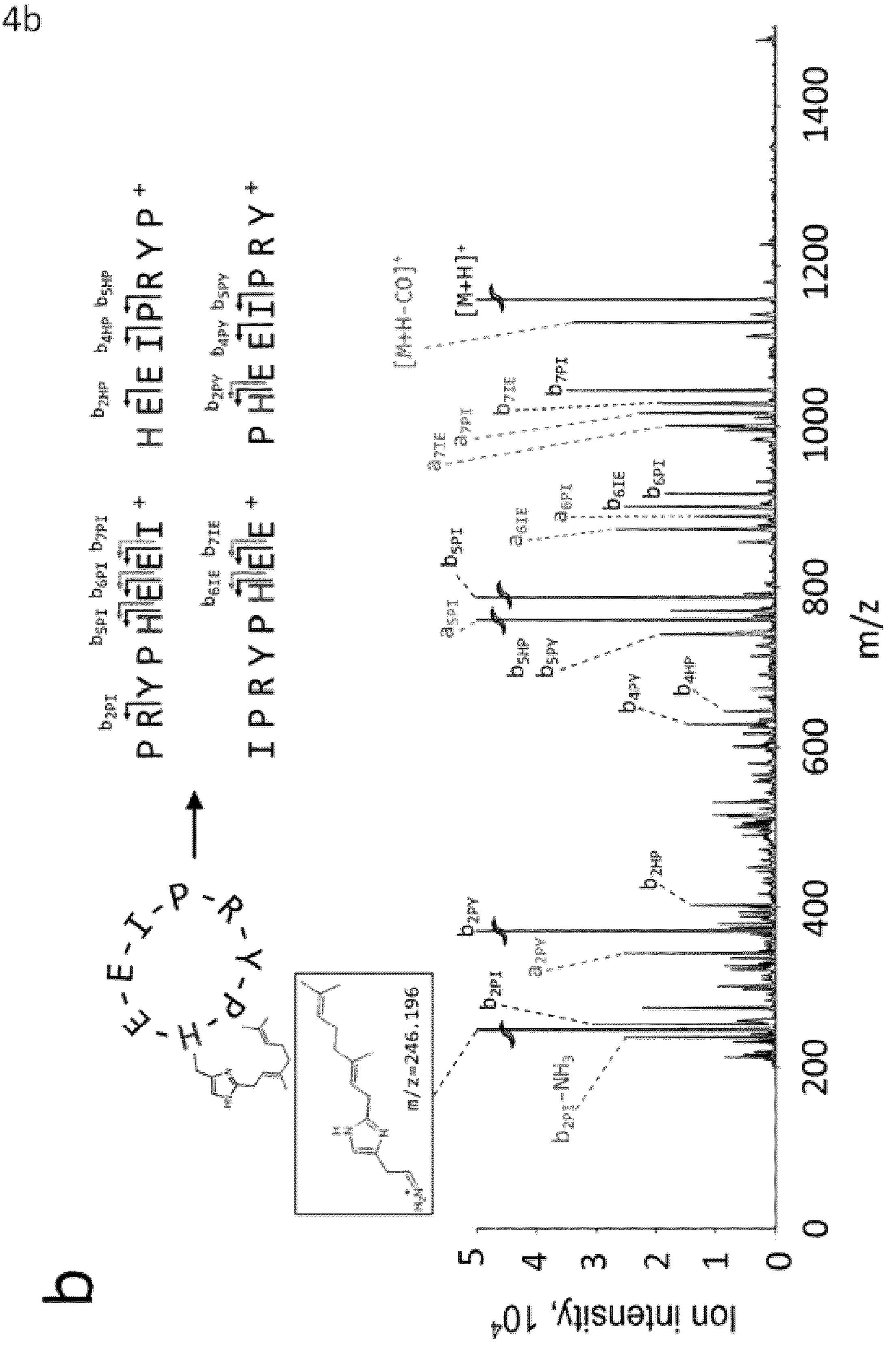

[FIG. 5]

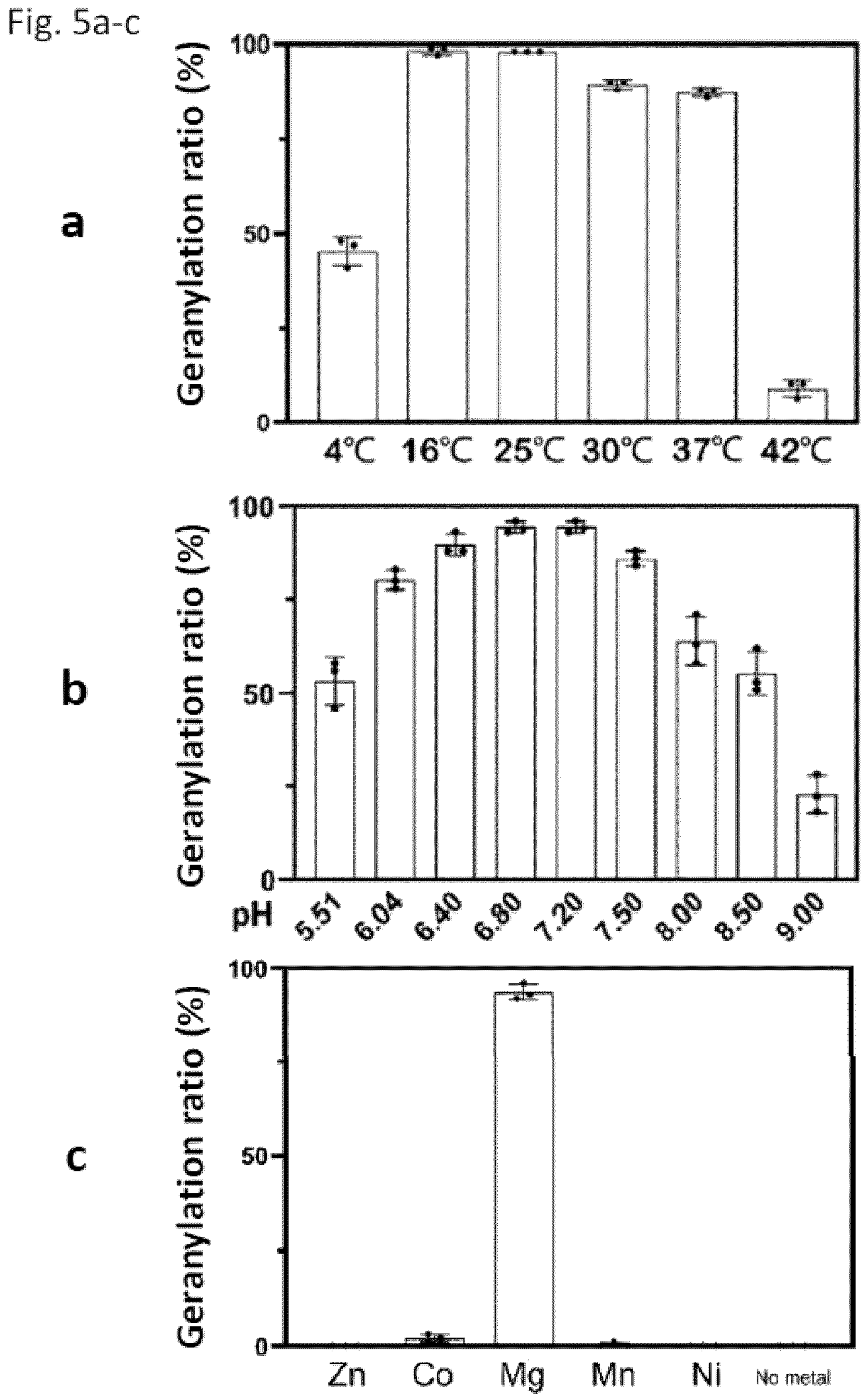
Fig. 5a-c
a
b
c
Geranylation ratio (%)
100
50
0
4℃
16℃
25℃
30℃
37℃
42℃
pH
5.51
6.04
6.40
6.80
7.20
7.50
8.00
8.50
9.00
Zn
Co
Mg
Mn
Ni
No metal

Fig. 5d

d

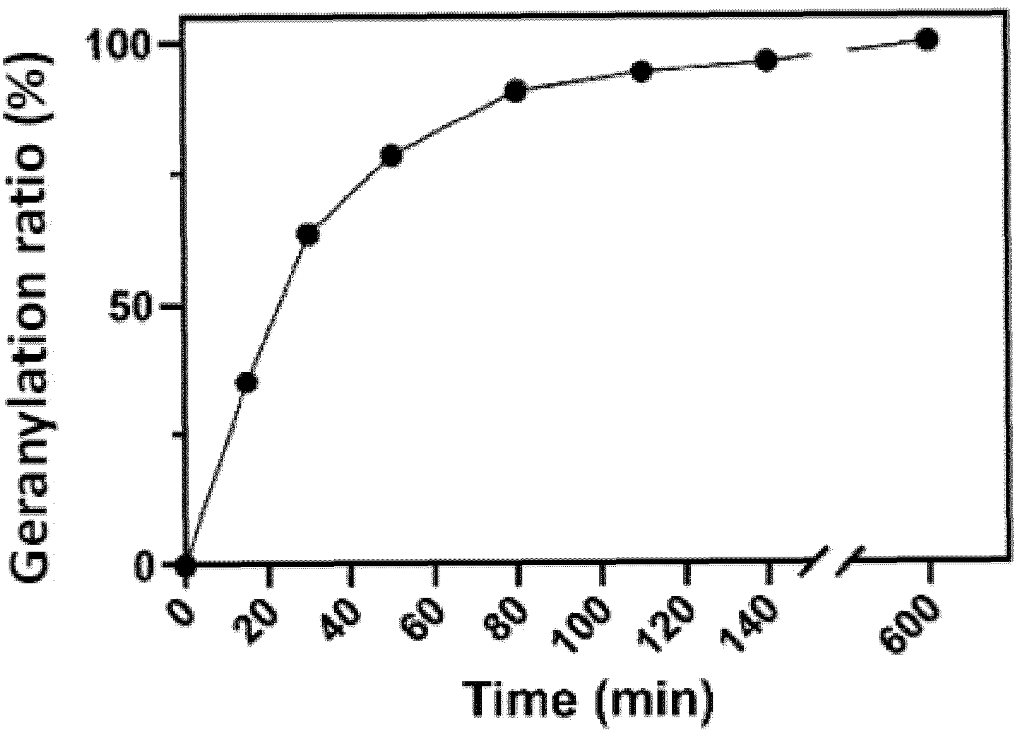
Geranylation ratio (%)
100
50
0
0
20
40
60
80
100
120
140
600
Time (min)

Fig. 5e-g

e

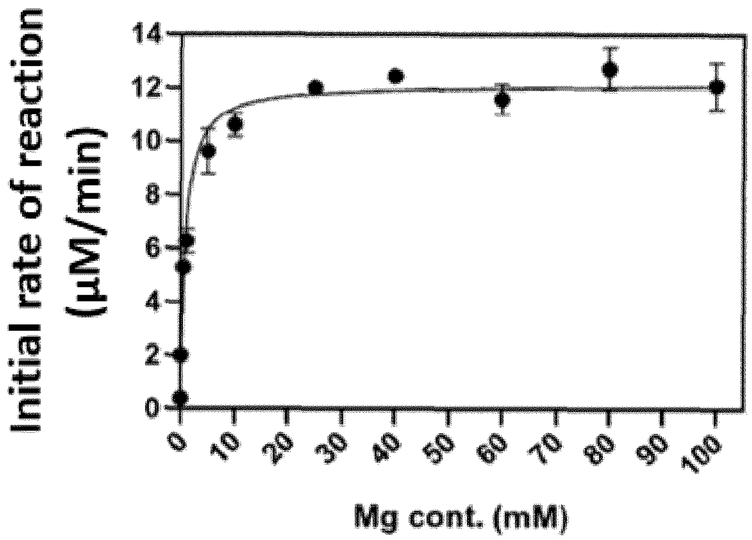
Initial rate of reaction (µM/min)
0 2 4 6 8 10 12 14
0 10 20 30 40 50 60 70 80 90 100
Mg cont. (mM)

f

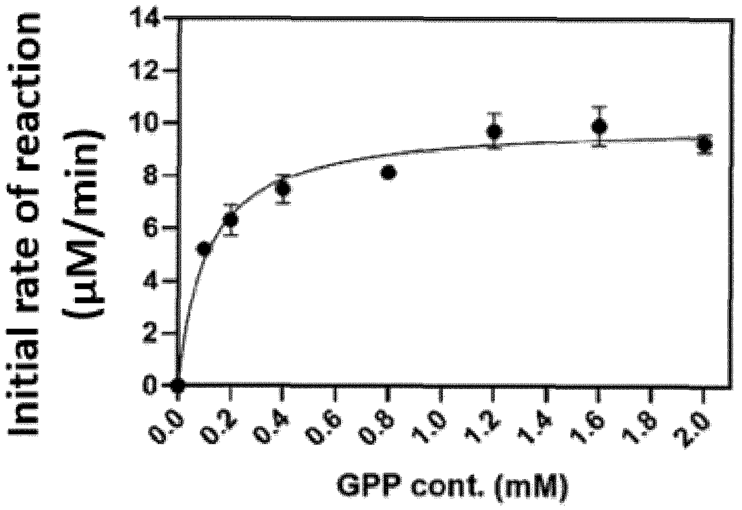
Initial rate of reaction (µM/min)
0 2 4 6 8 10 12 14
0.0 0.2 0.4 0.6 0.8 1.0 1.2 1.4 1.6 1.8 2.0
GPP cont. (mM)

g

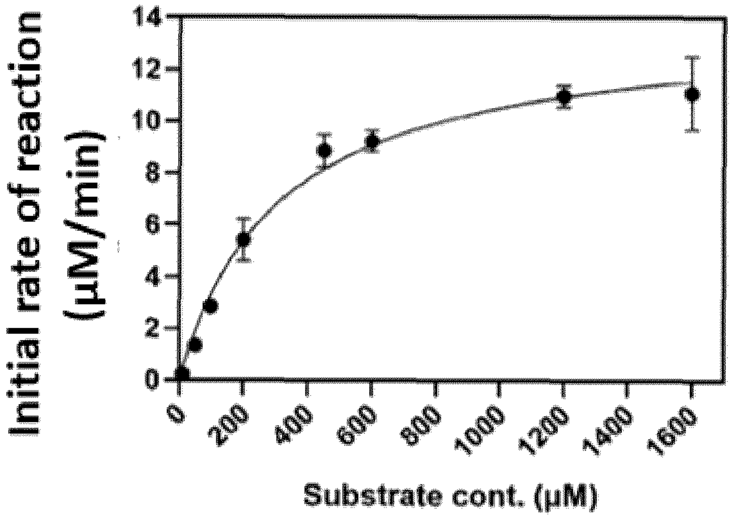
Initial rate of reaction (µM/min)
0 2 4 6 8 10 12 14
0 200 400 600 800 1000 1200 1400 1600
Substrate cont. (µM)

[FIG. 6]

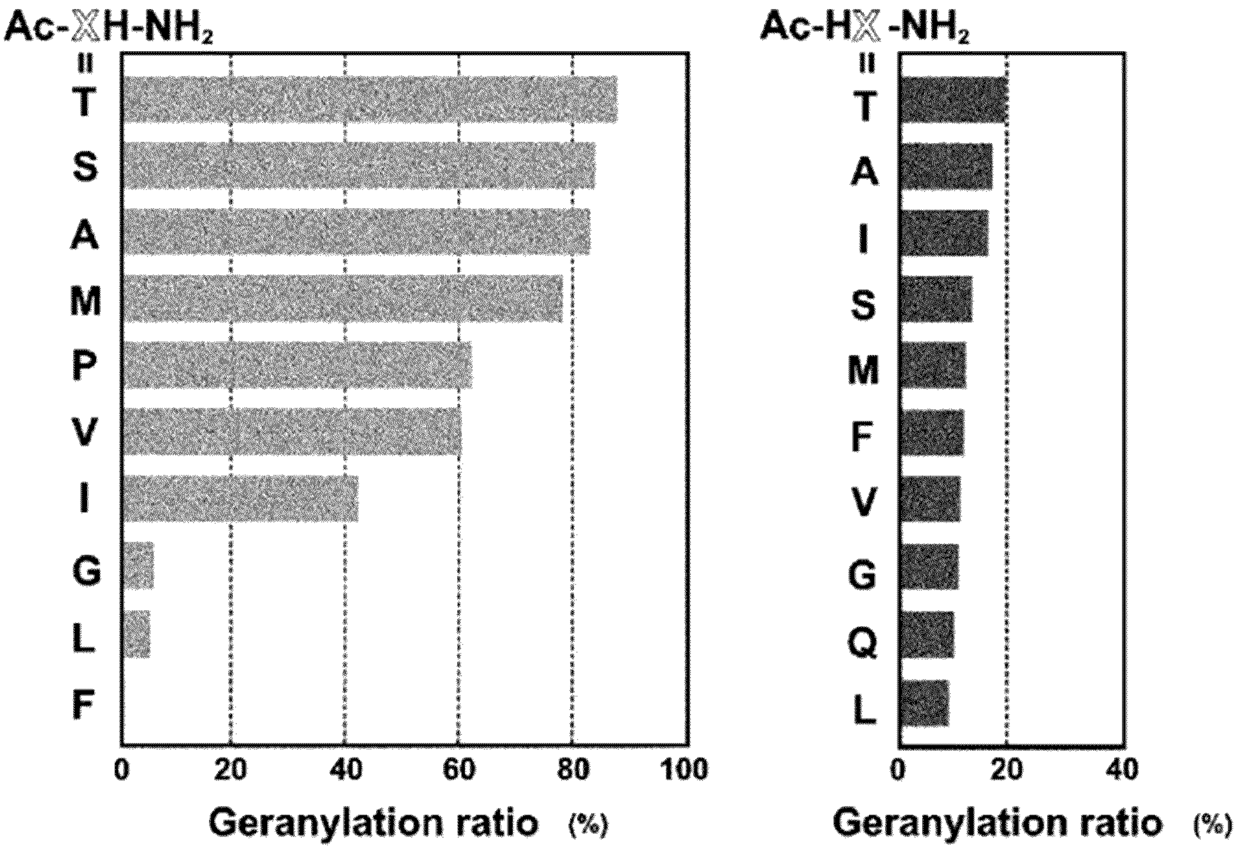
Ac-XH-NH2
T
S
A
M
P
V
I
G
L
F
0
20
40
60
80
100
Geranylation ratio (%)
Ac-HX-NH2
T
A
I
S
M
F
V
G
Q
L
0
20
40
Geranylation ratio (%)

[FIG. 7]

Fig. 7a

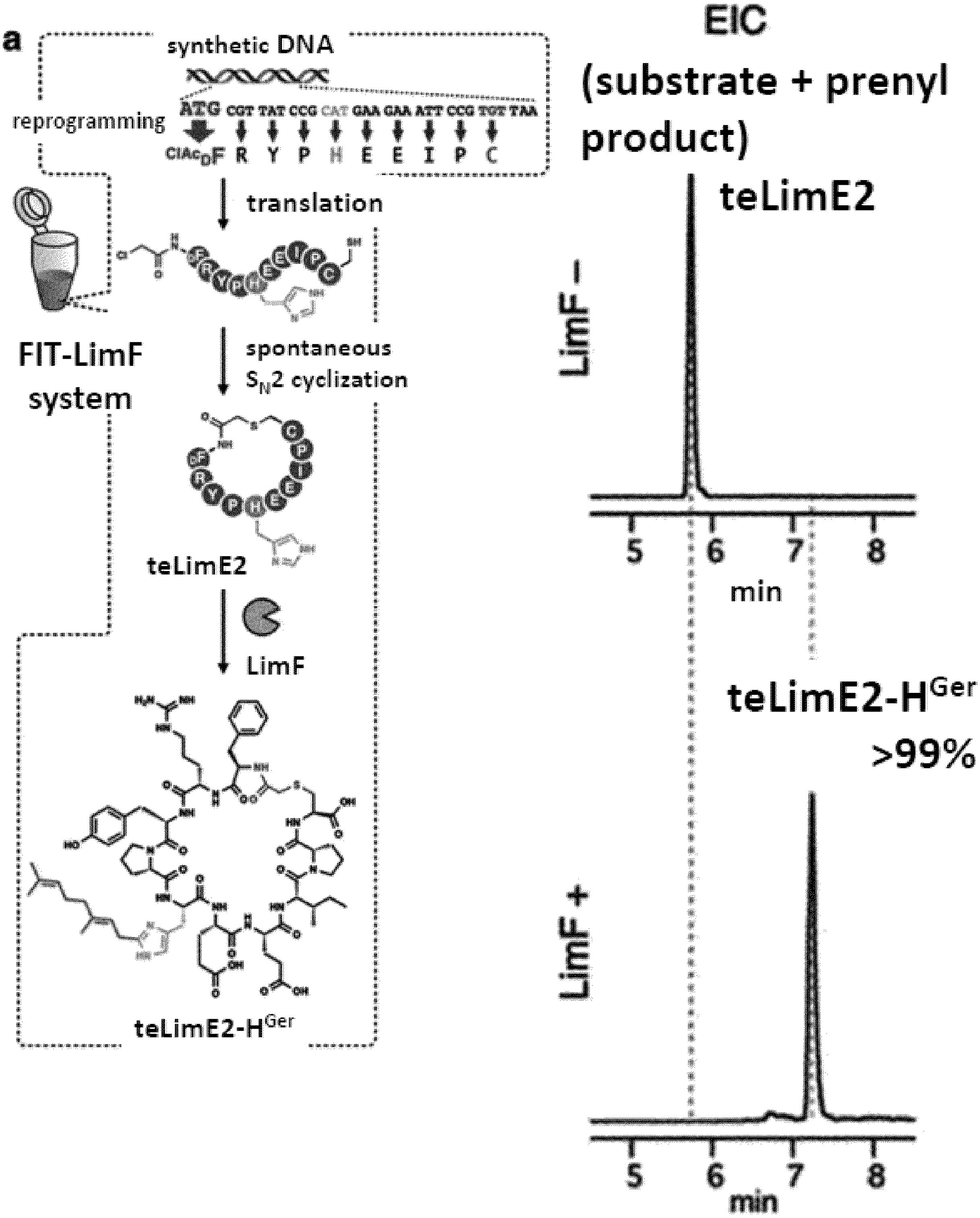
a
synthetic DNA
reprogramming
ATG CGT TAT CCG CAT GAA GAA ATT CCG TGT TAA
ClAcDF R Y P H E E I P C
translation
spontaneous
SN2 cyclization
FIT-LimF
system
teLimE2
LimF
teLimE2-HGer
EIC
(substrate + prenyl
product)
teLimE2
LimF –
5 6 7 8
min
teLimE2-HGer
>99%
LimF +
5 6 7 8
min

Fig. 7b-c

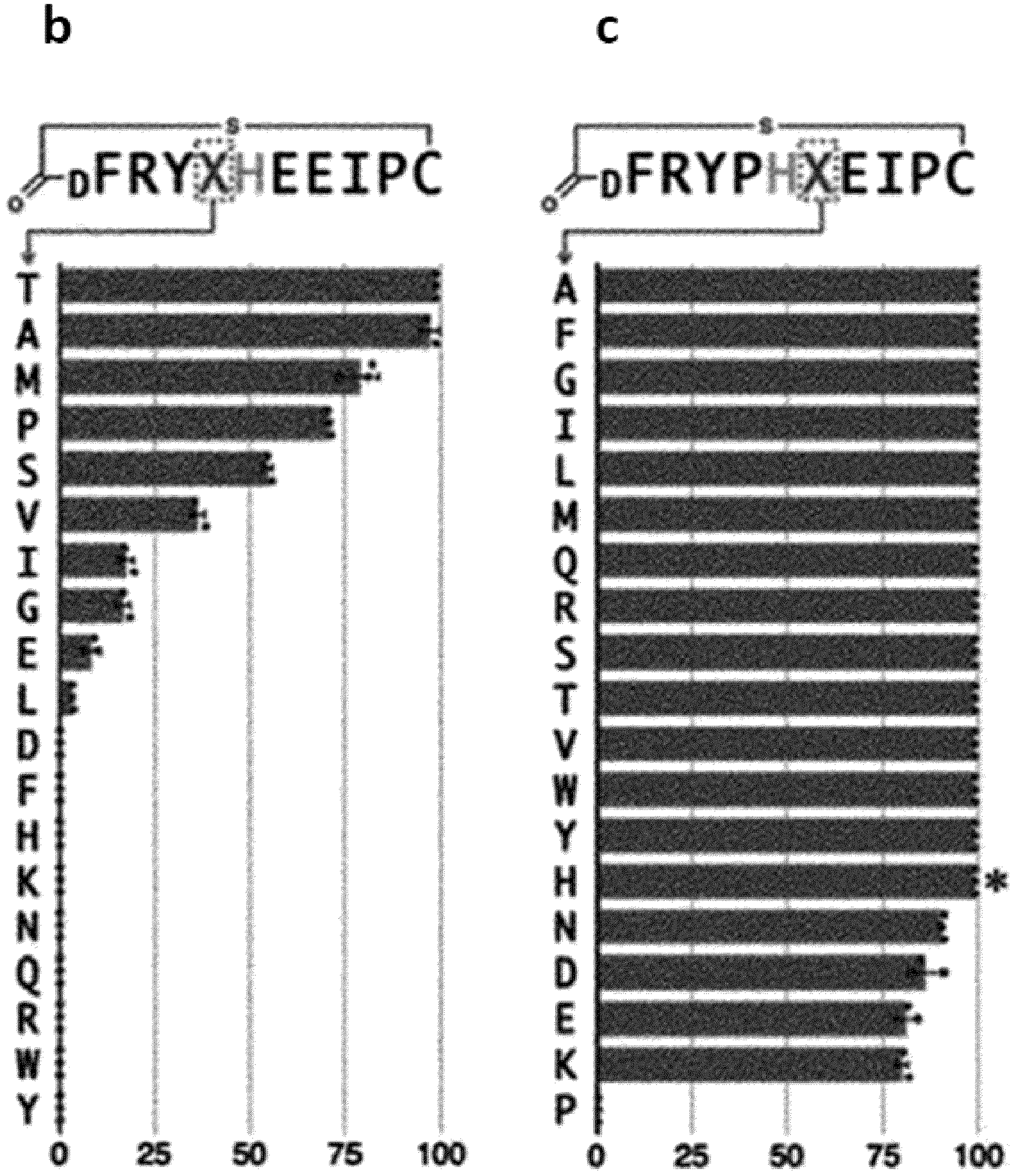
b
c
DFRYXHEEIPC
DFRYPHXEIPC
S
T
A
M
P
S
V
I
G
E
L
D
F
H
K
N
Q
R
W
Y
A
F
G
I
L
M
Q
R
S
T
V
W
Y
H
N
D
E
K
P
0
25
50
75
100
*

Fig. 7d-e

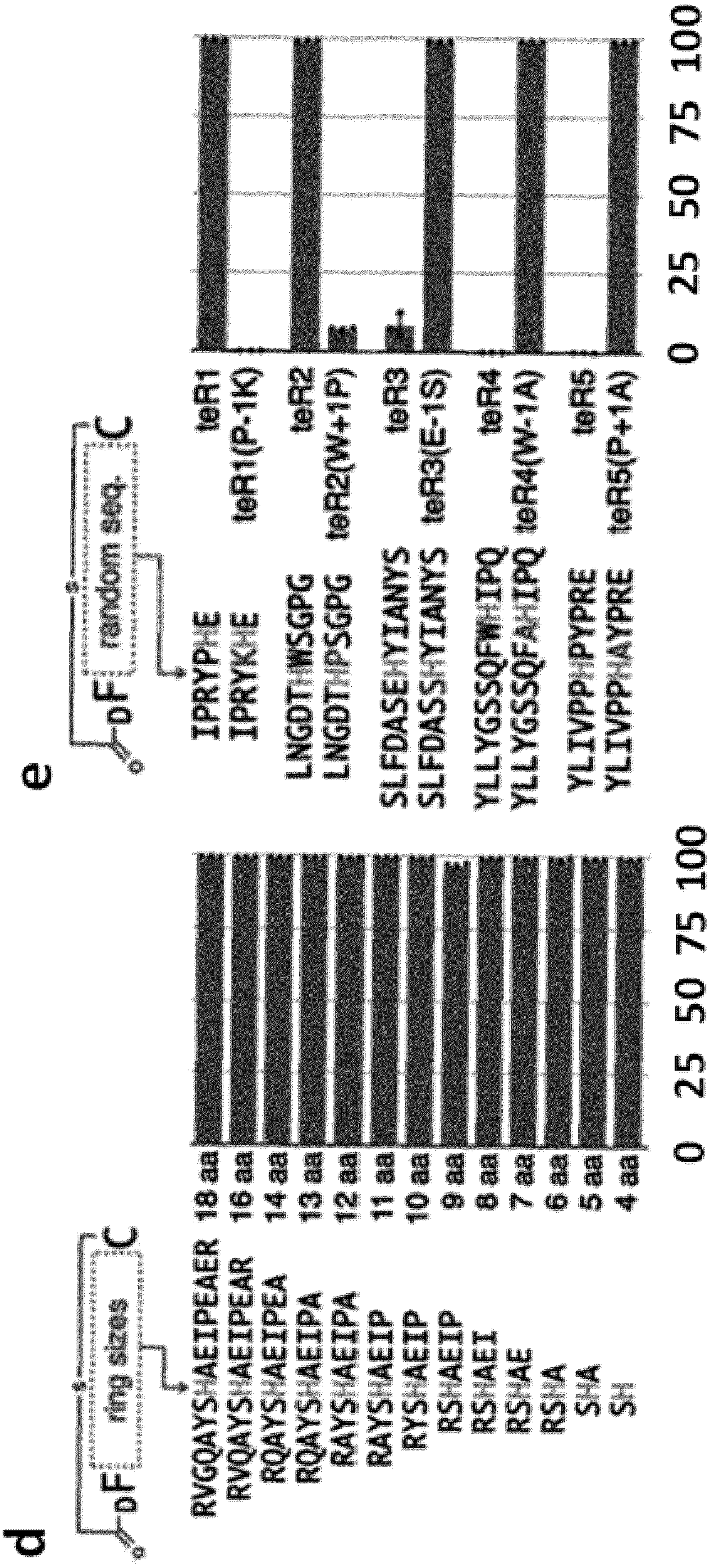
d
ring sizes
RVGQAYSHAEIPEAER 18 aa
RVQAYSHAEIPEAR 16 aa
RQAYSHAEIPEA 14 aa
RQAYSHAEIPA 13 aa
RAYSHAEIPA 12 aa
RAYSHAEIP 11 aa
RYSHAEIP 10 aa
RSHAEIP 9 aa
RSHAEI 8 aa
RSHAE 7 aa
RSHA 6 aa
SHA 5 aa
SH 4 aa
0 25 50 75 100
e
random seq.
IPRYPHE teR1
IPRYKHE teR1(P-1K)
LNGDTHWSGPG teR2
LNGDTHPSGPG teR2(W+1P)
SLFDASEHYIANYS teR3
SLFDASSHYIANYS teR3(E-1S)
YLLYGSSQFWHIPQ teR4
YLLYGSSQFAHIPQ teR4(W-1A)
YLIVPPHPYPRE teR5
YLIVPPHAYPRE teR5(P+1A)
0 25 50 75 100

Fig. 7f

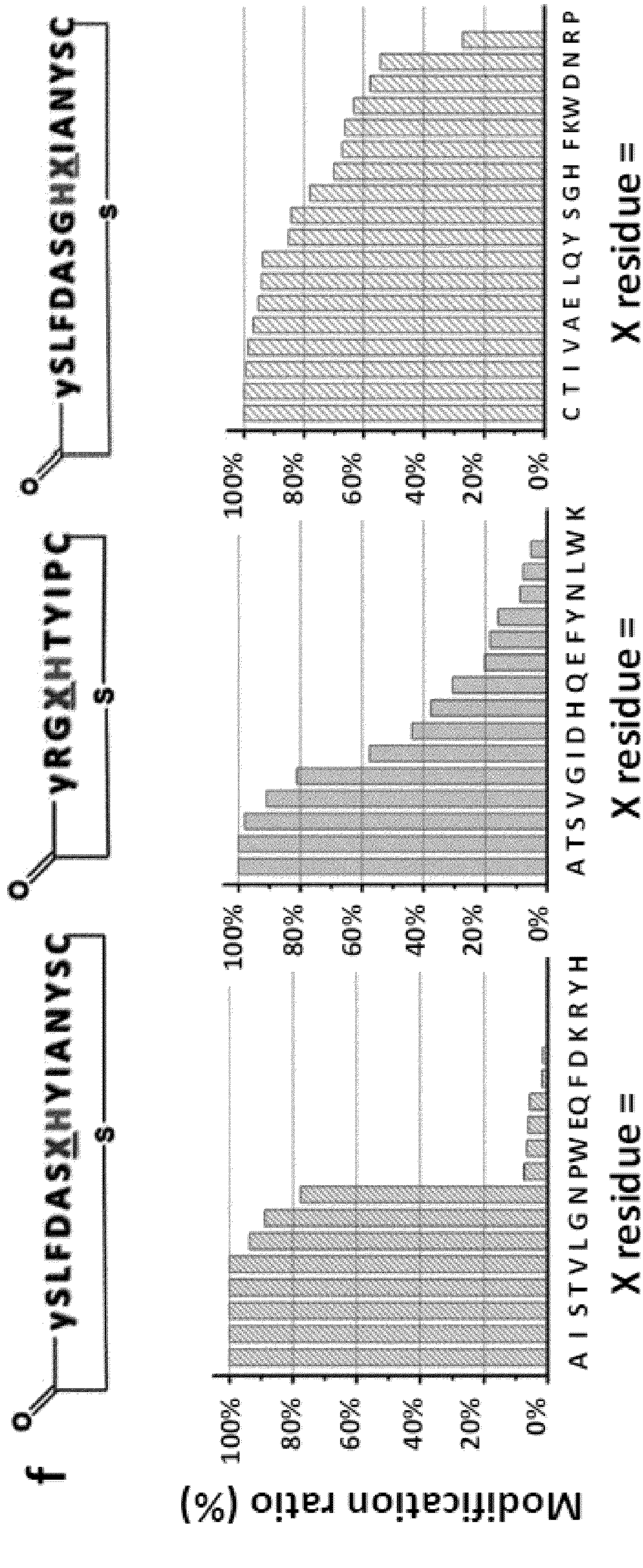
f
ySLFDASXHYIANYSC
S
yRGXHTYIPC
S
ySLFDASGHXIANYSC
S
Modification ratio (%)
100%
80%
60%
40%
20%
0%
A I S T V L G N P W E Q F D K R Y H
X residue =
A T S V G I D H Q E F Y N L W K
X residue =
C T I V A E L Q Y S G H F K W D N R P
X residue =

[FIG. 8]

Fig. 8a

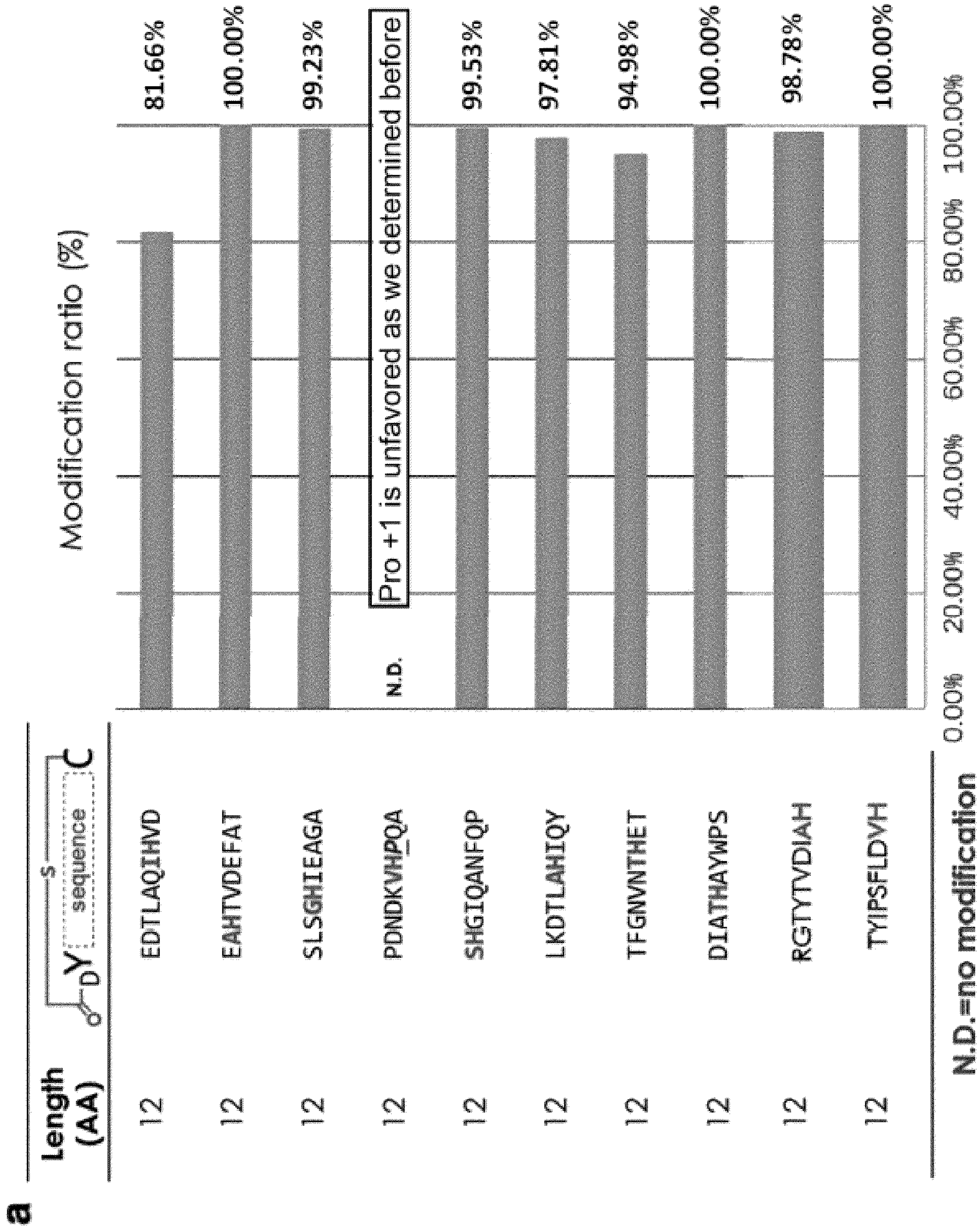
a
Length (AA)
sequence
Modification ratio (%)
Pro +1 is unfavored as we determined before
12 EDTLAQIHVD 81.66%
12 EAHTVDEFAT 100.00%
12 SLSGHIEAGA 99.23%
12 PDNDKVHPQA N.D.
12 SHGIQANFQP 99.53%
12 LKDTLAHIQY 97.81%
12 TFGNVNTHET 94.98%
12 DIATHAYWPS 100.00%
12 RGTYTVDIAH 98.78%
12 TYIPSFLDVH 100.00%
0.00% 20.00% 40.00% 60.00% 80.00% 100.00%
N.D.=no modification

Fig. 8b

**b**

| 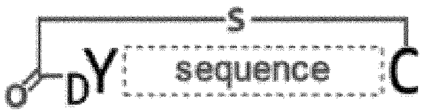 | % of modification | | | | Number of His |
|---|---|---|---|---|---|
| | plus 0 geranyl | plus 1 geranyl | plus 2 geranyl | plus 3 geranyl | |
| RYPHTGHYIP | N/D | 74.56% | 25.44% | N/A | 2 |
| RYPHTAGHYIP | N/D | 92.19% | 7.81% | N/A | 2 |
| RYPHTAAGHYIP | N/D | 82.56% | 17.44% | N/A | 2 |
| RYPHTAGAGHYIP | N/D | 78.41% | 21.59% | N/A | 2 |
| RYPHTAHAGHYIP | N/D | 4.43% | 95.57% | N/D | 3 |

LimF = 20 μM, GPP = 1 mM

Fig. 8c

C

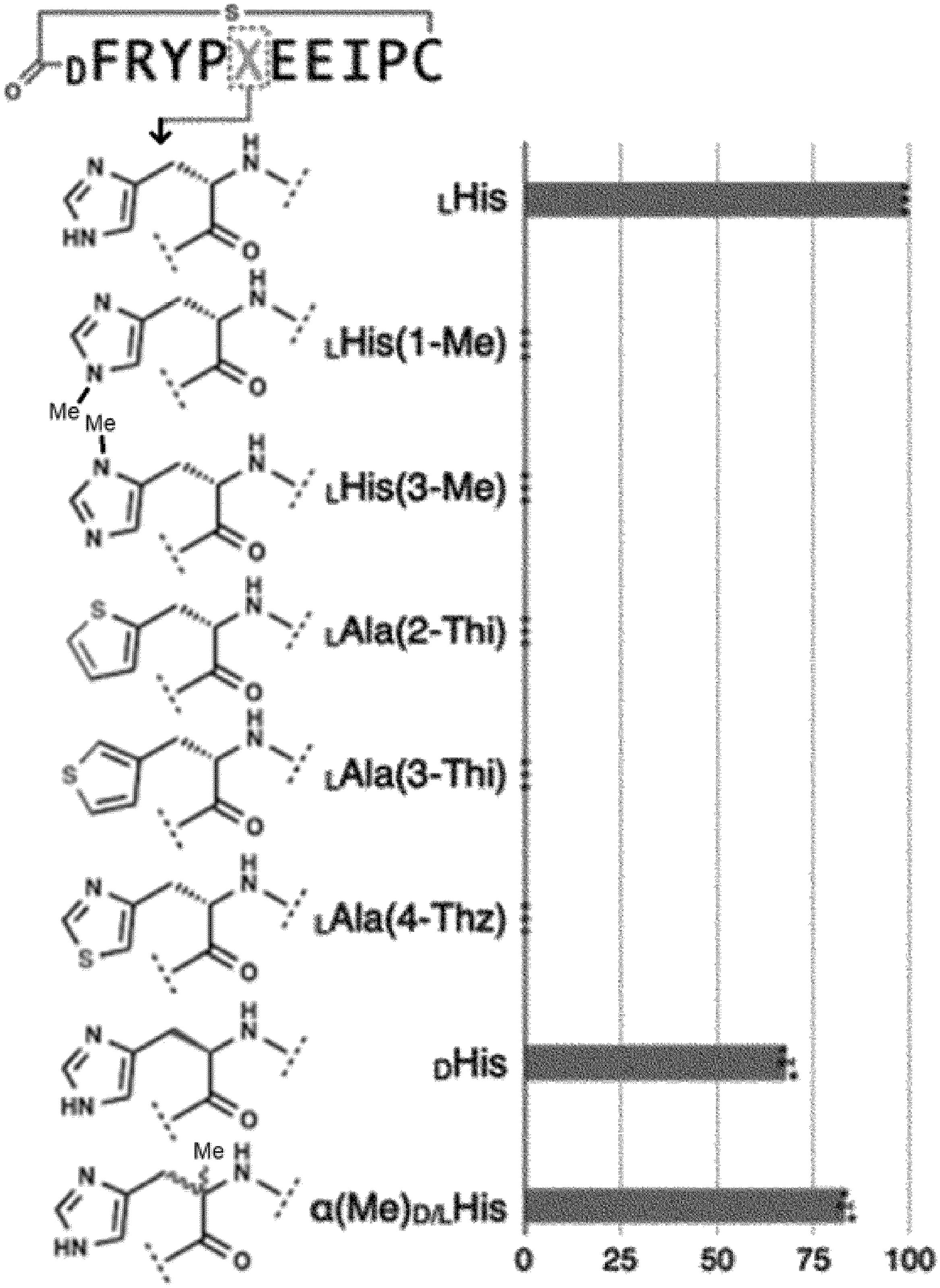
DFRYPXEEIPC
LHis
LHis(1-Me)
LHis(3-Me)
LAla(2-Thi)
LAla(3-Thi)
LAla(4-Thz)
DHis
α(Me)D/LHis
0
25
50
75
100

[FIG. 9]

Fig. 9a

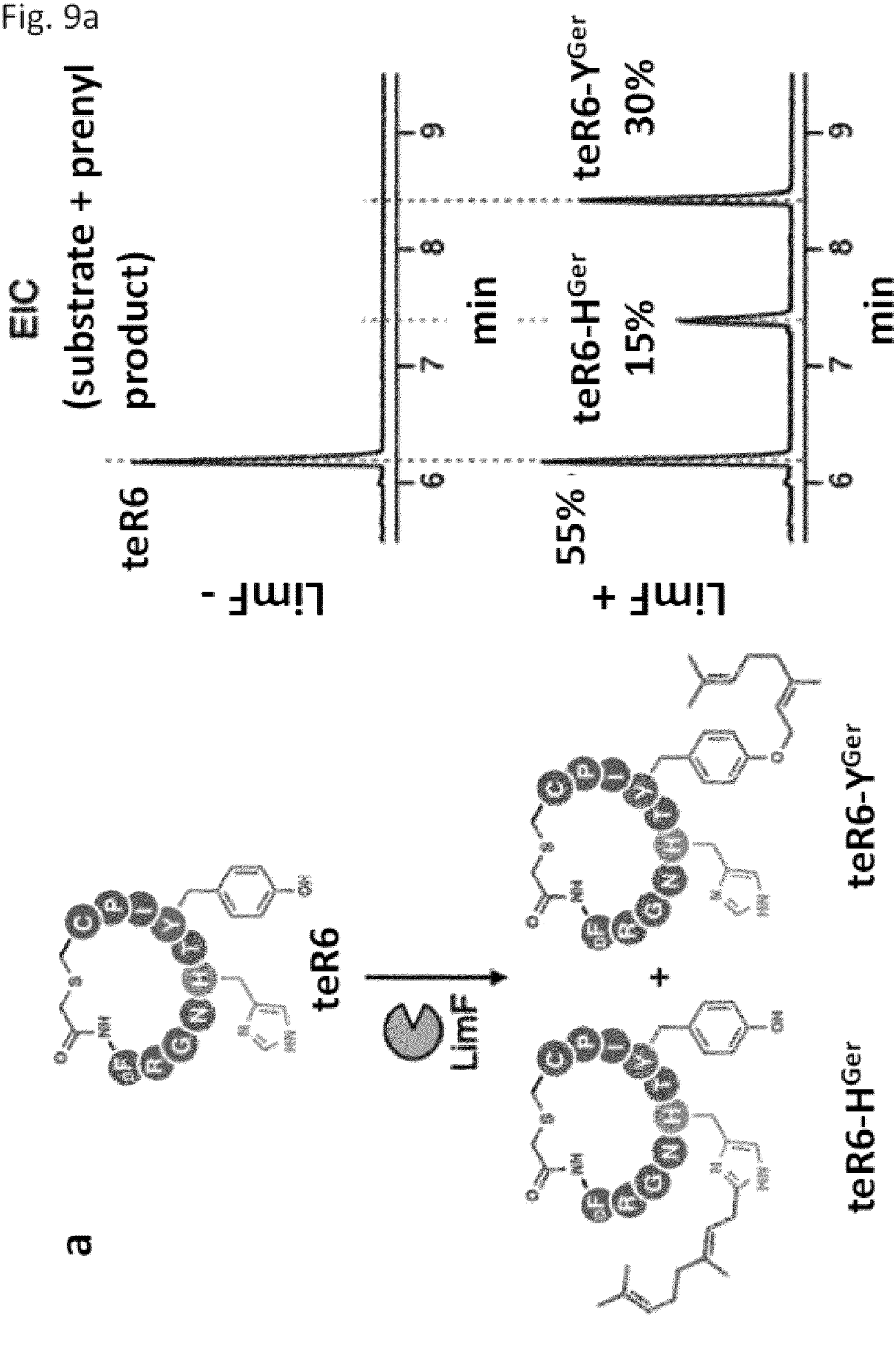
a
teR6
LimF
teR6-H$^{Ger}$
teR6-Y$^{Ger}$
EIC
(substrate + prenyl
product)
LimF -
LimF +
teR6
55%
teR6-H$^{Ger}$
15%
teR6-Y$^{Ger}$
30%
min
min

Fig. 9b

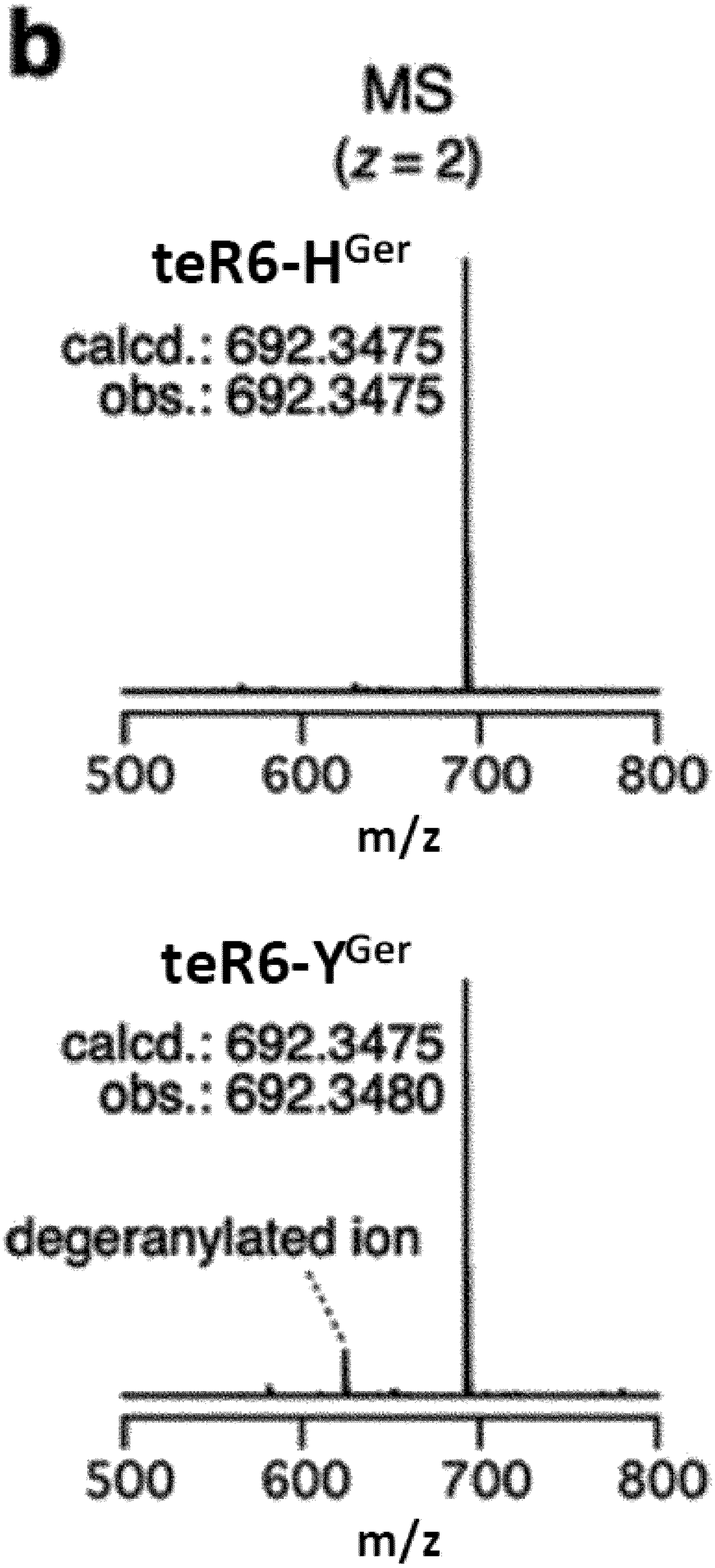
b
MS
(z = 2)
teR6-H^Ger
calcd.: 692.3475
obs.: 692.3475
500
600
700
800
m/z
teR6-Y^Ger
calcd.: 692.3475
obs.: 692.3480
degeranylated ion
500
600
700
800
m/z

Fig. 9c-d

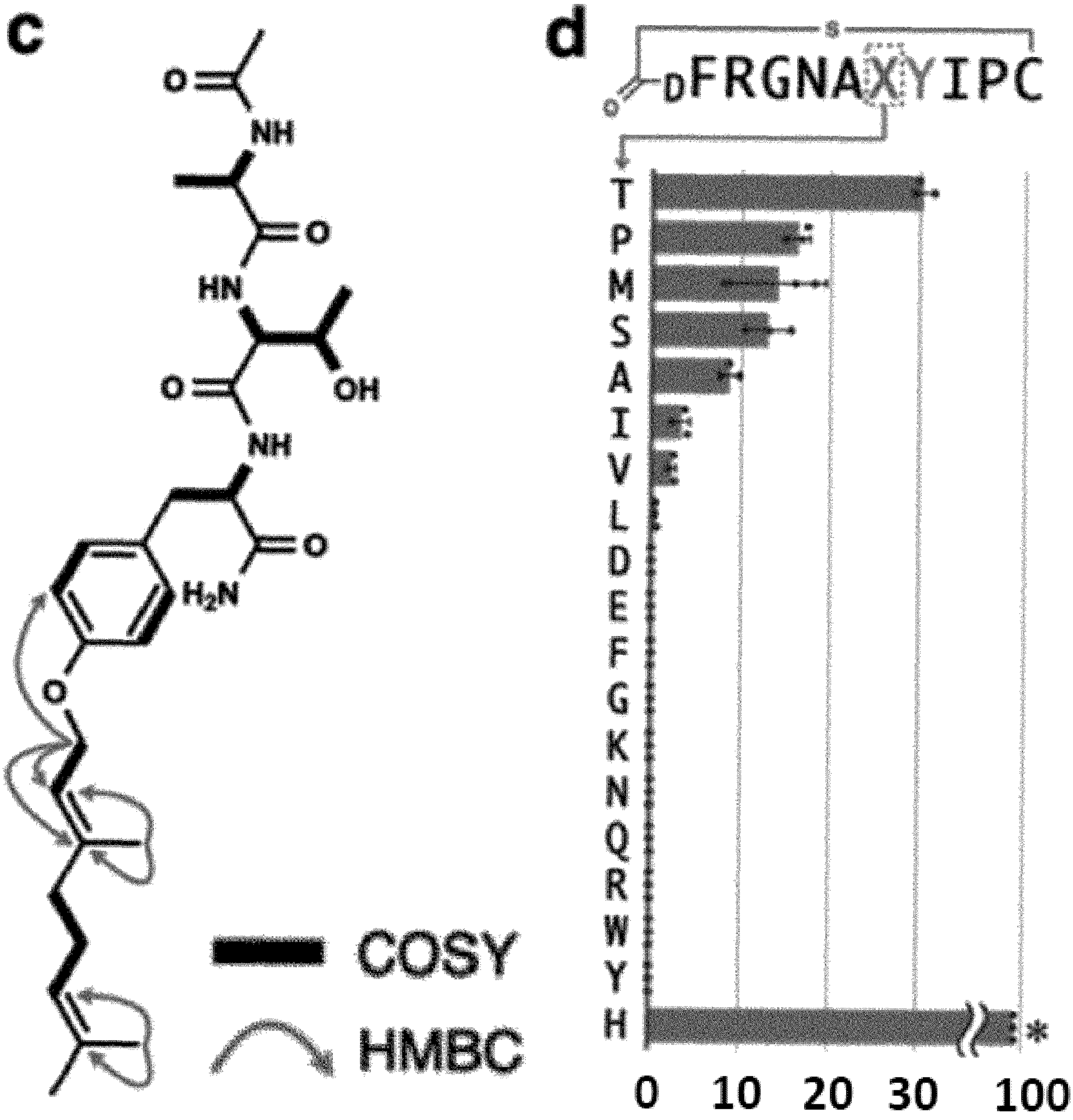
c
COSY
HMBC
d
DFRGNAXYIPC
T
P
M
S
A
I
V
L
D
E
F
G
K
N
Q
R
W
Y
H
0
10
20
30
100

[FIG. 10]

Fig. 10a-c

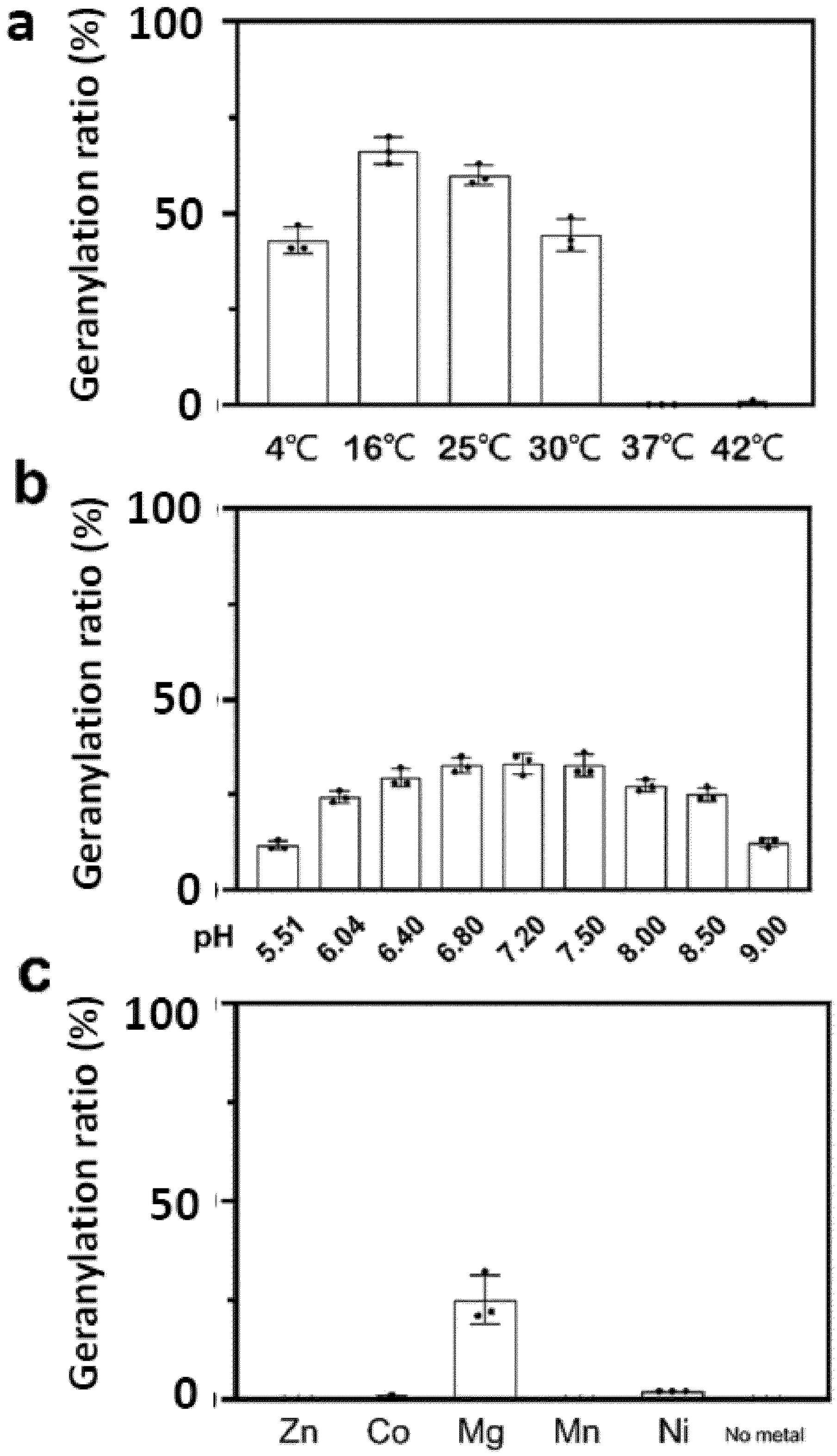

Fig. 10d-f

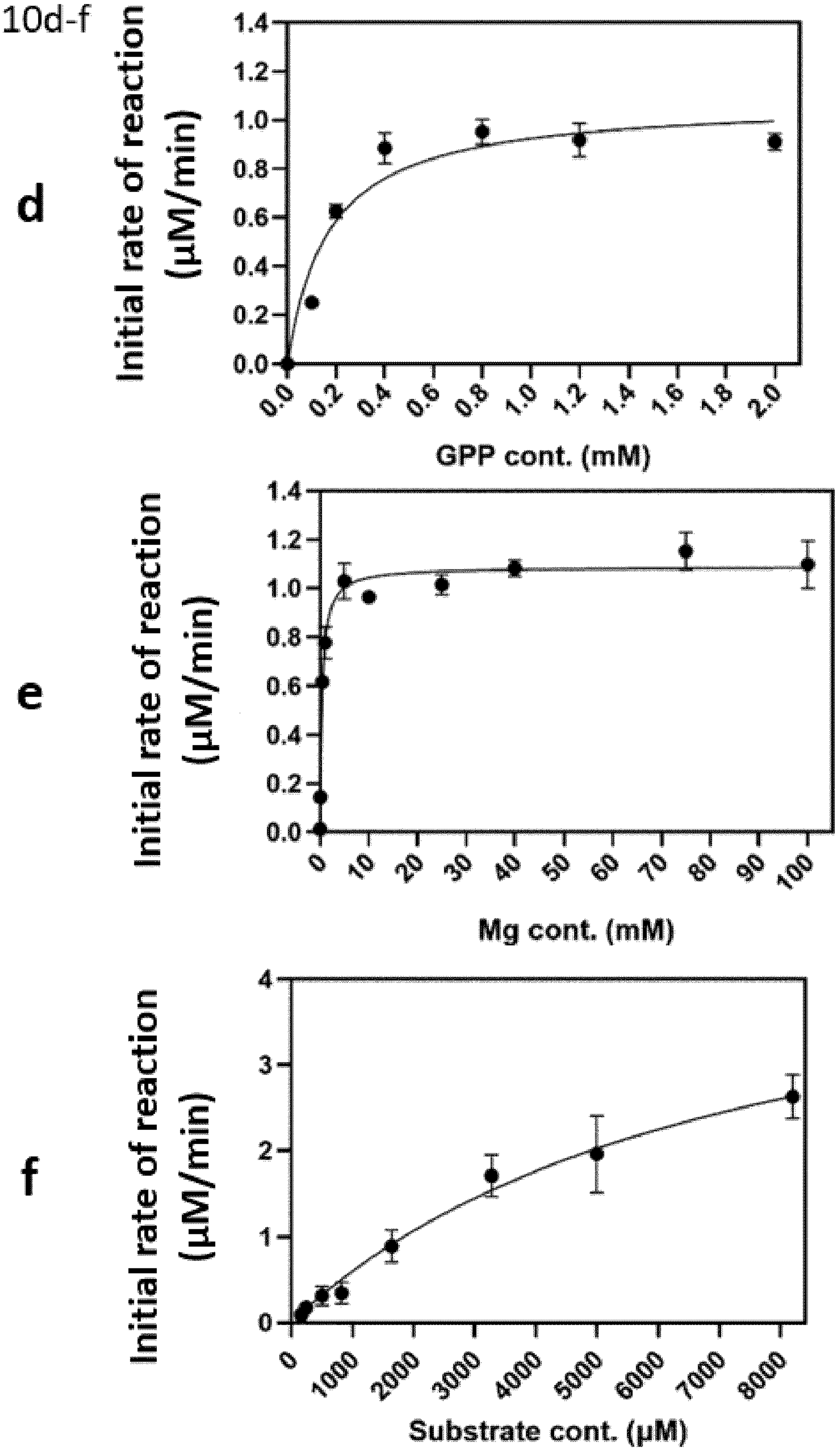
d
Initial rate of reaction (µM/min)
1.4
1.2
1.0
0.8
0.6
0.4
0.2
0.0
0.0 0.2 0.4 0.6 0.8 1.0 1.2 1.4 1.6 1.8 2.0
GPP cont. (mM)
e
Initial rate of reaction (µM/min)
1.4
1.2
1.0
0.8
0.6
0.4
0.2
0.0
0 10 20 30 40 50 60 70 80 90 100
Mg cont. (mM)
f
Initial rate of reaction (µM/min)
4
3
2
1
0
0 1000 2000 3000 4000 5000 6000 7000 8000
Substrate cont. (µM)

[FIG. 11]

| enzyme \ substrate | bcLimE2 $His^{Ger}$ | teR6 $His^{Ger}$ | teR6 $Try^{Ger}$ | teR6-H5A $Tyr^{Ger}$ |
|---|---|---|---|---|
| LimF (wild type) | >99% | 19% | 35% | 50% |
| LimF-E54A | <1% | <1% | <1% | <1% |
| LimF-E54Q | <1% | <1% | <1% | 2% |
| LimF-D70A | 91% | <1% | 65% | 68% |
| LimF-H172A | <1% | <1% | 4% | 7% |
| LimF-H172F | <1% | <1% | 5% | 22% |
| LimF-H172L | 2% | <1% | 80% | 87% |

[FIG. 12]

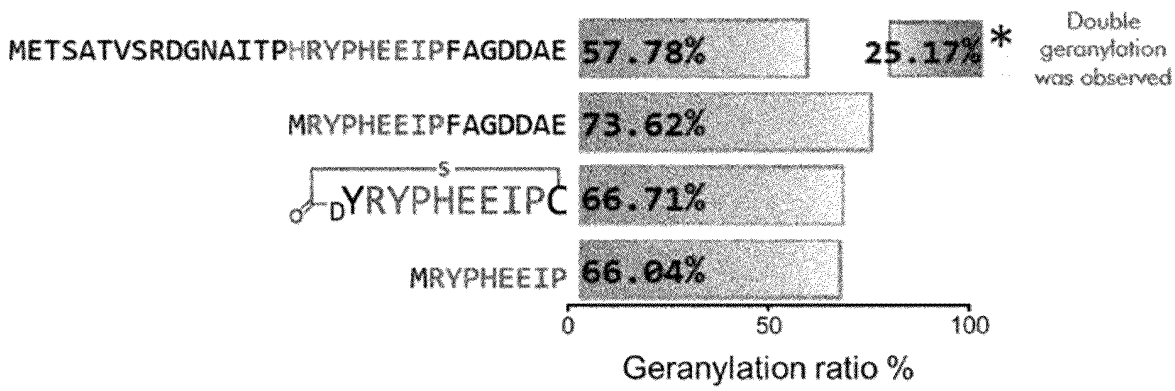

METSATVSRDGNAITPHRYPHEEIPFAGDDAE
57.78%
25.17%
*
Double geranylation was observed
MRYPHEEIPFAGDDAE
73.62%
S
DYRYPHEEIPC
66.71%
MRYPHEEIP
66.04%
0
50
100
Geranylation ratio %


[FIG. 13]

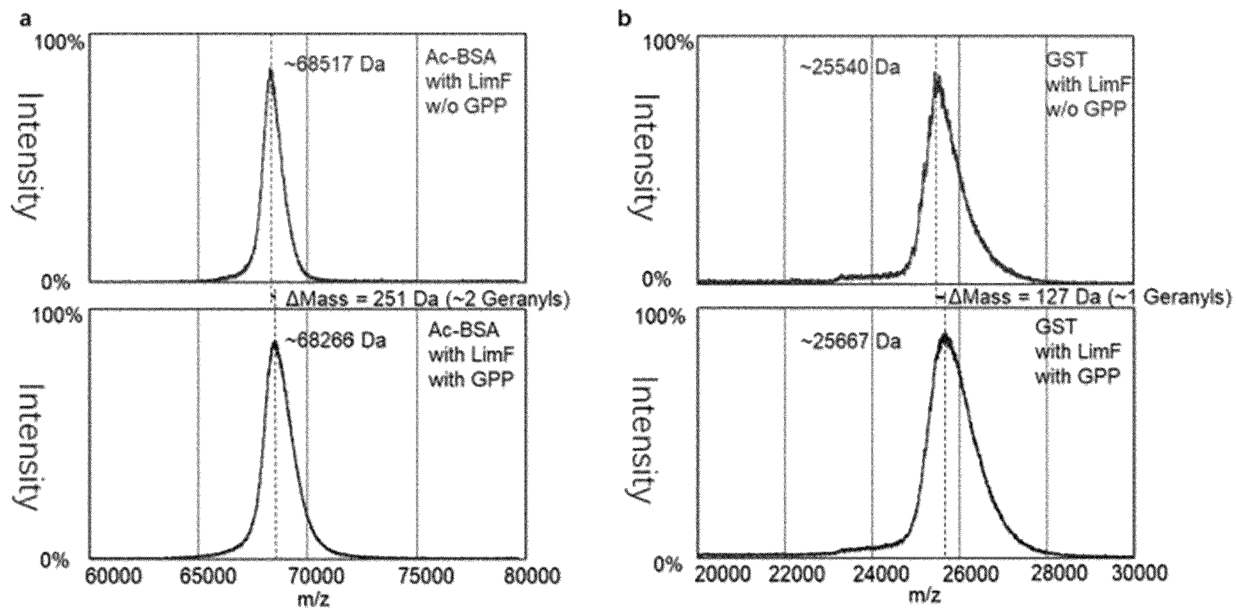

a
100%
Intensity
0%
~68517 Da
Ac-BSA with LimF w/o GPP
ΔMass = 251 Da (~2 Geranyls)
~68266 Da
Ac-BSA with LimF with GPP
60000
65000
70000
75000
80000
m/z
b
~25540 Da
GST with LimF w/o GPP
ΔMass = 127 Da (~1 Geranyls)
~25667 Da
GST with LimF with GPP
20000
22000
24000
26000
28000
30000
m/z

[FIG. 14-1]

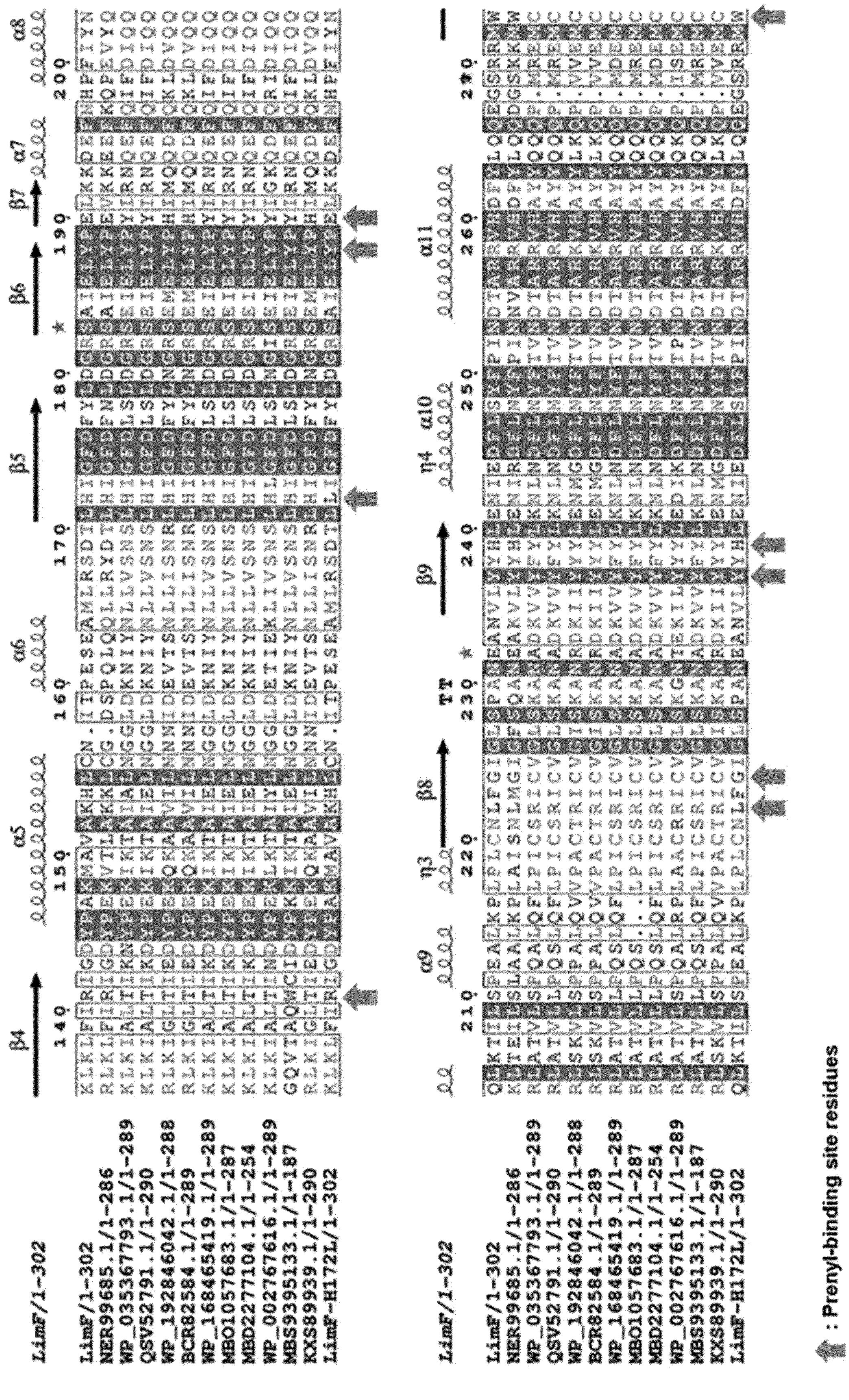
LimF/1-302
NER99685.1/1-286
WP_035367793.1/1-289
QSV52791.1/1-290
WP_192846042.1/1-288
BCR82584.1/1-289
WP_168465419.1/1-289
MBO1057683.1/1-287
MBD2277104.1/1-254
WP_002767616.1/1-289
MBS9395133.1/1-187
KXS89939.1/1-290
LimF-H172L/1-302
: Prenyl-binding site residues

[FIG. 14-2]

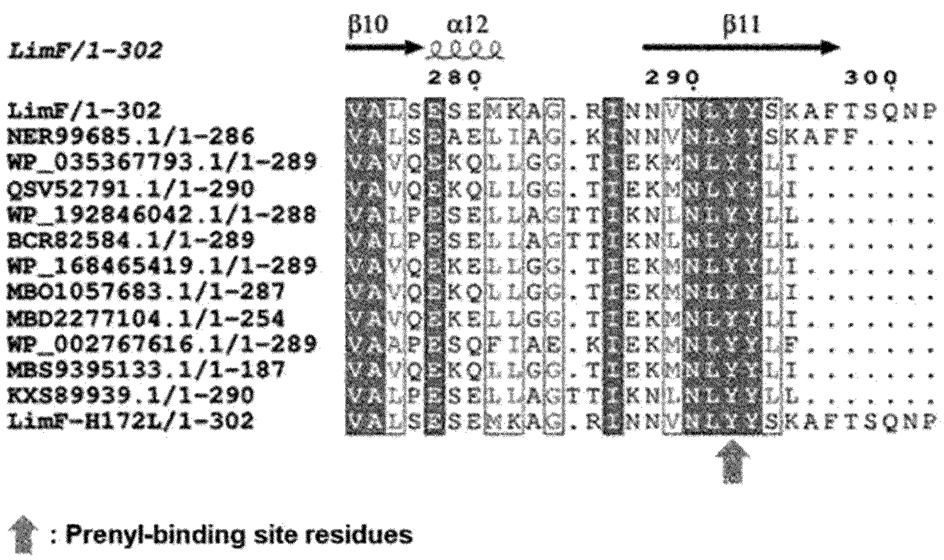
β10
α12
β11
LimF/1-302
280
290
300
LimF/1-302
NER99685.1/1-286
WP_035367793.1/1-289
QSV52791.1/1-290
WP_192846042.1/1-288
BCR82584.1/1-289
WP_168465419.1/1-289
MBO1057683.1/1-287
MBD2277104.1/1-254
WP_002767616.1/1-289
MBS9395133.1/1-187
KXS89939.1/1-290
LimF-H172L/1-302
: Prenyl-binding site residues

[FIG. 15]

Fig. 15(a)

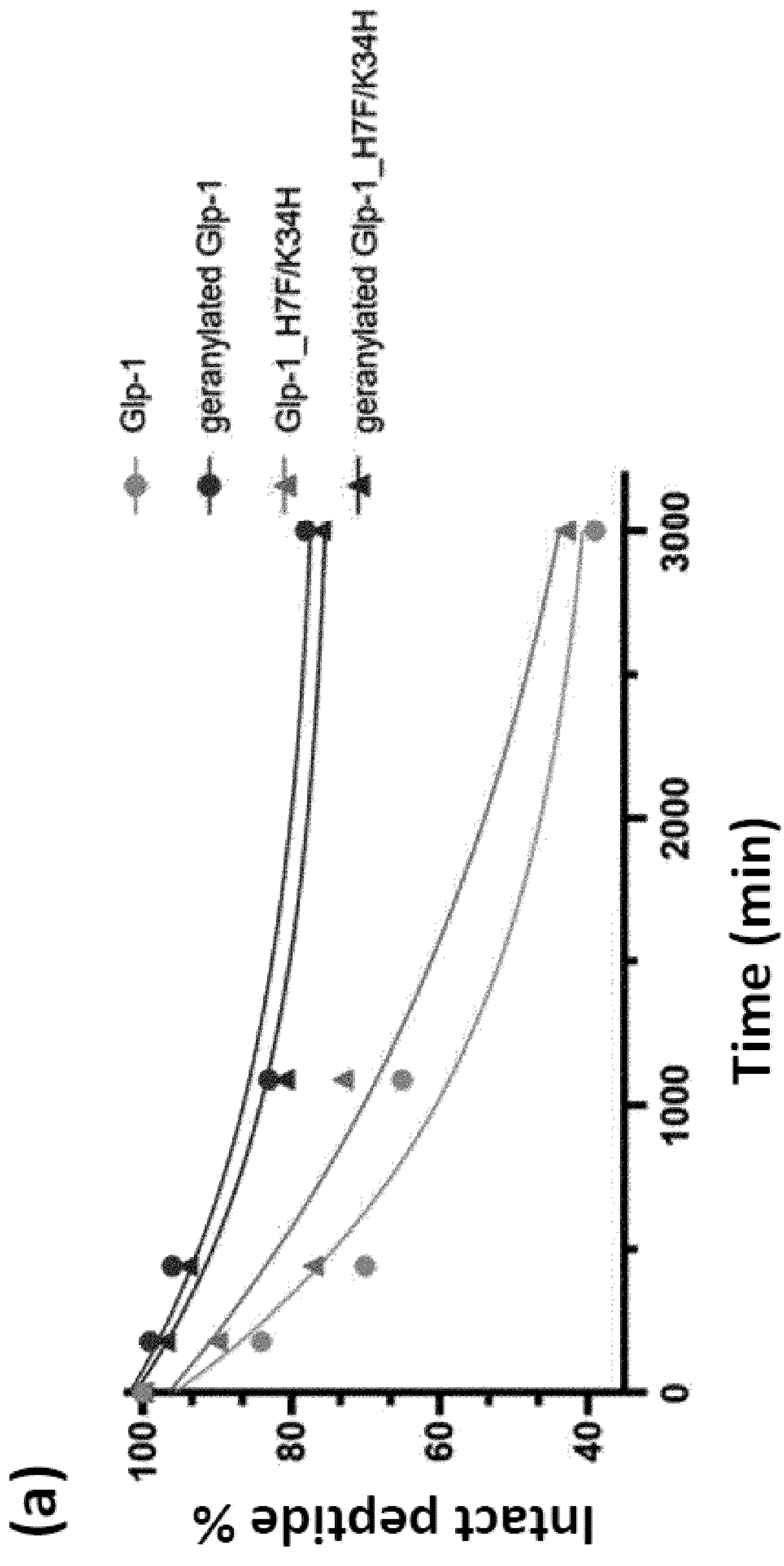

Fig. 15(b)

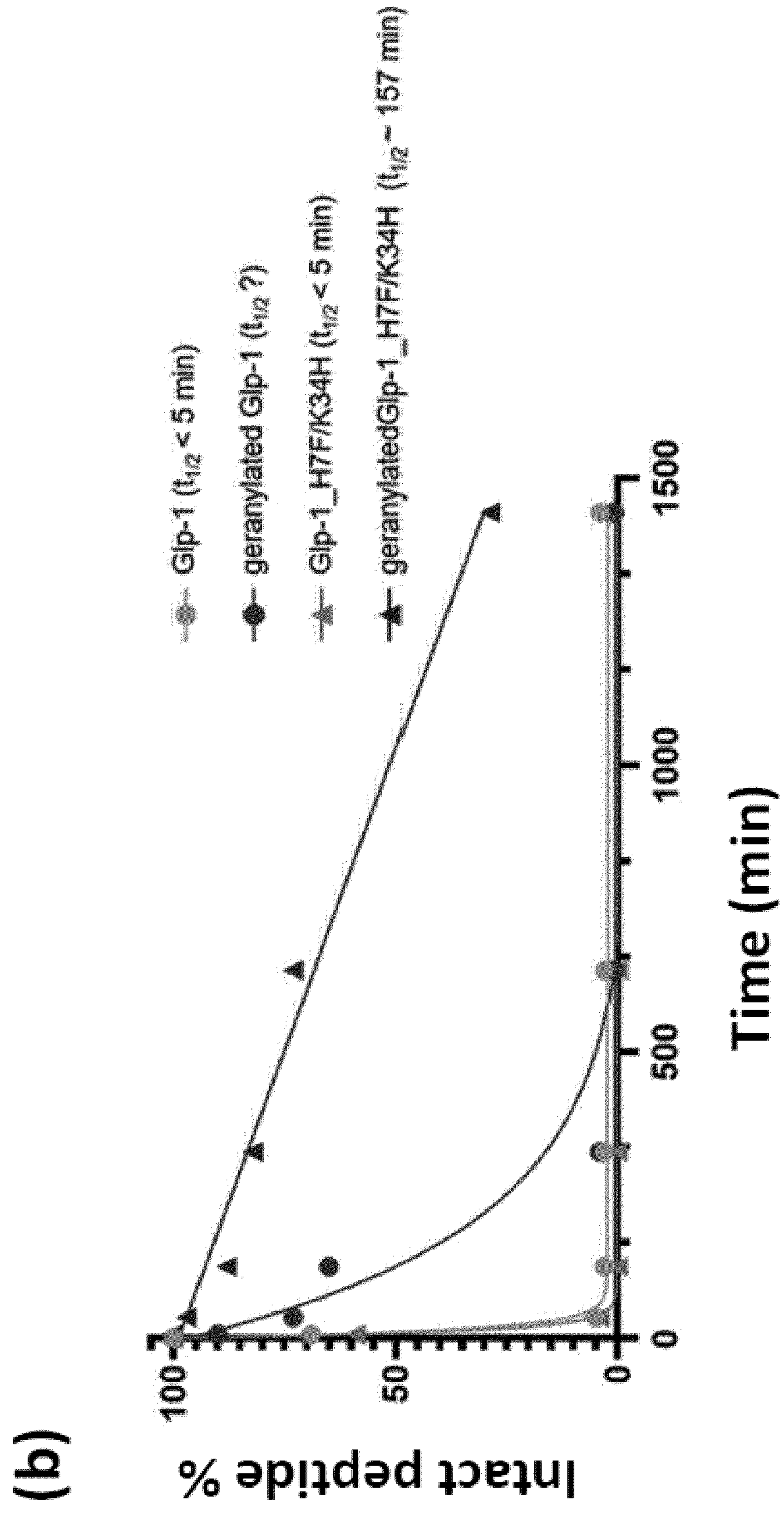
(b)
Intact peptide %
100
50
0
0
500
1000
1500
Time (min)
Glp-1 ($t_{1/2}$ < 5 min)
geranylated Glp-1 ($t_{1/2}$ ?)
Glp-1_H7F/K34H ($t_{1/2}$ < 5 min)
geranylatedGlp-1_H7F/K34H ($t_{1/2}$ ~ 157 min)

Fig. 15(c)

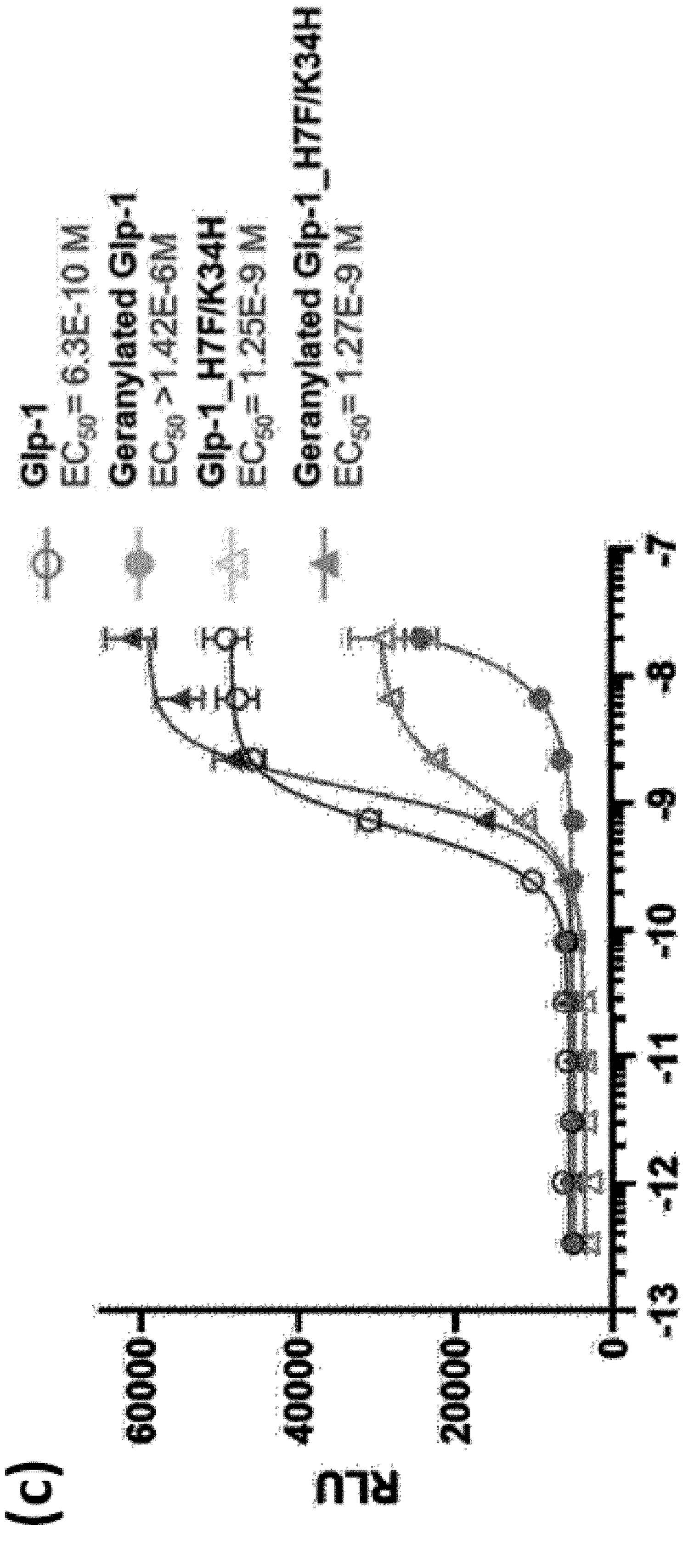
(c)
RLU
60000
40000
20000
0
-13
-12
-11
-10
-9
-8
-7
log conc. (M)
Glp-1
EC$_{50}$= 6.3E-10 M
Geranylated Glp-1
EC$_{50}$ >1.42E-6M
Glp-1_H7F/K34H
EC$_{50}$= 1.25E-9 M
Geranylated Glp-1_H7F/K34H
EC$_{50}$= 1.27E-9 M

[FIG. 16]

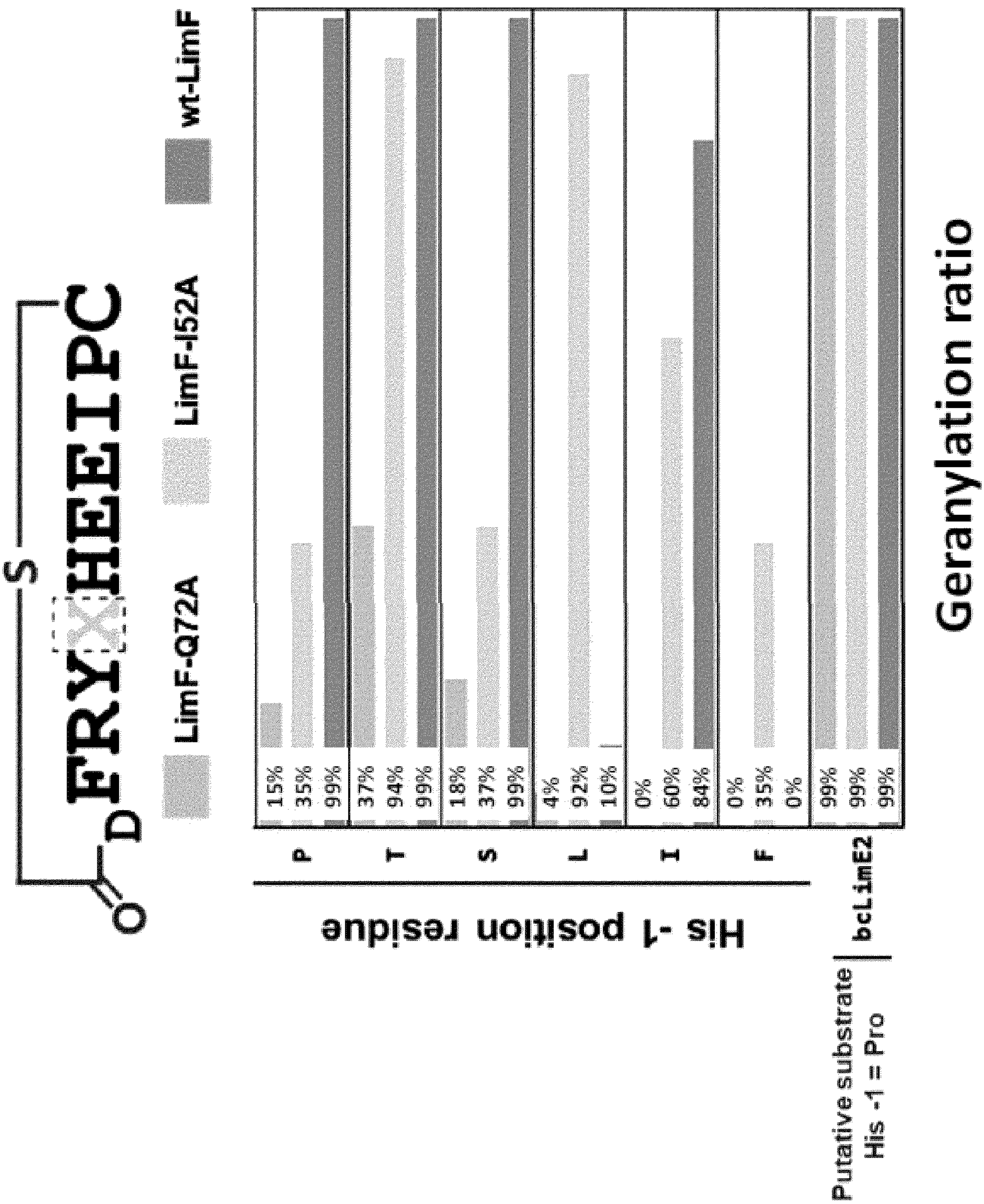
LimF-Q72A
LimF-I52A
wt-LimF
FRYXHEEIPC
Geranylation ratio
His -1 position residue
P
15%
35%
99%
T
37%
94%
99%
S
18%
37%
99%
L
4%
92%
10%
I
0%
60%
84%
F
0%
35%
0%
bcLimE2
99%
99%
99%
Putative substrate
His -1 = Pro

[FIG. 17]

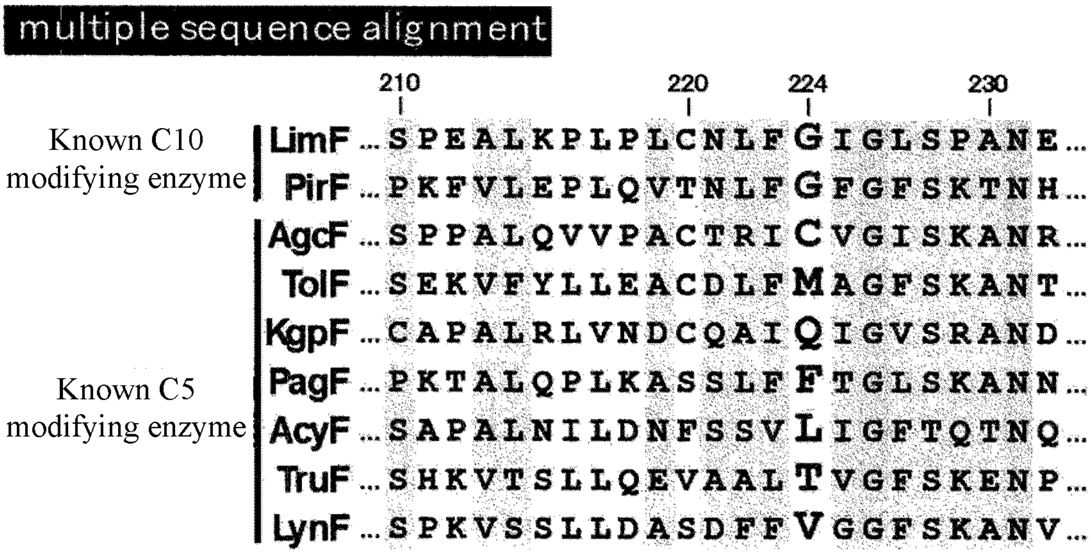
multiple sequence alignment
210
220
224
230
Known C10 modifying enzyme
LimF ... S P E A L K P L P L C N L F G I G L S P A N E ...
PirF ... P K F V L E P L Q V T N L F G F G F S K T N H ...
AgcF ... S P P A L Q V V P A C T R I C V G I S K A N R ...
TolF ... S E K V F Y L L E A C D L F M A G F S K A N T ...
KgpF ... C A P A L R L V N D C Q A I Q I G V S R A N D ...
Known C5 modifying enzyme
PagF ... P K T A L Q P L K A S S L F F T G L S K A N N ...
AcyF ... S A P A L N I L D N F S S V L I G F T Q T N Q ...
TruF ... S H K V T S L L Q E V A A L T V G F S K E N P ...
LynF ... S P K V S S L L D A S D F F V G G F S K A N V ...

[FIG. 18]

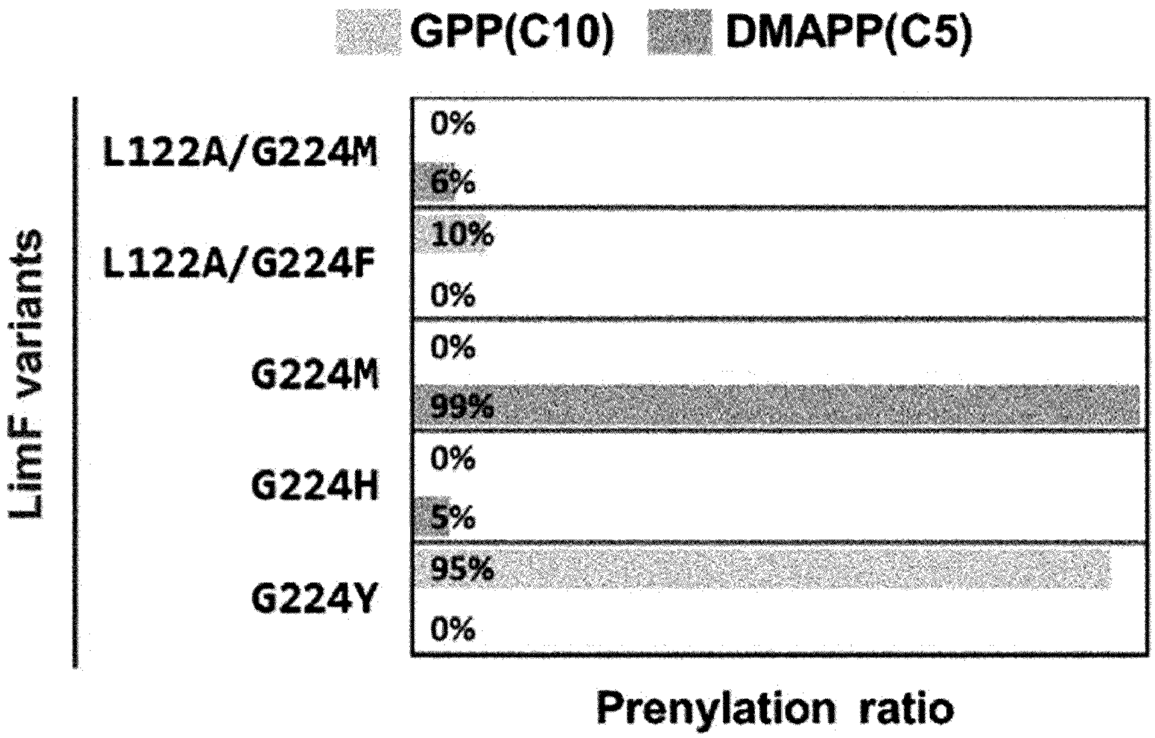
GPP(C10)
DMAPP(C5)
LimF variants
L122A/G224M
0%
6%
L122A/G224F
10%
0%
G224M
0%
99%
G224H
0%
5%
G224Y
95%
0%
Prenylation ratio

[FIG. 19]

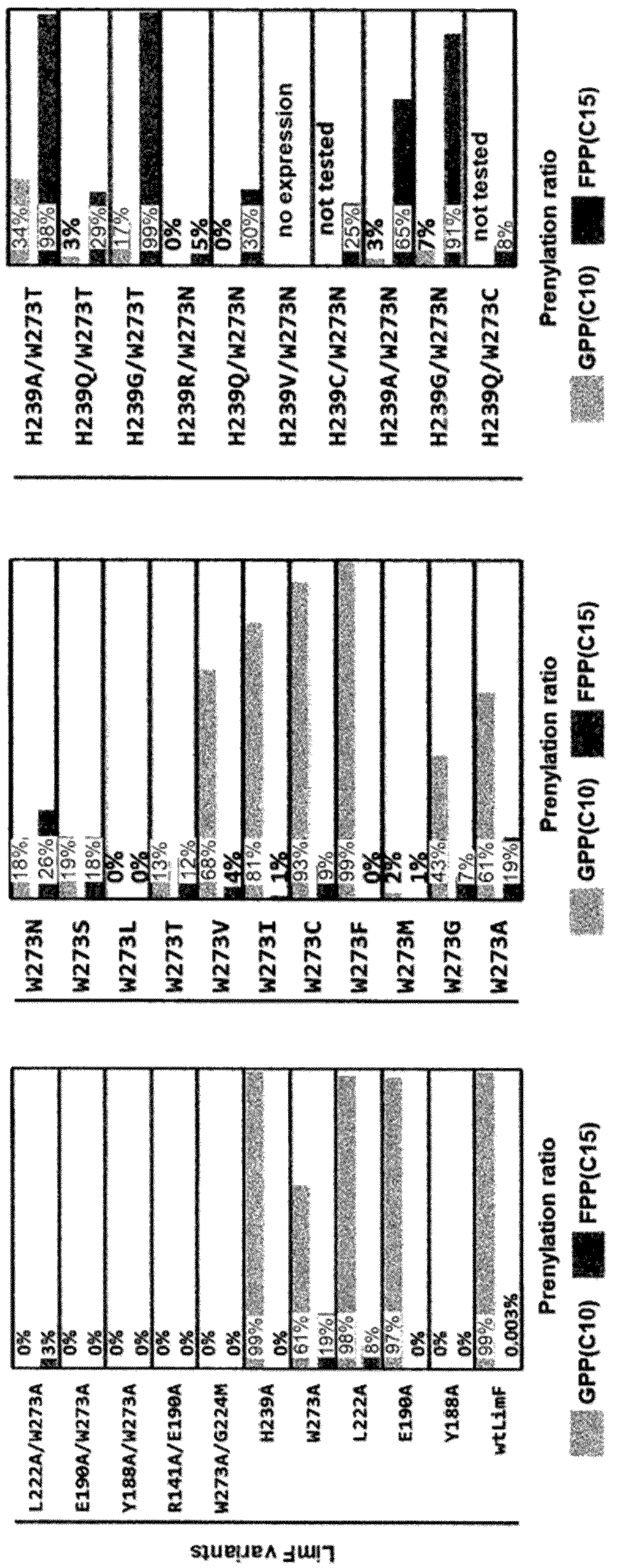
LimF variants
L222A/W273A
E190A/W273A
Y188A/W273A
R141A/E190A
W273A/G224M
H239A
W273A
L222A
E190A
Y188A
wtLimF
W273N
W273S
W273L
W273T
W273V
W273I
W273C
W273F
W273M
W273G
W273A
H239A/W273T
H239Q/W273T
H239G/W273T
H239R/W273N
H239Q/W273N
H239V/W273N
H239C/W273N
H239A/W273N
H239G/W273N
H239Q/W273C
no expression
not tested
not tested
Prenylation ratio
GPP(C10)
FPP(C15)

[FIG. 20]

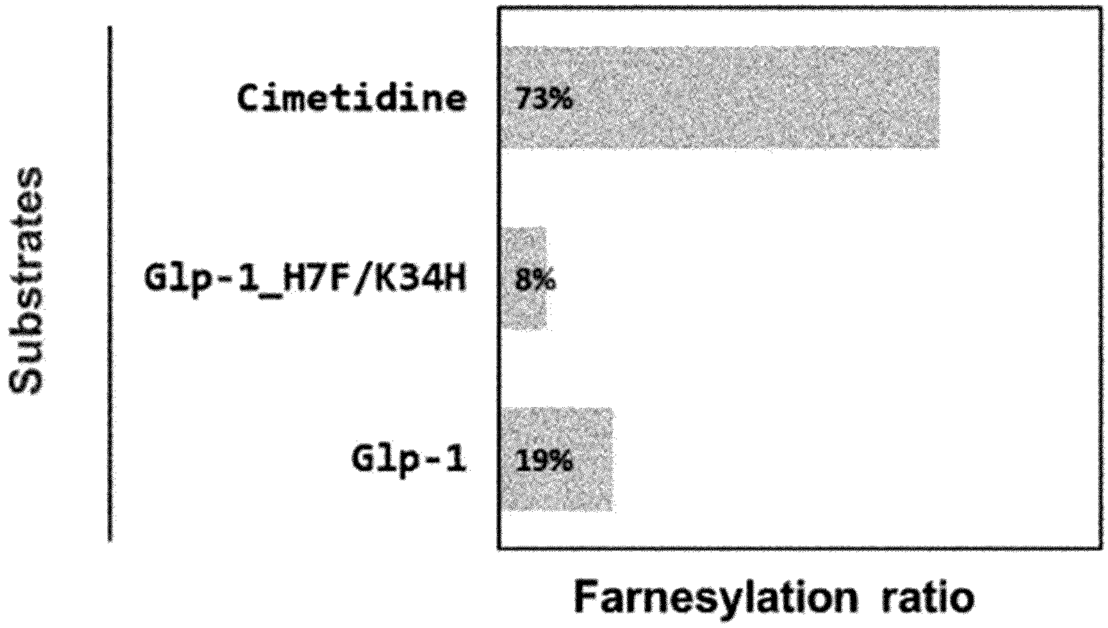
Substrates
Cimetidine
73%
Glp-1_H7F/K34H
8%
Glp-1
19%
Farnesylation ratio

**INTERNATIONAL SEARCH REPORT**

International application No.
**PCT/JP2022/038924**

**A. CLASSIFICATION OF SUBJECT MATTER**

***C07K 1/113***(2006.01)i; ***C40B 40/08***(2006.01)i; ***C40B 40/10***(2006.01)i; ***C12N 15/11***(2006.01)i; ***C12P 21/02***(2006.01)i; ***C12N 9/10***(2006.01)i

FI: C07K1/113 ZNA; C40B40/10; C12P21/02 C; C40B40/08; C12N9/10; C12N15/11 Z

According to International Patent Classification (IPC) or to both national classification and IPC

**B. FIELDS SEARCHED**

Minimum documentation searched (classification system followed by classification symbols)

C07K1/113; C40B40/08; C40B40/10; C12N15/11; C12N9/10; C12P21/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | HAO, Yue et al. Molecular basis for the broad substrate selectivity of a peptide prenyltransferase. Proceedings of the National Academy of Sciences of the United States of America. 2016, vol. 113, no. 49, pp. 14037-14042, doi:10.1073/pnas.1609869113 abstract, results, fig. 1, 6, table 1 | 1-2, 10-12 |
| Y | | 1-14 |
| X | MORITA, Maho et al. Post-Translational Tyrosine Geranylation in Cyanobactin Biosynthesis. Journal of the American Chemical Society. 2018, vol. 140, pp. 6044-6048, doi:10.1021/jacs.8b03137 abstract, table 1, fig. 1 | 1-2, 10-12 |
| Y | | 1-14 |
| X | BANDARI, Chandrasekhar et al. Determination of Alkyl-Donor Promiscuity of Tyrosine-O-Prenyltransferase SirD from Leptosphaeria maculans. ChemBioChem. 2017, vol. 18, pp. 2323-2327, doi:10.1002/cbic.201700469 abstract, scheme 1 | 1 |
| Y | | 1-9, 11-14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 December 2022** | **27 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/038924**

**C. DOCUMENTS CONSIDERED TO BE RELEVANT**

| Category* | Citation of document, with indication, where appropriate, of the relevant passages | Relevant to claim No. |
|---|---|---|
| Y | WO 2020/080490 A1 (THE UNIVERSITY OF TOKYO) 23 April 2020 (2020-04-23) claims, etc. | 1-14 |
| Y | FAN, Aili et al. A new member of the DMATS superfamily from Aspergillus niger catalyzes prenylations of both tyrosine and tryptophan derivatives. Appl. Microbial. Biotechnol. 2014, vol. 98, pp. 10119-10129, doi:10.1007/s00253-014-5872-7 abstract, discussion | 1-14 |
| Y | FAN, Aili et al. Prenylation of tyrosine and derivatives by a tryptophan C7-prenyltransferase. Tetrahedron Letters. 2014, vol. 55, pp. 5199-5202, doi:10.1016/j.tetlet.2014.07.080 abstract, table 1 | 1-14 |

Form PCT/ISA/210 (second sheet) (January 2015)

**INTERNATIONAL SEARCH REPORT**

International application No.
**PCT/JP2022/038924**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

The “form of an Annex C/ST.25 text file” is replaced with the “form of ST.26.”

**INTERNATIONAL SEARCH REPORT**
**Information on patent family members**

International application No.
**PCT/JP2022/038924**

| Patent document cited in search report | Publication date (day/month/year) | Patent family member(s) | Publication date (day/month/year) |
|---|---|---|---|
| WO 2020/080490 A1 | 23 April 2020 | EP 3879013 A1<br>claims | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020080490 A
- WO 2015030014 A
- WO 2008117833 A
- WO 2012074129 A


### Non-patent literature cited in the description


- **HAO, Y et al.** Molecular basis for the broad substrate selectivity of a peptide prenyltransferase. *Proc. Natl. Acad. Sci. USA,* 2016, vol. 113, 14037-14042
- **DALPONTE, L. et al.** N-Prenylation of tryptophan by an aromatic prenyltransferase from the cyanobactin biosynthetic pathway. *Biochemistry,* 2018, vol. 57, 6860-6867
- **TIANERO, M.D. et al.** Metabolic model for diversity-generating biosynthesis. *Proc. Natl. Acad. Sci. USA,* 2016, vol. 113, 1772-1777
- **PHAN, C.-S. et al.** Argicyclamides A-C Unveil Enzymatic Basis for Guanidine Bis-prenylation. *J. Am. Chem. Soc.,* 2021, vol. 143, 10083-10087
- **PARAJULI, A. et al.** A Unique Tryptophan C-Prenyltransferase from the Kawaguchipeptin Biosynthetic Pathway. *Angew. Chem.,* 2016, vol. 55, 3596-3599
- **OKADA, M. et al.** Stereospecific prenylation of tryptophan by a cyanobacterial post-translational modification enzyme. *Org. Biomol. Chem.,* 2016, vol. 14, 9639-9644
- **T. W. GREENE ; P. G. M. WUTS.** Protective Groups In Organic Synthesis. John Wiley&Sons, Inc,
- **H. MURAKAMI ; H. SAITO ; H. SUGA.** *Chem. Biol.,* 2003, vol. 10, 655-662
- **KAWAKAMI, T. et al.** *Nat. Chem. Biol.,* 2009, vol. 5, 888-890
- **YAMAGISHI, Y et al.** *ChemBioChem,* 2009, vol. 10, 1469-1472
- **SAKO, Y et al.** *J. Am. Chem. Soc.,* 2008, vol. 130, 7932-7934
- **GOTO, Y et al.** *ACS Chem. Biol.,* 2008, vol. 3, 120-129
- **KAWAKAMI T. et al.** *Chem. Biol.,* 2008, vol. 15, 32-42
- **YAMAGISHI, Y et al.** *Chembiochem,* 2009, vol. 10, 1469-1472
- **H. F. KUNG et al.** *The J. Biol. Chem.,* 1977, vol. 252 (19), 6889-6894
- **M. C. GONZA et al.** *Proc. Natl. Acad. Sci. U.S.A.,* 1985, vol. 82, 1648-1652
- **M. Y PAVLOV ; M. EHRENBERG.** *Arch. Biochem. Biophys.,* 1996, vol. 328 (1), 9-16
- **Y SHIMIZU et al.** *Nat. Biotechnol.,* 2001, vol. 19 (8), 751-755
- **H. OHASHI et al.** *Biochem. Biophys. Res. Commun,* 2007, vol. 352 (1), 270-276
- **YAMAGISHI, Y et al.** *Chem. Biol.,* 2011, vol. 18 (12), 1562-70
- **PASSIOURA, T. ; LIU, W. ; DUNKELMANN, D. ; HIGUCHI, T. ; SUGA, H.** Display selection of exotic macrocyclic peptides expressed under a radically reprogrammed 23 amino acid genetic code. *J. Am. Chem. Soc.,* 2018, vol. 140 (37), 11551-11555
- **GOTO, Y ; KATOH, T.** Flexizymes for genetic code reprogramming. *Suga, Nat. Protoc.,* 2011, vol. 6 (6), 779-790
- **GOTO, Y ; KATOH, T.** Flexizymes for genetic code reprogramming. *Suga, Nat. Protoc,* 2011, vol. 6 (6), 779-790
- **VINOGRADOV, A. A ; SHIMOMURA, M. ; GOTO, Y ; OZAKI, T. ; ASAMIZU, S. ; SUGAI, Y ; SUGA, H.** Minimal lactazole scaffold for in vitro thiopeptide bioengineering. *Onaka, Nat. Commun.,* 2020, vol. 11 (1), 1-13
- **ZALLOT, R ; OBERG, N.** Gerlt, The EFI web resource for genomic enzymology tools: leveraging protein, genome, and metagenome databases to discover novel enzymes and metabolic pathways. *Biochemistry,* 2019, vol. 58 (41), 4169-4182
- **GERLT, J. A ; BOUVIER, J. T ; DAVIDSON, D. B. ; IMKER, H. J. ; SADKHIN, B. ; SLATER, D. R ; WHALEN, K. L.** Enzyme function initiative-enzyme similarity tool (EFI-EST): a web tool for generating protein sequence similarity networks. *BBA. PROTEINS AND PROTEOMICS,* 2015, vol. 1854 (8), 1019-1037
- **KUMAR, S. ; STECHER, G.** Tamura, MEGA7: molecular evolutionary genetics analysis version 7.0 for bigger datasets. *Mol. Biol. E,* 2016, vol. 33 (7), 1870-1874
- **LIMA, A.R.J. et al.** Insights into Limnothrix sp. *Metabolism Based on Comparative Genomics. Front. Microbiol.,* 2018, vol. 9

- **DONIA, M.S. ; SCHMIDT.** Linking chemistry and genetics in the growing cyanobactin natural products family. *Chem. Biol.,* 2011, vol. 18, 508-519
- **SARKAR, S. ; GU, W. ; SCHMIDT.** Expanding the chemical space of synthetic cyclic peptides using a promiscuous macrocyclase from prenylagaramide biosynthesis. *ACS Catal.,* 2020, vol. 10, 7146-7153
- **HUANG, Y ; WIEDMANN, M.M. ; SUGA.** RNA display methods for the discovery of bioactive macrocycles. *Chem. Rev.,* 2018, vol. 119, 10360-10391
- **GOTO, Y et al.** Reprogramming the translation initiation for the synthesis of physiologically stable cyclic peptides. *ACS Chem. Biol.,* 2008, vol. 3, 120-129
- **GOTO, Y ; KATOH, T. ; SUGA.** Flexizymes for genetic code reprogramming. *Nat. Protoc.,* 2011, vol. 6, 779-790
- **MCINTOSH, J.A. ; DONIA, M.S. ; NAIR, S.K. ; SCHMIDT.** Enzymatic basis of ribosomal peptide prenylation in cyanobacteria. *J. Am. Chem. Soc.,* 2011, vol. 133, 13698-13705
- **GRUNDMANN, A. ; KUZNETSOVA, T. ; AFIYATULLOV, S. S. ; LI, S.-M.** FtmPT2, an N-Prenyltransferase from Aspergillus fumigatus, Catalyses the Last Step. *Biosynthesis of Fumitremorgin B. Chembiochem,* 2008, vol. 9 (13), 2059-2063
- **BAI, N ; LI, G.-H ; LUO, S.-L. ; DU, L. ; HU, Q.-Y ; XU, H.-K. ; ZHANG, K.-Q. ; ZHAO, P.-J.** Vib-PT, an aromatic prenyltransferase involved in the biosynthesis of vibralactone from Stereum vibrans. *Appl. Environ. Microbiol.,* 2020, vol. 86 (10), e02687-19
- **HAAGEN, Y ; UNSOLD, I ; WESTRICH, L. ; GUST, B. ; RICHARD, S. B. ; NOEL, J. P ; HEIDE, L.** A soluble, magnesium-independent prenyltransferase catalyzes reverse and regular C-prenylations and O-prenylations of aromatic substrates. *FEBS Lett.,* 2007, vol. 581 (16), 2889-2893
- **LIAO, S.-M. ; DU, Q.-S. ; MENG, J.-Z. ; PANG, Z.-W. ; HUANG.** The multiple roles of histidine in protein interactions. *Chem. Cent. J.,* 2013, vol. 7, 1-12
- **JIA, S. ; HE, D. ; CHANG, C.J.** Bioinspired Thiophosphorodichloridate Reagents for Chemoselective Histidine Bioconjugation. *Journal of the American Chemical Society,* 2019, vol. 141, 7294-7301
- **KEE, J.-M. ; MUIR, T.W.** Chasing Phosphohistidine, an Elusive Sibling in the Phosphoamino Acid Family. *ACS Chem. Biol.,* 2012, vol. 7, 44-55
- **LARSEN, T.O. ; FRISVAD, J.C. ; JENSEN, AURANTIAMINE.** a diketopiperazine from two varieties of Penicillium aurantiogriseum. *Phytochemistry,* 1992, vol. 31, 1613-1615
- **KANOH, K. et al.** Phenylahistin: a new mammalian cell cycle inhibitor produced by Aspergillus ustus. *Bioorg. Med. Chem. Lett.,* 1997, vol. 7, 2847-2852
- **JAN, C. ; DIPPENAAR, A. ; HOLZAPFEL.** Crystal structure of the metal complexes of viridamine. *S. Afr. J. Chem.,* 1977, vol. 30, 161-168
- **TAGUCHI, S ; SUGA.** Targeting of extracellular protein-protein interactions with macrocyclic peptides. *Curr. Opin. Chem. Biol.,* 2021, vol. 62, 82-89
- **PEACOCK, H. ; SUGA.** Discovery of De Novo Macrocyclic Peptides by Messenger RNADisplay. *Trends Pharmacol. Sci.,* 2021, vol. 42, 385-397
- **NOISIER, A.F. et al.** Late-Stage Functionalization of Histidine in Unprotected Peptides. *Angew. Chem.,* 2019, vol. 58, 19096-19102
- **CHEN, X. et al.** Histidine-Specific Peptide Modification via Visible-Light-Promoted C-H Alkylation. *J. Am. Chem. Soc.,* 2019, vol. 141, 18230-18237